# EUROPEAN PATENT APPLICATION

(11) **EP 4 755 877 A1**
(43) Date of publication of application: **10.06.2026**
(21) Application number: 24848410.7
(22) Date of filing: 05.08.2024
(51) Int. Cl.: C07D 311/30, C07D 213/74, A61K 31/05, A61K 31/7048, A61P 25/24

(54) **MODIFIED FLAVONE COMPOUNDS AND USE THEREOF**

(30) Priority: 03.08.2023 CN 202310973370
(71) Applicant: Suzhou Glenkol Pharma Technology Co., Ltd., Suzhou, Jiangsu, 215127 (CN); Huiankai (Xiamen) Pharmaceutical Technology Co., Ltd., Xiamen, Fujian 361028 (CN); Quanzhou Haichuang Pharmaceutical Technology Co., Ltd., Quanzhou, Fujian 362221 (CN)
(72) Inventor: CHEN, Jinhui, Suzhou, Jiangsu 215127 (CN); WU, Jie, Suzhou, Jiangsu 215127 (CN); HUANG, Peipei, Suzhou, Jiangsu 215127 (CN); ZHAO, Shu, Suzhou, Jiangsu 215127 (CN); WANG, Xiaoting, Suzhou, Jiangsu 215127 (CN)
(74) Representative: Hautier IP - MC/EP
(86) International application number: PCT/CN2024/109929
(87) International publication number: WO 2025/026457

(57) **Abstract**

The present disclosure provides modified flavone compounds and the use thereof, wherein the compounds are compounds as shown in formula (I), or a pharmaceutically acceptable stereoisomer, geometric isomer, tautomer, nitrogen oxide, hydrate, solvate, cocrystal, metabolite, salt, deuterated form, or prodrug thereof.

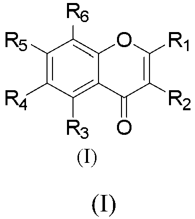

## Description

### TECHNICAL FIELD

The present disclosure relates to the field of pharmaceuticals, and specifically to modified flavone compounds and use thereof.

### BACKGROUND

Traumatic brain injury (TBI) is a clinically common brain injury caused by mechanical forces such as head impact, sudden acceleration, or sudden deceleration. TBI primarily manifests as impaired balance and coordination, reduced learning and cognitive abilities, diminished memory, and emotional disorders. Additionally, severe TBI patients may also exhibit a series of central nervous system damage symptoms, including repeated vomiting, dilated pupils, and lower limb weakness. Survivors of brain injury often develop a range of neurological dysfunctions as well as impairments in motion, sensory, and other bodily functions. Furthermore, brain injury increases patients' susceptibility to subsequent neurodegenerative diseases, such as Alzheimer's and Parkinson's disease. Currently, pharmacological sedation remains the primary treatment option for increased intracranial pressure caused by severe traumatic brain injury. No effective therapeutic agents exist to reduce cell death following traumatic brain injury.

In 2010, a study by Professor YE, Keqiang's team at Emory University, published in the Proceedings of the National Academy of Sciences*,* demonstrated that 7, 8-DHF can cross the blood-brain barrier. As a small-molecule agonist of the tropomyosin receptor kinase B (TrkB receptor), it effectively mimics the effects of brain-derived neurotrophic factor (BDNF) to protect neurons. 7, 8-DHF is the first reported drug capable of simulating BDNF effects, which can enter brain tissue by crossing the blood-brain barrier and demonstrate therapeutic potential for various neurodegenerative diseases and other brain dysfunction. It is of great significance to develop more modified flavone compounds for treating neurological diseases.

### SUMMARY

The present disclosure aims to address, at least to a certain extent, one of the technical problems in the art. To this end, an objective of the present disclosure is to provide modified flavone compounds that serve as small-molecule agonists for the tropomyosin receptor kinase B (TrkB receptor), and can effectively mimic the action of brain-derived neurotrophic factor (BDNF) to protect neurons, thereby aiding in the treatment of neurological and psychiatric diseases. Moreover, pharmacokinetic studies in animals have demonstrated exellent absorption, high bioactivity and bioavailability, and low toxicity. Notably, the compounds exhibit strong stability in vivo, which enables them to resist degradation and prolong their therapeutic duration. Consequently, they achieve better therapeutic efficacy and demonstrate promising application potential.

In an aspect of the present disclosure, a compound is provided. According to embodiments of the present application, the compound is a compound as shown in Formula (I), or a pharmaceutically acceptable stereoisomer, geometric isomer, tautomer, nitrogen oxide, hydrate, solvate, cocrystal, metabolite, salt, or prodrug thereof: 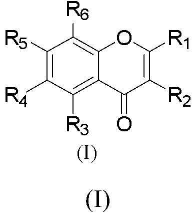 wherein,
R₁ is C₁₋₆ alkyl, C₂₋₆ alkenyl, C₂₋₆ alkynyl, aryl, C₃₋₆ cycloalkyl, or C₂₋₁₂ heterocyclyl, wherein the C₁₋₆ alkyl, C₂₋₆ alkenyl, C₂₋₆ alkynyl, aryl, C₃₋₆ cycloalkyl, and C₂₋₆ heterocyclyl are optionally substituted by one or more R₇;
R₂, R₃, R₄, R₅, R₆, and R₇ are each independently hydrogen, hydroxyl, halogen, C₁₋₆ alkyl, C₁₋₆ alkoxy, C₃₋₆ cycloalkoxy, C₁₋₆ heterocyclyl, glycosyl, 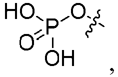 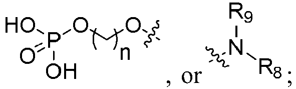 or R₄ and R₅ together with carbon atoms to which they are respectively attached form a 3-6-membered ring; or R₅ and R₆ together with carbon atoms to which they are respectively attached form a 3-6-membered ring, wherein the C₁₋₆ alkyl, C₁₋₆ alkoxy, C₃₋₆ cycloalkoxy, C₁₋₆ heterocyclyl, glycosyl, 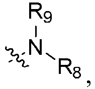 3-6 membered ring are optionally substituted by one or more substituents selected from hydrogen, hydroxyl, halogen, C₁₋₆ alkyl, C₁₋₆ alkoxy, C₃₋₆ cycloalkoxy, C₁₋₆ heterocyclyl, or 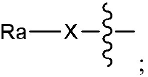
R₈ and R₉ are each independently hydrogen, C₁₋₆ alkyl, C₂₋₆ alkenyl, C₂₋₆ alkynyl, C₃₋₆ cycloalkyl, C₂₋₆ heterocyclyl, halo C₁₋₆ alkyl, aryl, or C₂₋₅ heteroaryl, or 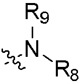 forms a 5-10 membered ring;
X is hydrogen, CH₂, S, NH, or O;
Rₐ is hydrogen, C₁₋₃ alkyl, C₂₋₄ alkenyl, halo C₁₋₃ alkyl, aryl, or C₃₋₆ heteroaryl;
n is an integer from 0 to 10;
when R₁ is aryl, the compound satisfies at least one of the following conditions:
   (1) at least one of R₄, R₅, R₆, and R₇ is halogen or hydroxyl;
   (2) at least one of R₅ and R₆ is glycosyl, 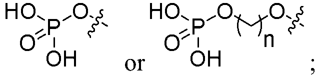
   (3) R₅ and R₆ together with carbon atoms to which they are respectively attached form 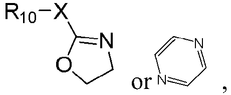 wherein X is a bond, hydrogen, CH₂, S, NH, or O, and R₁₀ is hydrogen, C₁₋₆alkyl, C₂₋₆ alkenyl, halo C₁₋₆ alkyl, aryl, or C₂₋₅ heteroaryl.

According to an embodiment of the present disclosure, the compound is a compound as shown in formula (I), or a pharmaceutically acceptable stereoisomer, geometric isomer, tautomer, nitrogen oxide, hydrate, solvate, cocrystal, metabolite, salt, or prodrug thereof: 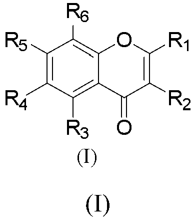 wherein,
R₁ is C₁₋₆ alkyl, C₂₋₆ alkenyl, C₂₋₆ alkynyl, aryl, C₃₋₆ cycloalkyl, or C₂₋₅ heterocyclyl, wherein the C₁₋₆ alkyl, C₂₋₆ alkenyl, C₂₋₆ alkynyl, aryl, C₃₋₆ cycloalkyl, and C₂₋₆ heterocyclyl are optionally substituted by one or more R₇;
R₂, R₃, R₄, R₅, R₆, and R₇ are each independently hydrogen, hydroxy, halogen, C₁₋₆ alkyl, C₁₋₆ alkoxy, glycosyl, 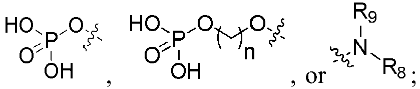 or R₅ and R₆ together with carbon atoms to which they are respectively attached form a 3-6 membered ring;
R₈ and R₉ are each independently hydrogen, C₁₋₆alkyl, C₂₋₆alkenyl, C₂₋₆ alkynyl, C₃₋₆ cycloalkyl, C₂₋₆ heterocyclyl, halo C₁₋₆ alkyl, aryl, or C₂₋₅ heteroaryl, or 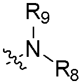 forms a 5-10 membered ring;
n is an integer from 0 to 10;
When R₁ is aryl, the compound satisfies at least one of the following conditions:
   (1) at least one of R₄, R₅, R₆, and R₇ is halogen;
   (2) at least one of R₅ and R₆ is a glycosyl, 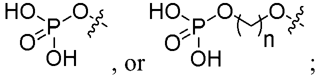
   (3) R₅ and R₆ together with carbon atoms to which they are respectively attached form 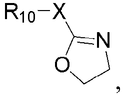
   wherein X is hydrogen, CH₂, S, NH, or O; and R₁₀ is hydrogen, C₁₋₆ alkyl, C₂₋₆ alkenyl, halo C₁₋₆ alkyl, aryl, or C₂₋₅ heteroaryl.

According to an embodiment of the disclosure, R₂ is hydrogen, and R₃ is hydrogen or OH.

According to an embodiment of the present disclosure, R₁ is aryl, and R₇ is an ortho-substituent;
R₄, R₅, and R₆ are each independently hydrogen, halogen, hydroxy, or C₁₋₆ heterocyclyl;
R₇ is halogen, C₂₋₄ alkyl, or C₁₋₄ alkoxy.

According to embodiments of the present disclosure, R₁ is aryl, R₇ is an ortho-substituent, and preferably, R₁ is 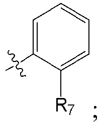
R₄, R₅, and R₆ are each independently hydrogen, halogen, or hydroxyl;
R₇ is halogen, C₂₋₄ alkyl, or C₁₋₄ alkoxy.

According to an embodiment of the present disclosure, R₁ is 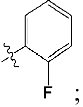
R₄ is hydrogen;
R₅ and R₆ are each independently hydroxy or halogen; more preferably, R₅ and R₆ are hydroxyl.

According to an embodiment of the present disclosure, R₁ is aryl;
at least one of R₄, R₅, and R₆ is halogen or hydroxyl, and remainder are hydrogen, hydroxyl, or C₁₋₆ heterocyclyl.

According to an embodiment of the present disclosure, R₁ is aryl;
at least one of R₄, R₅, and R₆ is halogen, andremainder are hydrogen or hydroxyl.

According to an embodiment of the present disclosure, R₁ is aryl;
R₄ is hydrogen or halogen;
R₅ and R₆ are each independently hydroxyl, halogen, or C₁₋₆ heterocyclyl;
more preferably, R₄ is hydrogen; R₅ is hydroxyl; and R₆ is halogen, preferably fluorine;
or R₄ is hydrogen; R₅ is halogen, preferably fluorine; and R₆ is hydroxyl;
or R₄ is hydrogen; R₅ is halogen, preferably fluorine; and R₆ is halogen, preferably fluorine;
or R₄ is halogen, preferably fluorine; and R₅ and R₆ are hydroxyl;
or R₄ is hydrogen; R₅ is hydroxyl; and R₆ is piperidine.

According to an embodiment of the present disclosure, one of R₅ and R₆ is a glycosyl, 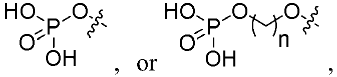 and the other is hydrogen, hydroxyl, halogen, glycosyl, 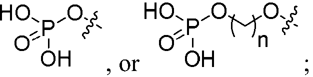
the glycosyl is tetrosyl, pentosyl, or hexosyl;
R₁ is aryl or 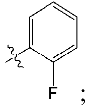
R₂ and R₃ are hydrogen;
R₄ is hydrogen or halogen;
R7 is hydrogen, deuterium, halogen, or a C₁₋₆ heterocyclyl.

According to an embodiment of the present disclosure, one of R₅ and R₆ is glycosyl, 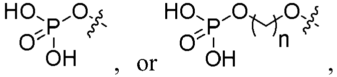 and the other is hydrogen, hydroxyl, halogen, glycosyl, 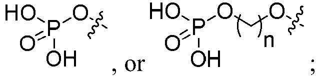
the glycosyl is tetrosyl, pentosyl, or hexosyl;
R₁ is aryl or 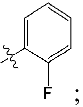
R₂ and R₃ are hydrogen;
R₄ is hydrogen or halogen;
R₇ is hydrogen or deuterium.

According to an embodiment of the present disclosure, one of R₅ and R₆ is glycosyl, and the other is hydroxyl;
the glycosyl is 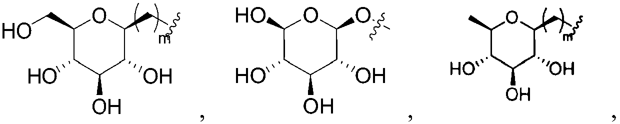 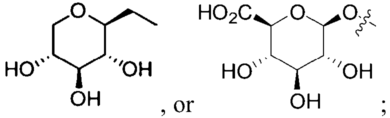
R₁ is aryl;
R₂, R₃, and R₄ are hydrogen;
m is an integer from 0 to 10, preferably 0, 1, 2, or 3.

According to an embodiment of the present disclosure, n is 0, 1, 2, or 3.

According to an embodiment of the present disclosure, R₁ is aryl;
R₂, R₃, and R₄ are each independently hydrogen, hydroxyl, C₁₋₃ alkyl, or halogen;
R₄ and R₅ together with the carbon atoms to which they are respectively attached form 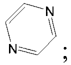 or R₅ and R₆ together with the carbon atoms to which they are respectively attached form 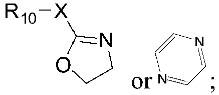
R₇ is hydroxyl or 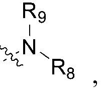 being a meta- or para-substituent; more preferably, R₁ 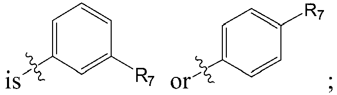
R₈ and R₉ are each independently hydrogen, C₁₋₃ alkyl, C₂₋₄ alkenyl, C₂₋₄alkynyl, C₄₋₆ cycloalkyl, C₄₋₆ heterocyclyl, aryl, or C₂₋₆ heteroaryl, or 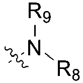 forms a 5-10 membered ring;
the 5-10 membered ring is C₄₋₆ cycloalkyl, C₄₋₆ heterocyclyl, C₈₋₁₀ fused cyclyl or C₈₋₁₀ fused heterocyclyl;
the 5-10 membered ring is optionally substituted by C₄₋₆ cycloalkyl or C₄₋₆ heterocyclyl, more preferably, the 5-10 membered ring is C₄₋₆ cycloalkyl or C₄₋₆ heterocyclyl, wherein the C₄₋₆ cycloalkyl and C₄₋₆ heterocyclyl are optionally substituted by C₄₋₆ cycloalkyl or C₄₋₆ heterocyclyl;
X is hydrogen, CH₂, S, NH, or O;
R₁₀ is hydrogen, C₁₋₃ alkyl, C₂₋₄ alkenyl, halo C₁₋₃ alkyl, aryl, or C₃₋₅ heteroaryl.

According to an embodiment of the present disclosure, R₁ is aryl;
R₂, R₃, and R₄ are each independently selected from hydrogen, hydroxyl, C₁₋₃ alkyl, or halogen;
R₅ and R₆ together with the carbon atoms to which they are respectively attached form 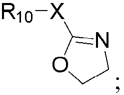
R₇ is selected from hydroxyl or 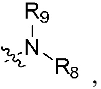 wherein R₇ is a meta- or para-substituent; more preferably, R₁ is 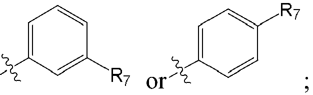
R₈ and R₉ are each independently hydrogen, C₁₋₃ alkyl, C₂₋₄ alkenyl, C₂₋₄ alkynyl, C₄₋₆ cycloalkyl, C₄₋₆ heterocyclyl, or C₂₋₅ heteroaryl, or 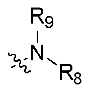 forms a 5-10 membered ring;
the 5-10 membered ring is C₄₋₆ cycloalkyl, C₄₋₆ heterocyclyl, C₈₋₁₀ fused cyclyl, or C₈₋₁₀ fused heterocyclyl;
the 5-10 membered ring is optionally substituted by C₄₋₆ cycloalkyl or C₄₋₆ heterocyclyl; more preferably, the 5-10 membered ring is C₄₋₆ cycloalkyl or C₄₋₆ heterocyclyl, the C₄₋₆ cycloalkyl and C₄₋₆ heterocyclyl are optionally substituted by C₄₋₆ cycloalkyl or C₄₋₆ heterocyclyl;
X is selected from hydrogen, CH₂, S, NH, or O;
R₁₀ is selected from hydrogen, C₁₋₃ alkyl, C₂₋₄ alkenyl, halo C₁₋₃ alkyl, aryl, or C₃₋₅ heteroaryl.

According to an embodiment of the present disclosure, 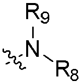 is 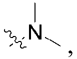 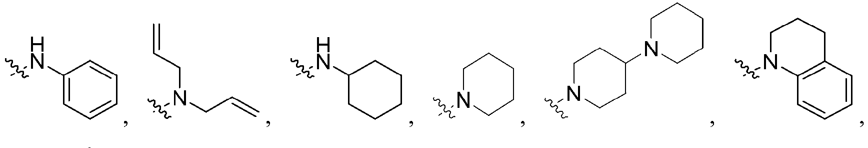 or 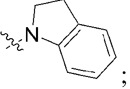 R₁₀ is hydrogen or C₁₋₃ alkyl.

According to an embodiment of the present disclosure, 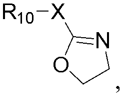 which is formed by R₅ and R₆ together with the carbon atoms to which they are respectively attached, is

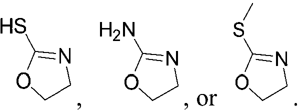

According to an embodiment of the present disclosure, R₁ is C₂₋₄ alkenyl, C₂₋₄ alkynyl, C₃₋₆ cycloalkyl, or C₄₋₆ heterocyclyl;
R₂, R₃, and R₄ are each independently hydrogen, hydroxyl, C₁₋₃ alkyl, or halogen;
R₅ and R₆ are each independently hydrogen, halogen, or hydroxyl.

According to an embodiment of the present disclosure, R₁ is C₂₋₁₂ heterocyclyl;
R₂, R₃, R₄, R₅, R₆, and R₇ are each independently hydrogen, hydroxyl, halogen, C₁₋₆ alkyl, C₁₋₆ alkoxy, C₃₋₆ cycloalkoxy, C₁₋₆ heterocyclyl, glycosyl, 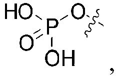 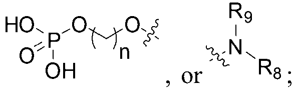 or R₄ and R₅ together with the carbon atoms to which they are respectively attached form a 3-6-membered ring; or R₅ and R₆ together with the carbon atoms to which they are respectively attached form a 3-6-membered ring;
R₈ and R₉ are each independently hydrogen, C₁₋₆ alkyl, C₂₋₆ alkenyl, C₂₋₆ alkynyl, C₃₋₆ cycloalkyl, C₂₋₆ heterocyclyl, halo C₁₋₆ alkyl, aryl, or C₂₋₅ heteroaryl, or 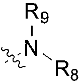 forms a 5-10-membered ring; and
n is an integer from 0 to 10.

According to an embodiment of the present disclosure, R₁ is C₂₋₆ heterocyclyl;
R₂, R₃, and R₄ are each independently hydrogen, hydroxyl, C₁₋₃ alkyl, or halogen;
R₅ and R₆ are each independently hydrogen, hydroxyl, halogen, glycosyl, C₁₋₆ heterocyclyl, or a C₁₋₆ alkoxy; or R₅ and R₆ together with the carbon atoms to which they are respectively attached form 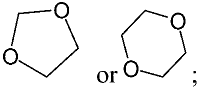
wherein the glycosyl is

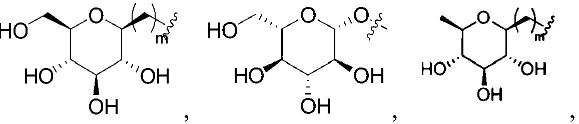

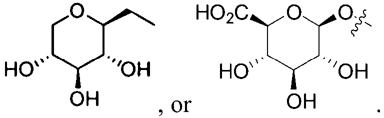

According to an embodiment of the present disclosure, R₁ is 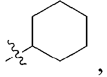 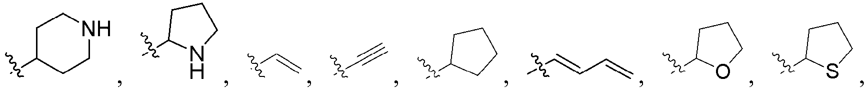 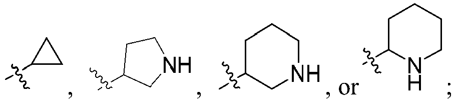
R₂, R₃, and R₄ are each independently hydrogen, hydroxyl, or halogen;
at least one of R₅ and R₆ is hydroxyl, and the other is hydroxyl or halogen.

According to an embodiment of the present disclosure, the compound is a compound as shown in formula (I), or a stereoisomer, geometric isomer, tautomer, nitrogen oxide, hydrate, solvate, metabolite, pharmaceutically acceptable salt, or prodrug thereof:

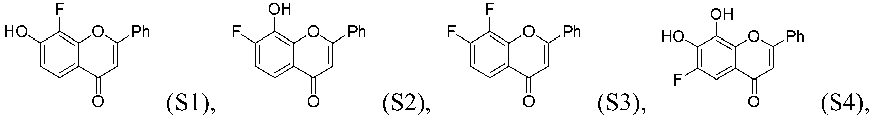

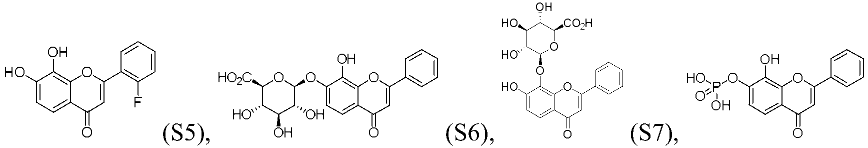

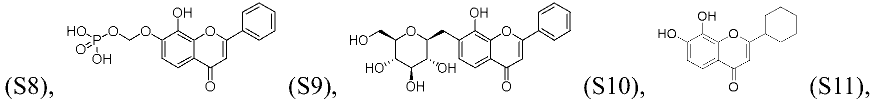

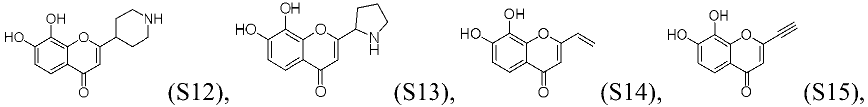

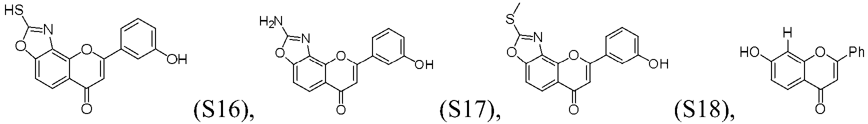

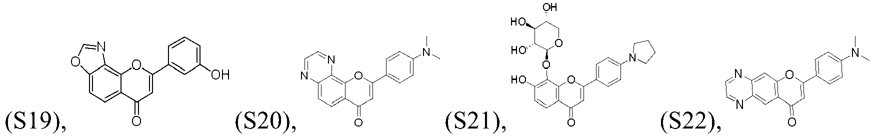

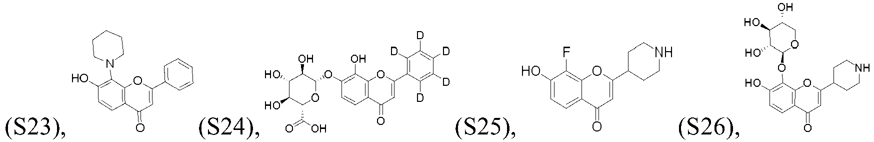

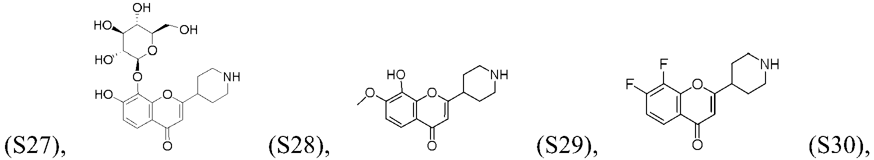

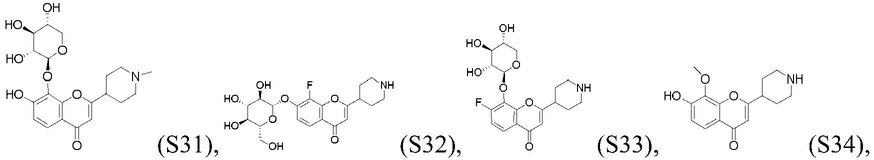

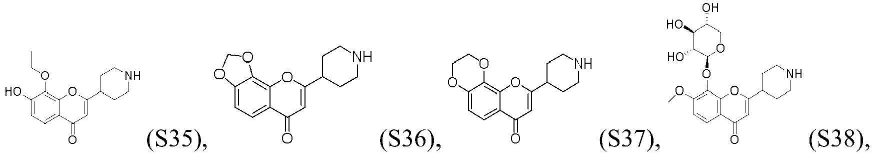

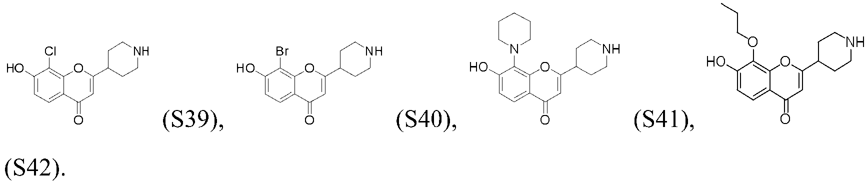

According to an embodiment of the present disclosure, the compound is not a compound as shown below:

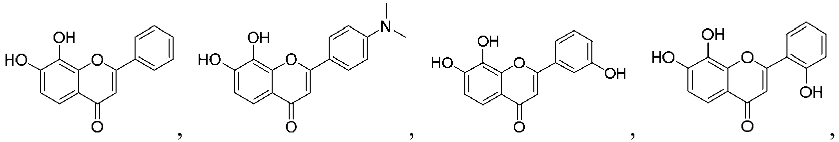

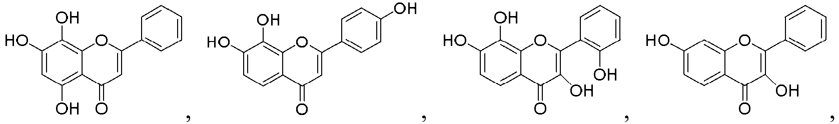

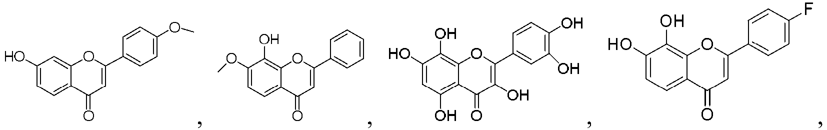

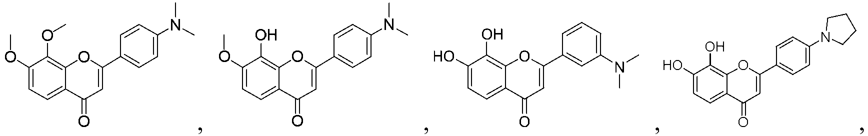

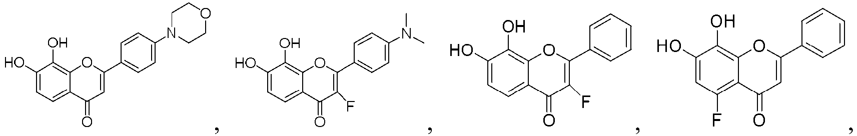

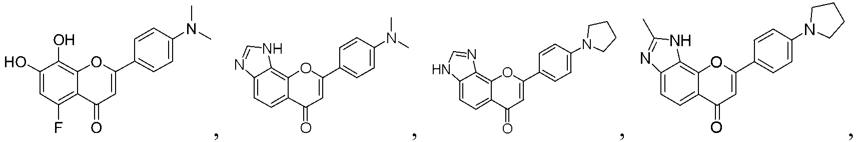

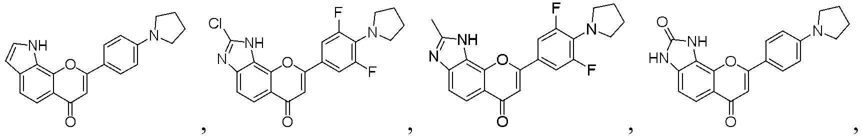

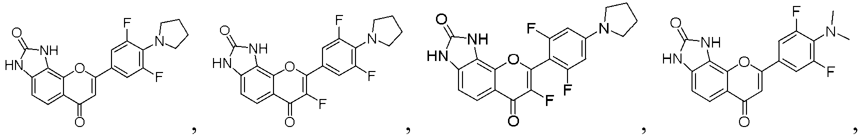

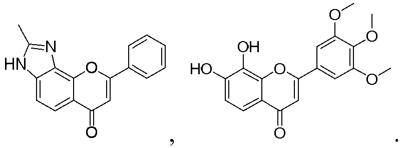

In another aspect of the present disclosure, a pharmaceutical composition is provided. According to an embodiment of the present disclosure, the pharmaceutical composition includes the compound described above.

According to an embodiment of the present disclosure, the pharmaceutical composition further includes a pharmaceutically acceptable excipient, wherein the excipient includes: a filler, a carrier, an adjuvant, a vehicle, or combinations thereof.

In yet another aspect of the present disclosure, a combination drug is provided. According to an embodiment of the disclosure, the combination drug includes the aforementioned pharmaceutical composition; one or more therapeutic agents for treating diseases; wherein the diseases include: neurological diseases, psychiatric diseases.

According to an embodiment of the present disclosure, the neurological diseases include: central nervous system injury, peripheral nerve injury, neurodegenerative diseases, and hereditary neurological dysfunctions, preferably brain injury, and more preferably traumatic brain injury.

According to an embodiment of the present disclosure, the therapeutic agents for treating diseases include at least one of the following: neuroprotective agents, neurotrophic agents, neurorestorative agents, B-vitamins, antidepressants, anxiolytics, lithium salts as mood stabilizers, antipsychotics, atypical antipsychotics, antiepileptics, antiparkinsonian agents, sedative-hypnotics, antihistamines, monoamine oxidase inhibitors, melatonin receptor agonists, non-opioid and opioid analgesics.

According to an embodiment of the present disclosure, the therapeutic agents for treating diseases include at least one of the following: edaravone, butylphthalide, glutathione, vitamin C, vitamin E, dexmedetomidine, ketamine, propofol, midazolam, remimazolam, citalopram, B vitamins, methylcobalamin, nerve growth factor, gangliosides, levetiracetam, oxiracetam, piracetam, aniracetam, citicoline, donepezil, rivastigmine, galantamine, selegiline, memantine, brexpiprazole, amantadine, levodopa, carbidopa, entacapone, benserazide, bromocriptine, pramipexole, ropinirole, piribedil, cabergoline, apomorphine, lisuride, rotigotine, safinamide, rasagiline, istradefylline, pimavanserin, carbamazepine, oxcarbazepine, lamotrigine, phenytoin sodium, valproic acid, phenobarbital, ethosuximide, topiramate, lacosamide, paroxetine, venlafaxine, duloxetine, escitalopram, sertraline, alprazolam, mirtazapine, morphine, codeine, oxycodone, hydrocodone, fentanyl, pethidine, methadone, dextropropoxyphene, endorphin, dynorphin, chlorpromazine, and haloperidol.

In yet another aspect of the present disclosure, use of the aforementioned compound, pharmaceutical composition, or combination drug in the manufacture of a medicament. According to an embodiment of the present disclosure, the medicament is used for treating neurological diseases and psychiatric diseases.

According to an embodiment of the present disclosure, the neurological diseases include: central nervous system injury, peripheral nervous system injury, neurodegenerative diseases, and hereditary neurological dysfunctions, preferably brain injury, and more preferably traumatic brain injury.

In yet another aspect of the present disclosure, provided herein is a method for treating neurological and psychiatric diseases. According to an embodiment of the present application, the method includes: administering the aforementioned compound, pharmaceutical composition, or combination drug to a subject in need thereof.

According to an embodiment of the present application, the neurological diseases include: central nervous system injury, peripheral nerve injury, neurodegenerative diseases, and hereditary neurological dysfunctions.

According to an embodiment of the present application, the neurological diseases are brain injury.

According to an embodiment of the present application, the neurological diseases are traumatic brain injury.

In yet another aspect of the present disclosure, provided is a method for activating the TrkB receptor on neurons. According to an embodiment of the disclosure, the method includes: providing neurons having the TrkB receptor; and contacting the neurons with the aforementioned compound, pharmaceutical composition, or combination drug.

In yet another aspect of the disclosure proposes a method for treating neurological and psychiatric diseases. According to an embodiment of the disclosure, the method includes administering the aforementioned compound, pharmaceutical composition, or combination drug to a subject in need thereof.

Additional aspects and advantages of the present application will be given in part in the following description, which will become apparent from the following description or be learned through the practice of the present application.

### DETAILED DESCRIPTION

### Definitions and General Terms

Some embodiments disclosed herein will now be described in detail, examples of which are illustrated by the accompanying structural and chemical formulas. The disclosure is intended to cover all alternative, modified and equivalent technical solutions, all of which are included within the scope disclosed herein as defined by the claims. One skilled in the art will recognize that many methods and materials similar or equivalent to those described herein can be used in the practice disclosed herein. Disclosed herein is in no way limited to the methods and materials. In the event that one or more of the incorporated literature, patents and similar materials differ from or contradict this application (including but not limited to the term definition, term usage and/or described techniques, and the like), the present application shall prevail.

It should be further recognized that certain features disclosed herein have been described in multiple independent embodiments for clarity, but may be also provided in combination in a single embodiment. On the contrary, various features disclosed herein have been described in a single embodiment for the sake of brevity, but can be also provided individually or in any suitable subcombination.

Unless otherwise stated, all scientific and technical terms used in the present disclosure have the same meanings as those commonly understood by those skilled in the art. All patents and public publications involved in the disclosure are incorporated herein by reference in their entireties.

Unless otherwise stated, the following definitions used in the present application should be used. For purposes of the present disclosure, the chemical elements are identified in accordance with the Periodic Table of the Elements, CAS version and the Handbook of Chemistry and Physics, 75th Ed. 1994. Additionally, general principles of organic chemistry are described in *"*Organic Chemistry", Thomas Sorrell, University Science Books, Sausalito: 1999, and "March's Advanced Organic Chemistry" by Michael B. Smith and Jerry March, John Wiley & Sons, New York: 2007, all of which are incorporated herein by reference in their entireties.

The articles "a", "an" and "the", as used herein, are intended to include "at least one" or "one or more" unless otherwise indicated herein or clearly contradicted by the context. Thus, the articles are used herein to refer to one or more than one (i.e. at least one) of the objects. By way of example, "a component" means one or more components, and thus, possibly, more than one component is contemplated and may be employed or used in an implementation of the described embodiments.

The term "comprising" is an open-ended expression, meaning it includes the contents specified herein without excluding other contents.

### Compound

The present disclosure provides modified flavone compounds, or pharmaceutically acceptable stereoisomers, geometric isomers, tautomers, nitrogen oxides, hydrates, solvates, cocrystals, metabolites, salts, or prodrugs thereof. These compounds serve as small-molecule agonists for the tropomyosin receptor kinase B (TrkB receptor), and can effectively mimic the action of brain-derived neurotrophic factor (BDNF) to protect neurons, thereby aiding in the treatment of neurological and psychiatric diseases. Furthermore, pharmacokinetic studies in animals have demonstrated excellent absorption, high bioactivity and bioavailability, and low toxicity. Notably, the compounds exhibit strong stability in vivo, which enables them to resist degradation and prolong their therapeutic duration. Consequently, they achieve better therapeutic efficacy and demonstrate promising application potential.

In an aspect, the present disclosure relates to a compound, which is a compound as shown in Formula (I), or a pharmaceutically acceptable stereoisomer, geometric isomer, tautomer, nitrogen oxide, hydrate, solvate, cocrystal, metabolite, salt, or prodrug thereof: 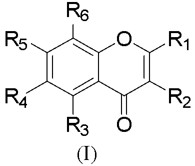 wherein R₁, R₂, R₃, R₄, R₅, and R₆ have the meanings described herein.

"Stereoisomer" refers to compounds, which have identical chemical constitution, but differ with regard to the arrangement of the atoms or groups in space. Stereoisomers include enantiomers, diastereomers, conformational isomers (rotamers), geometric isomers (cis/trans isomers), atropisomers, and the like.

"Chiral" refers to a molecule that has the property of non-superimposability of the mirror image, while "achiral" refers to a molecule which are superimposable on their mirror image.

"Enantiomer" refers to two stereoisomers of a compound which are non-superimposable mirror images of one another.

"Diastereomer" refers to a stereoisomer with two or more centers of chirality and whose molecules are not mirror images of one another. Diastereomers have different physical properties, e.g., melting points, boiling points, spectral properties or biological activities. Mixture of diastereomers may separate under high resolution analytical procedures such as electrophoresis and chromatography such as HPLC.

The stereochemical definitions and conventions used herein generally follow S.P. Parker, Ed., McGraw-Hill Dictionary of Chemical Terms (1984) McGraw-Hill Book Company, New York; and Eliel, E. and Wilen, S., Stereochemistry of Organic Compounds, John Wiley & Sons, Inc., New York, 1994.

Many organic compounds exist in optically active forms, i.e., they have the ability to rotate the plane of plane-polarized light. In describing an optically active compound, the prefixes D and L, or R and S, are used to denote the absolute configuration of the molecule about its chiral center(s). The prefixes d and 1 or (+) and (-) are employed to designate the sign of rotation of plane-polarized light by the compound, with (-) or 1 meaning that the compound is levorotatory. A compound prefixed with (+) or d is dextrorotatory. A specific stereoisomer may also be referred to as an enantiomer, and a mixture of such isomers is often called an enantiomeric mixture. A 50:50 mixture of enantiomers is referred to as a racemic mixture or a racemate, which may occur where there has been no stereoselection or stereospecificity in a chemical reaction or process.

Any asymmetric atom (e.g., carbon) of the compound of the present disclosure can be present in racemic or enantiomerically enriched, for example the (R)-, (S)- or (R, S)-configuration. In certain embodiments, each asymmetric atom has at least 50% enantiomeric excess, at least 60% enantiomeric excess, at least 70% enantiomeric excess, at least 80% enantiomeric excess, at least 90% enantiomeric excess, at least 95% enantiomeric excess, or at least 99% enantiomeric excess in the (R)- or (S)-configuration.

Depending on the choice of starting materials and methods, the compounds of the present disclosure may exist in the form of one of the possible isomers or as mixtures thereof, such as racemates and diastereomeric mixtures (depending on the number of asymmetric carbon atoms). Optically active (R)- or (S)-isomers may be prepared using chiral synthons or chiral reagents, or separated by conventional techniques. If the compound contains a double bond, substituents may be in the E or Z configuration; if the compound contains a disubstituted cycloalkyl, substituents on the cycloalkyl may be in the cis or trans configuration.

Any mixture of stereoisomers obtained can be separated into pure or essentially pure geometric isomers, enantiomers, or diastereomers based on differences in the physicochemical properties of the components, for example, by chromatography and/or fractional crystallization.

Any resulting racemate of final product or intermediate can be separated into optical enantiomers by methods known to those skilled in the art, such as through separation of the obtained diastereomeric salts. Racemic products may also be separated by chiral chromatography, such as high-performance liquid chromatography (HPLC) using chiral adsorbents. Specifically, enantiomers may be prepared via asymmetric synthesis, referring to, for example, Jacques, et al., Enantiomers, Racemates and Resolutions (Wiley Interscience, New York, 1981); Principles of Asymmetric Synthesis (2nd Ed. Robert E. Gawley, Jeffrey Aubé, Elsevier, Oxford, UK, 2012); Eliel, E.L. Stereochemistry of Carbon Compounds (McGraw-Hill, NY, 1962); Wilen, S.H. Tables of Resolving Agents and Optical Resolutions, p. 268 (E.L. Eliel, Ed., Univ. of Notre Dame Press, Notre Dame, IN 1972);Chiral Separation Techniques: A Practical Approach (Subramanian, G. Ed., Wiley-VCH Verlag GmbH & Co. KGaA, Weinheim, Germany, 2007).

The term "tautomer" or "tautomeric form" refers to structural isomers of different energies that can interconvert via a low energy barrier. If tautomerism is possible (e.g., in solution), chemical equilibrium between tautomers can be achieved. For example, a proton tautomer (also referred to as prototropic tautomer) includes interconversion via proton transfer, such as keto-enol isomerism and imine-enamine isomerism.

As described herein, the compounds disclosed herein may optionally be substituted with one or more substituents, such as are illustrated by the general formula of compound, or as exemplified by particular classes, subclasses, and species of the invention.

Generally, the term "substituted" indicates that one or more hydrogen atoms in the given structure are replaced by specific substituents. Unless otherwise indicated, a substituted group may have one substituent at any replaceable position within the group. When more than one position in the given structure can be substituted by one or more substituents selected from specific groups, substituents may be identical or different at each replaceable position.

Additionally, it should be noted that unless otherwise explicitly stated, the descriptive terms "each...independently" and "...each independently" and "...independently" used in the disclosure are interchangeable and shall be interpreted broadly. They may indicate that, among different groups, the specific options represented by the same symbols do not affect each other, or they may indicate that, within the same group, the specific options represented by the same symbols do not affect each other.

Throughout the various sections of this specification, the substituents of the compounds disclosed herein are disclosed according to group type or range. It is specifically noted that the present disclosure encompasses each independent secondary combination of every member within these group types and ranges. For example, the term "C₁₋₆ alkyl" specifically refers to independently disclosed methyl, ethyl, C₃ alkyl, C₄ alkyl, C₅ alkyl, and C₆ alkyl.

Throughout the various sections of this specification, linking substituents are described. Where the structure clearly requires a linking group, the Markush variable listed for that group should be understood as a linking group. For example, if the structure requires a linking group and the Markush group definition for that variable lists "alkyl" or "aryl, " it should be understood that the "alkyl" or "aryl" respectively represents a linking alkylene group or arylene group.

The term "alkyl" or "alkyl group" as used herein denotes a saturated, straight-chain or branched, monovalent hydrocarbon group, wherein the alkyl group may optionally be substituted by one or more substituents described herein. Examples of alkyl groups include, but are not limited to, methyl (Me, -CH₃), ethyl (Et, -CH₂CH₃), n-propyl (*n*-Pr, -CH₂CH₂CH₃), isopropyl (*i*-Pr, -CH(CH₃)₂), n-butyl (*n*-Bu, -CH₂CH₂CH₂CH₃), isobutyl (*i*-Bu, -CH₂CH(CH₃)₂), sec-butyl (*s*-Bu, -CH(CH₃)CH₂CH₃), tert-butyl (*t*-Bu, -C(CH₃)₃), n-pentyl (-CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH (CH₃)₂), 3-methyl-1-butyl (-CH₂CH₂CH(CH₃)₂), 2-methyl-1-butyl (-CH₂CH(CH₃)CH₂CH₃), n-hexyl (-CH₂CH₂CH₂CH₂CH₂CH₃), 2-hexyl (-CH(CH₃)CH₂CH₂CH₂CH₃), 3- hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃), and the like.

The term "alkoxy" denotes an alkyl group connected to the remainder of the molecule via an oxygen atom, wherein the alkyl group has the meaning described herein. Unless otherwise specified, the alkoxy group contains 1-6 carbon atoms. In another embodiment, the alkoxy group contains 1-4 carbon atoms; in yet another embodiment, the alkoxy group contains 1-3 carbon atoms.

Examples of alkoxy groups include, but are not limited to, methoxy (MeO, -OCH₃). ethoxy (EtO, -OCH₂CH₃), 1-propoxy (*n*-PrO, *n*-propoxy, -OCH₂CH₂CH₃), 2-propoxy (*i*-PrO, *i*-propoxy, -OCH(CH₃)₂), 1-butoxy (*n*-BuO, n-butoxy, -OCH₂CH₂CH₂CH₃), 2-methyl-1-propoxy (i-BuO, *i*-butoxy, -OCH₂CH(CH₃)₂), 2-butoxy (*s*-BuO, *s*-butoxy, -OCH(CH₃)CH₂CH₃), 2-methyl-2-propoxy (t-BuO, *t*-butoxy, -OC(CH₃)₃), 1-pentoxy (n-pentoxy, -OCH₂CH₂CH₂CH₂CH₃), 2-pentoxy (-OCH(CH₃)CH₂CH₂CH₃), 3-pentoxy (-OCH(CH₂CH₃)₂), 2-methyl-2-butoxy (-OC(CH₃)₂CH₂CH₃), 3-methyl-2-butoxy (-OCH(CH₃)CH(CH₃)₂), 3-methyl-1-butoxy (-OCH₂CH₂CH(CH₃)₂), 2-methyl-1-butoxy (-OCH₂CH(CH₃)CH₂CH₃), and the like _{∘}

The term "3-6-membered ring" denotes a cyclic group comprising 3 to 6 atoms, including saturated or partially unsaturated cycloalkyl or heterocyclyl.

According to an embodiment of the present disclosure, R₅ and R₆ form 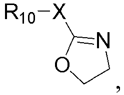 i.e., the structure of formula (I) is 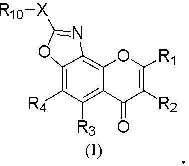 X may be a bond, representing

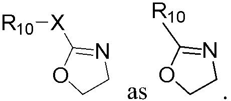

The term "cycloalkyl" denotes a monovalent or multivalent saturated monocyclic, bicyclic, or tricyclic system containing 3 to 6 ring carbon atoms. In one embodiment, the cycloalkyl contains 3 to 4 ring carbon atoms. In another embodiment, the cycloalkyl contains 3 to 5 ring carbon atoms. The cycloalkyl group may be independently unsubstituted or substituted with one or more substituents described herein.

Examples of cycloalkyl groups further include cyclopropyl, cyclobutyl, cyclopentyl, 1-cyclopentyl-1-enyl, 1-cyclopentyl-2-enyl, 1-cyclopentyl-3-enyl, cyclohexyl, 1-cyclohexyl-1-enyl, 1-cyclohexyl-2-enyl, 1-cyclohexyl-3-enyl, cyclohexadienyl, and the like.

The term "heterocyclyl" is used interchangeably herein and refers to saturated or partially unsaturated, non-aromatic, monovalent or polyvalent monocyclic, bicyclic, or tricyclic rings containing 3-6 ring atoms, wherein at least one ring atom is selected from nitrogen, sulfur, or oxygen atoms. Unless otherwise specified, the heterocyclyl may be carbon-based or nitrogen-based, and the -CH₂- group may optionally be substituted by -C(=O)-. The sulfur atom in the ring may optionally be oxidized to an S-oxide. The nitrogen atom in the ring may optionally be oxidized to an N-oxide.

The term "aryl" may be used alone or as a major part of "aralkyl", "aralkoxy", or "aryloxyalkyl", denoting a monocyclic, bicyclic, or tricyclic carbocyclic ring system containing 6 to 14 ring atoms, wherein at least one ring system is aromatic, with each ring system containing 3 to 7 ring atoms and having only one attachment point to the rest of the molecule. The term "aryl" may be used interchangeably with "aromatic ring" which, for example, may include phenyl, naphthyl, and anthryl.

The term "heteroatom" refers to O, S, N, P, and Si, including any oxidized form of N, S, and P; primary, secondary, tertiary amine, and quaternary ammonium salt forms; or forms where hydrogen atoms on nitrogen atoms in heterocycles are substituted. For example, N (as in N in 3, 4-dihydro-2H-pyrrolyl), NH (as in NH in pyrrolidinyl), or NR (*as* in NR in N-substituted pyrrolidinyl).

The term "halogen" refers to fluorine (F), chlorine (Cl), bromine (Br), or iodine (I).

The term "glycosyl" denotes the monosaccharide moiety in a molecule formed by the monosaccharide with another substance that is not a carbohydrate, referred to as the glycosyl of the molecule. According to an embodiment of the present disclosure, the glycosyl is selected from tetrosyl, pentosyl, or hexosyl. As the tetrosyl, it may be erythrosyl and the like. As the pentosyl, it may be arabinosyl, lyxosyl, ribosyl, xylosyl and the like. As the hexosyl, it may be allosyl, fructosyl, galactosyl, glucosyl, gulosyl, mannosyl, tagatosyl, talosyl, sialyl, glucuronosyl, C-glucosyl and the like.

In some embodiments, the glycosyl structural formulae are as follows: 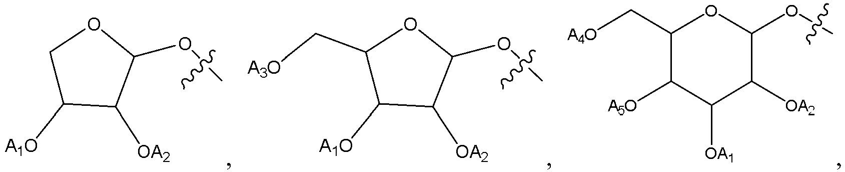 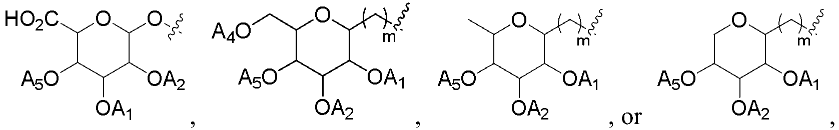 wherein A₁, A₂, A₃, A₄, and A₅ are selected from hydrogen, hydroxyl, carboxyl, nitro, sulfo, phosphate, or C₁₋₄ alkyl. m may be 0, 1, 2, 3, 4, 5, 6, 7, 8, 9, 10, or any range formed by two values as endpoints.

According to an embodiment of the present disclosure, R₅ and R₆, together with the carbon atoms to which they are respectively attached, form 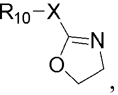 forming

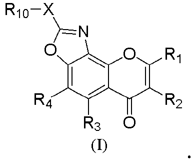

The term "prodrug" as used herein denotes a compound that is converted in vivo to the compound shown in formula (I). Such conversion is influenced by the hydrolysis of the prodrug in blood or its enzymatic conversion to the parent structure in blood or tissue. Prodrug compounds of the present disclosure may be esters, and examples of esters that may serve as prodrugs in the present disclsoure include phenyl esters, aliphatic (C₁-C₂₄) esters, acyloxymethyl esters, carbonates, carbamates, and amino acid esters. For example, when a compound in the present disclosure contains a hydroxyl group, it can be acylated to yield a prodrug thereof. Other prodrug forms include phosphates, such as those obtained by phosphorylation of a hydroxyl group on the parent compound. A comprehensive discussion of prodrugs can refer to the following literature: T. Higuchi and V Stella, Pro-drugs as Novel Delivery Systems, Vol. 14 of the A.C.S. Symposium Series, Edward B. Roche, ed., Bioreversible Carriers in Drug Design, American Pharmaceutical Association and Pergamon Press, 1987**;** J. Rautio et al., Prodrugs: Design and Clinical Applications, Nature Review Drug Discovery, 2008, 7, 255-270, and S. J. Hecker et al., Prodrugs of Phosphates and Phosphonates, Journal of Medicinal Chemistry, 2008, 51, 2328-2345.

"Metabolite" refers to a product obtained through the metabolism of a specific compound or its salt in vivo. The metabolite of a compound can be identified using techniques well known in the art and its activity can be characterized experimentally as described herein. Such products may be obtained from the administered compound via processes such as oxidation, reduction, hydrolysis, amidation, deamidation, esterification, deesterification, or enzymatic cleavage. Accordingly, the present disclosure encompasses metabolites of the compounds, including those generated by contacting the compounds of the disclosure with a mammal for a sufficient period.

The term "pharmaceutically acceptable salt" as used herein refers to organic and inorganic salts of the compounds disclosed herein. Pharmaceutically acceptable salts are well known in the art. For example, S. M. Berge et al. describe pharmaceutically acceptable salts in J. Pharmaceutical Sciences, 1977, 66: 1-19.

The term "solvate" in the present disclosure refers to an association formed between one or more solvent molecules and the compound of the present disclosure. Solvents forming solvates include, but are not limited to, water, isopropanol, ethanol, methanol, dimethyl sulfoxide, ethyl acetate, acetic acid, and aminoethanol. The term "hydrate" refers to an association in which the solvent molecule is water.

When the solvent is water, the term "hydrate" may be used. In some embodiments, a compound molecule of the present disclosure may be associated to one water molecule, such as a monohydrate; in other embodiments, a compound molecule of the present disclosure may be associated to more than one water molecule, such as a dihydrate; and in yet other embodiments, a compound molecule of the present disclosure may be associated to less than one water molecule, such as a hemihydrate. It should be noted that the hydrates of the present disclosure retain the bioactivity of the compounds in non-hydrated forms.

In other aspects, the present disclosure relates to intermediates for preparing compounds shown in formula (I).

In other aspects, the present disclosure relates to methods for preparing, separating, and purifying the compounds shown in formula (I).

### Pharmaceutical Compositions and Combination Drugs

The present disclosure provides a pharmaceutical composition, comprising a compound disclosed herein, such as the compounds in the examples. In a specific example of the disclosure, the pharmaceutical composition may further include a pharmaceutically acceptable excipient, wherein the excipient includes: a filler, a carrier, an adjuvant, a vehicle, or combinations thereof.

Additionally, the present disclosure provides a combination drug, comprising the pharmaceutical composition of the present disclosure and one or more therapeutic agents for treating diseases. The disclosure provides methods for treating, preventing, or ameliorating diseases or disorders, including administering a therapeutically effective amount of a combination drug comprising a compound disclosed herein and one or more therapeutic agents. The combination drug includes one or more other therapeutic agents for treating neurological or psychiatric diseases.

Other therapeutic agents for treating neurological and psychiatric diseases include but are not limited to: neuroprotective agents, neurotrophic agents, neurorestorative agents, B-vitamins, antidepressants, anxiolytics, lithium salts as mood stabilizers, antipsychotics, atypical antipsychotics, antiepileptics, antiparkinsonian agents, sedative-hypnotics, antihistamines, monoamine oxidase inhibitors, melatonin receptor agonists, non-opioid and opioid analgesics, or any combination thereof.

Other therapeutic agents for treating neurological and psychiatric diseases include but are not limited to: edaravone, butylphthalide, glutathione, vitamin C, vitamin E, dexmedetomidine, ketamine, propofol, midazolam, remimazolam, citalopram, B vitamins, methylcobalamin, nerve growth factor, gangliosides, levetiracetam, oxiracetam, piracetam, aniracetam, citicoline, donepezil, rivastigmine, galantamine, selegiline, memantine, brexpiprazole, amantadine, levodopa, carbidopa, entacapone, benserazide, bromocriptine, pramipexole, ropinirole, piribedil, cabergoline, apomorphine, lisuride, rotigotine, safinamide, rasagiline, istradefylline, pimavanserin, carbamazepine, oxcarbazepine, lamotrigine, phenytoin sodium, valproic acid, phenobarbital, ethosuximide, topiramate, lacosamide, paroxetine, venlafaxine, duloxetine, escitalopram, sertraline, alprazolam, mirtazapine, morphine, codeine, oxycodone, hydrocodone, fentanyl, pethidine, methadone, dextropropoxyphene, endorphin, dynorphin, chlorpromazine, haloperidol, or any combination thereof.

As used herein, the term "treatment" of any disease or disorder refers to all that may slow, interrupt, halt, control, or stop the progression of the disease or disorder, but does not necessarily mean the complete elimination of all symptoms of the disease or disorder. It also includes preventive treatment of the symptoms, particularly in patients susceptible to such disease or disorder. In some embodiments, "treatment" means ameliorating the disease or disorder (i.e., slowing, halting, or alleviating the progression of the disease or at least one of clinical symptoms thereof). In other embodiments, "treatment" means alleviating or ameliorating at least one physical parameter, including those that may not be discernible by the patient. In other embodiments, "treatment" refers to regulating the disease or disorder physically (e.g., stabilization of discernible symptoms) or physiologically (e.g., stabilization of physical parameters), or both. In other embodiments, "treatment" refers to preventing or delaying the onset or development or progression of the disease or disorder.

As used herein, the term "therapeutically effective amount" or "therapeutically effective dose" refers to an amount of a compound disclosed herein that is capable of eliciting a biological or medical response in a subject (e.g., reducing or inhibiting enzyme or protein activity, ameliorating symptoms, alleviating discorders, slowing or delaying disease progression, or preventing diseases).

The dosage of the active ingredient in the pharmaceutical composition or combination drug of the present disclosure may be varied. However, the amount of the active ingredient must be such that an appropriate dosage form can be obtained. The active ingredient can be administered to subjects (animals and humans) in need of such treatment in doses that provide optimal therapeutic efficacy. The selected dosage depends on the desired therapeutic effect, the route of administration, and the duration of treatment. Dosage will vary among patients, depending on nature and severity of diseases, the weight and specific diet of a subject, concomitant medications, and other factors recognized by those skilled in the art. The dosage range is typically about 0.5 mg to 1.0 g per subject per day, administered as a single or multiple doses. In one embodiment, the dosage range is about 0.5 mg to 500 mg per subject per day; in another embodiment, the dosage range is about 0.5 mg to 200 mg per subject per day; and still yet another embodiment, the dosage range is about 5 mg to 50 mg per subject per day.

It should also be appreciated that certain compounds of the present disclosure may exist in free form for treatment, or, where appropriate, may exist in the form of pharmaceutically acceptable derivatives. Pharmaceutically acceptable derivatives include pharmaceutically acceptable prodrugs, salts, esters, salts of such esters, or any additional adducts or derivatives that, upon administration to a subject in need thereof, directly or indirectly provide the compounds of the present disclosure or their metabolites or residues.

The pharmaceutical compositions or combination drugs disclosed herein may be prepared and packaged in bulk form, from which a safe and effective amount of the compound of formula (I) may be extracted and then administered to a subject in powder or syrup form. Typically, a patient is administered at dosage levels between 0.0001 and 10 mg/kg body weight per day to achieve effective antagonism of the orexin receptor. Alternatively, the pharmaceutical compositions disclosed herein may be prepared and packaged as unit dosage forms, wherein each physically discrete unit contains a safe and effective amount of a compound of formula (I). When prepared as unit dosage forms, the pharmaceutical compositions disclosed herein may typically contain, for example, 0.5 mg to 1 g, or 1 mg to 700 mg, or 5 mg to 100 mg of the compound of the disclosure.

When the combination drug of the present disclosure contains one or more other active ingredients in addition to the compound of the present disclosure, the weight ratio of the compound of the present disclosure to the second active ingredient may vary and depends on the effective dose of each ingredient. Typically, an effective dose of each is used. Thus, for example, when the compound of the present disclosure is mixed with another pharmaceutical agent, the weight ratio of the compound of the present disclosure to another pharmaceutical agent is typically in the range of about 1000:1 to about 1:1000, for example about 200:1 to about 1:200. Mixtures of the compound of the present disclosure with other active ingredients are also typically within the above range, but in each case, the effective dose of each active ingredient should be used.

The term "excipient" as used herein refers to pharmaceutically acceptable materials, mixtures, or vehicles that are associated with the consistency of the dosage form or pharmaceutical composition. Each excipient must be compatible with the other components of the pharmaceutical composition when mixted to avoid interactions that would significantly reduce the efficacy of the disclosed compounds when administered to a subject and to prevent interactions that would result in a pharmaceutically unacceptable pharmaceutical composition. Furthermore, each excipient must be pharmaceutically acceptable, such as possessing sufficiently high purity.

Suitable excipients will vary depending on the specific dosage form selected. Furthermore, excipients may be selected based on their specific function that they may serve in the composition. For example, certain excipients may be chosen for their ability to facilitate the production of uniform dosage forms; certain excipients may be chosen for their ability to facilitate the production of stable dosage forms; certain excipients may be chosen for their ability to facilitate the carrying or delivering the compound of the present disclosure from one organ, or part of the body, to another organ, or part of the body during administration to the subject; and certain excipients may be chosen for their ability to enhance subject compliance.

Suitable excipients include the following types: diluents, fillers, binders, disintegrants, lubricants, glidants, granulating agents, coating agents, wetting agents, solvents, cosolvents, suspending agents, emulsifiers, sweeteners, flavoring agents, taste-masking agents, colorants, anti-caking agents, humectants, chelating agents, plasticizers, thickeners, antioxidants, preservatives, stabilizers, surfactants, and buffering agents. Those skilled in the art will recognize that certain excipients may serve more than one function and may serve alternative functions, depending on the amount of the excipient present in the formulation and which other excipients are present in the formulation.

The pharmaceutical compositions of the present disclosure include those suitable for oral, nasal, topical, buccal, sublingual, rectal, and/or parenteral administration. They are typically formulated into dosage forms suitable for administering to a subject via the desired route. For example, dosage forms include injections, tablets, capsules, caplets, pills, lozenges, powders, syrups, tinctures, suspensions, solutions, emulsions, sachets, cachets, transdermal patches, suppositories, aerosols, solutions, dry powders, creams, ointments, lotions, pastes, sprays, foams, gels, and the like.

### Uses

The compounds, pharmaceutical compositions, or combination drugs of the present disclosure may be used for the preparation of a medicament for treating, preventing, ameliorating, controlling, or alleviating neurological and psychiatric diseases in mammals, particularly humans.

The compounds or pharmaceutical compositions of the disclosure may be used to prevent, treat, or alleviate neurological and psychiatric diseases; wherein the neurological diseases include: brain injury, central nervous system injury, peripheral nerve injury, neurodegenerative diseases, and hereditary neurological dysfunctions, preferably brain injury, and more preferably traumatic brain injury.

As used herein, the term "subject" refers to an animal. Typically, the animal is a mammal. A subject also refers to for example, primates (e.g., humans, male or female), cows, sheep, goats, horses, dogs, cats, rabbits, rats, mice, fish, birds and the like. In certain embodiments, the subject is a primate. In yet other embodiments, the subject is a human.

The subjects of the compounds, pharmaceutical compositions, or combination drugs of the present disclosure are animals. Typically, the animal is a mammal. Subjects, for example, also refer to primates (e.g., humans, male or female), cows, sheep, goats, horses, dogs, cats, rabbits, rats, mice, fish, birds, and the like. In certain embodiments, the subject is a primate. In other embodiments, the subject is a human.

The term "patient" in the present disclosure refers to humans (including adults and children) or other animals. In some embodiments, "patient" refers to humans.

Besides benefit for human treatment, the compounds, pharmaceutical compositions, or combination drugs of the present disclosure may also be used for veterinary treatment of pets, imported species and farm animals, including mammals. Other examples of animals include horses, dogs and cats. Herein, the compounds of the present disclosure include their pharmaceutically acceptable derivatives.

### Methods

In one embodiment, the therapeutic methods disclosed herein involve administering the compound of the disclosure, pharmaceutical composition containing the disclosed compound, or combination drug containing the compound of the disclosure to a subject in need thereof. The various embodiments disclosed herein include methods for treating the aforementioned diseases by administering a safe and effective amount of the disclosed compound, pharmaceutical composition containing the disclosed compound, or combination drug containing the disclosed compound to a subject in need thereof.

In one embodiment, the disclosed compound, the pharmaceutical composition containing the disclosed compound, or the combination drug containing the disclosed compound may be administered via any suitable route of administration, including systemic and local administration. Systemic administration includes oral administration, parenteral administration, transdermal administration, and rectal administration. Typical parenteral administration refers to administration by injection or infusion, including intravenous, intramuscular, and subcutaneous injection or infusion. Topical administration includes application to the skin, as well as intraocular, auricular, intravaginal, inhalation, and intranasal administration. In one embodiment, the disclosed compound, pharmaceutical composition containing the disclosed compound, or combination drug containing the disclosed compound may be administered orally. In another embodiment, the disclosed compound, pharmaceutical composition containing the disclosed compound, or combination drug containing the disclosed compound may be administered by inhalation. In yet another embodiment, the disclosed compound, pharmaceutical composition containing the disclosed compound, or combination drug containing the disclosed compound may be administered intranasally.

In one embodiment, the disclosed compound, pharmaceutical composition containing the disclosed compound, or combination drug containing the disclosed compound may be administered as a single dose or multiple doses at different intervals over a specified period according to an administration regimen. For example, it may be administered once, twice, three times, or four times per day. In one embodiment, it is administered once per day. In another embodiment, it is administered twice per day. Administration may continue until the desired therapeutic effect is achieved or indefinitely to maintain the desired therapeutic effect. The appropriate administration regimen for the disclosed compound, pharmaceutical composition containing the disclosed compound, or combination drug containing the disclosed compound depends on pharmacokinetic properties of the compound, such as absorption, distribution, and half-life, which can be determined by those skilled in the art. Furthermore, the appropriate administration regimen for the disclosed compound, pharmaceutical composition containing the disclosed compound, or combination drug containing the disclosed compound, including the duration of the regimen, depends on factors within the knowledge and experience of a person skilled in the art, such as the diseases being treated, the severity of the diseases, the age and physical condition of the patients being treated, the medical history of the patients being treated, the nature of concomitant therapies, and the desired therapeutic effect. Such a person skilled in the art should also understand that adjustment to the administration regimen may be required based on an individual patient's response to the regimen or as the individual patient's needs change over time.

The compound disclosed herein may be administered concurrently with, prior to, or subsequent to one or more other therapeutic agents. The compound disclosed herein may be administered separately from other therapeutic agents via the same or different routes of administration.

For individuals weighing about 50-70 kg, the pharmaceutical composition and combination drug disclosed herein may be in unit dosage form containing about 1-1000 mg, or about 1-500 mg, or about 1-250 mg, or about 1-150 mg, or about 0.5-100 mg, or about 1-50 mg of active ingredient. The therapeutically effective amount of the compound, pharmaceutical composition, or combination drug depends on the species, body weight, age, and individual condition, as well as the disorder or disease being treated and its severity. A physician, clinician, or veterinarian possessing ordinary skill can readily determine the effective amount of each active ingredient required to prevent, treat, or inhibit the progression of the disorder or disease.

In one embodiment, the present disclosure provides a method for activating the TrkB receptor on neurons, comprising: providing neurons having the TrkB receptor; and contacting the neurons with the aforementioned compound, pharmaceutical composition, or combination drug. The compound disclosed herein is small-molecule agonist of the TrkB receptor, which can effectively activate the TrkB receptor, thereby promoting neuronal survival, enhancing neuroplasticity, facilitating neuroregeneration, and repairing nerve damage, and the like.

### General Synthesis Steps

To describe the present disclosure, examples are provided below. However, it should be understood that the disclosure is not limited to these examples, which merely provide methods for practicing the disclosure.

Generally, the compound of the present disclosure may be prepared by the methods described herein, unless otherwise specified, wherein the definitions of substituents are as shown in formula (I). The reaction schemes and examples below are provided to further illustrate the content of the present disclosure.

Those skilled in the art will recognize that the chemical reactions described herein may be used to suitably prepare many other compounds of the present disclosure, and other methods for preparing the compounds of the present disclosure are considered to be within the scope of the present disclosure. For example, the synthesis of non-illustrative compounds of the present disclosure may be successfully accomplished by those skilled in the art through modifying the methods, such as employing appropriate protecting groups, utilizing other known reagents besides those described herein, or making routine modifications to reaction conditions. Furthermore, the reactions disclosed herein or known reaction conditions are also recognized as applicable to the preparation of other compounds of the present disclosure.

A method for preparing modified flavone compounds as shown in general formula I is provided.

In one embodiment, the modified flavone compounds as shown in General Formula I of the present disclosure can be obtained via the following reaction.

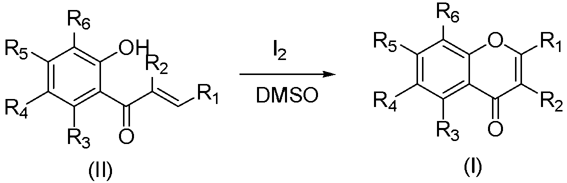

This reaction primarily involves dissolving compound II in dimethyl sulfoxide, followed by iodine-catalyzed aromatization.

In one embodiment, the modified flavone compounds as shown in General Formula I of the present disclosure may also be obtained via the following reaction.

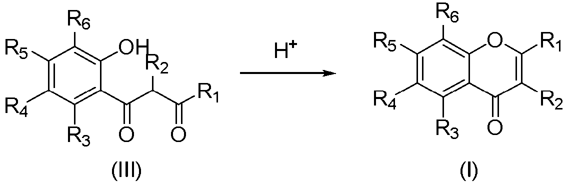

This reaction primarily involves the ring-closing of compound III under the action of an acid, characterized in that the acid used is an organic acid, an inorganic acid, or a combination thereof. Further, the organic acids are selected from formic acid, acetic acid, propionic acid, trifluoroacetic acid, methanesulfonic acid, ethylsulfonic acid, benzenesulfonic acid, and p-toluenesulfonic acid. The inorganic acids are selected from concentrated hydrochloric acid, concentrated sulfuric acid, hydrobromic acid, hydroiodic acid, nitric acid, and phosphoric acid. The combination of the acids is selected from the mixed systems of acetic acid/concentrated hydrochloric acid, acetic acid/concentrated sulfuric acid, trifluoroacetic acid/concentrated hydrochloric acid, and trifluoroacetic acid/concentrated sulfuric acid. Furthermore, the acid used is the mixed system of acetic acid/concentrated sulfuric acid.

The present disclosure will now be described in detail with reference to the following examples. Those skilled in the art will appreciate that the examples are merely intended to illustrate the disclosure and should not be construed as limiting the scope of the present invention. In the examples where no specific techniques or conditions are indicated, the process shall be performed according to the techniques or conditions described in the literature in the art, or according to the product specifications. For the reagents or instruments used without indicating the manufacturer, they are all conventional products commercially available.

### Example 1 Synthesis of Compound S1

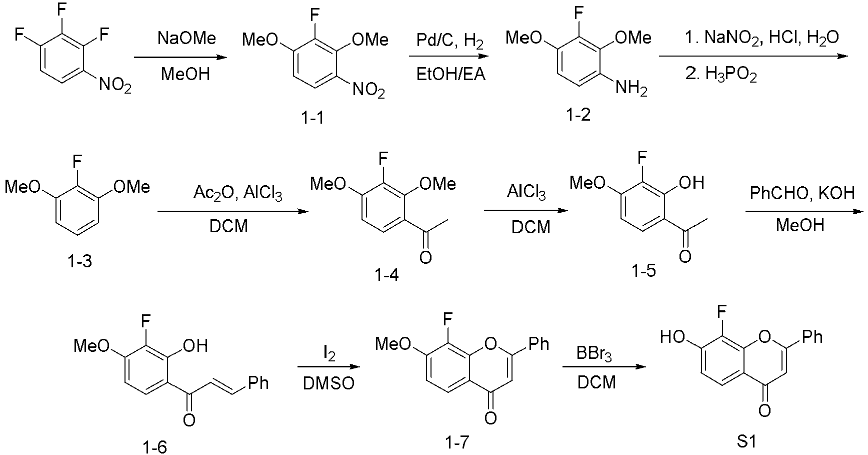

### Synthesis of Intermediate 1-1

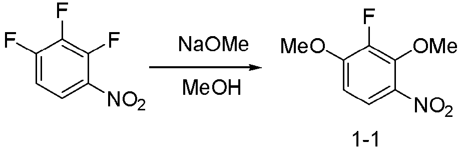

2,3,4-Trifluoronitrobenzene (250 g, 1.41 mol) was dissolved in 1.4 L of methanol. Under stirring, a 30% sodium methoxide in methanol solution (585.22 g) was added dropwise, with the temperature controlled below 50°C. After completing the addition of the sodium methoxide solution, the mixture was stirred overnight. Citric acid (320 g) was dissolved in 2.8 L of water to prepare a solution. Upon TLC confirmation of reaction completion, the prepared citric acid solution was poured into the reaction mixture. After stirring for 20 minutes, vacuum filtration was performed. The filter cake was washed with water and dried to yield 293.90 g of pale yellow solid.

¹H NMR (400 MHz, CDCl₃) *δ* 7.72 (dd, *J* = 9.5,2.2 Hz, 1H), 6.71 (dd, *J* = 9.4, 7.5 Hz, 1H), 4.08 (s, 3H), 3.95 (s, 3H).

### Synthesis of Intermediate 1-2

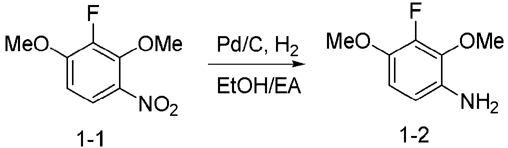

Intermediate 1-1 (9.1 g, 42.24 mmol) was dissolved in a solvent of ethanol and ethyl acetate. Pd/C (1 g) was added, and the mixture was stirred overnight under a hydrogen atmosphere at 1 atm. After TLC confirmation of reaction completion, Pd/C was filtered off. The filtrate was evaporated under reduced pressure to remove the solvent. Ethyl acetate was added to the residue. After solid precipitation, the solid was removed by filtration. The solvent in the filtrate was evaporated under reduced pressure to afford 4.1 g of yellow liquid.

¹H NMR (400 MHz, CDCl₃) *δ* 6.52 (t, *J* = 8.7 Hz, 1H), 6.40 (dd, *J =* 8.8, 2.1 Hz, 1H), 3.92 (s, 3H), 3.80 (s, 3H)

### Synthesis of Intermediate 1-3

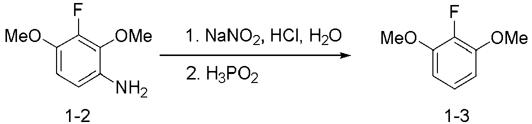

Intermediate 1-2 (3.88 g, 22. 67 mmol) was dissolved in 60 mL of water and 30 mL of concentrated hydrochloric acid was added. The mixture was stirred below 0°C. Sodium nitrite (3.12 g, 45.34 mmol) was dissolved in 20 mL of water and then added to the reaction solution. After stirring at around 0°C for 30 minutes, 29 mL of hypophosphorous acid was added dropwise. After complete addition of the hypophosphorous acid, stirring continued overnight. Upon TLC confirmation of reaction completion, pH was adjusted to approximately 8 using a 10% NaOH solution. The mixture was then extracted with 200 mL of dichlorohexane. The organic phase was dried over anhydrous sodium sulfate and the solid was filtered off. The solvent in the filtrate was evaporated under reduced pressure to afford 4 g of an oily substance.

¹H NMR (400 MHz, DMSO-d₆) δ 7.10 - 7.01 (m, 1H), 6.77 (dd, *J* = 8.5, 7.5 Hz, 2H), 3.83 (s, 6H).

¹³C NMR (100 MHz, DMSO-d⁶) δ 148.50 (d, *J =* 7.9 Hz), 142.04 (d, *J* = 242.2 Hz), 124.02 (d, *J =* 5.3 Hz), 106.36, 56.53.

¹⁹F NMR (376 MHz, DMSO-d⁶) δ -159.14.

### Synthesis of Intermediate 1-4

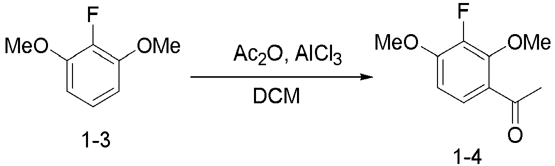

Intermediate 1-3 (3.9 g, 24.97 mmol) was dissolved in dichloromethane and approximately 6.66 g of aluminum trichloride (49.95 mmol) was added at around 0°C. After stirring for 30 minutes, 2.8 g of acetic anhydride (27.47 mmol) was added, and the reaction was continued under stirring. Upon TLC confirmation of reaction completion, the reaction was quenched with water. The mixture was separated, and the organic phase was washed with water, then dried over anhydrous sodium sulfate. The solid was filtered off, and the solvent in the filtrate was evaporated under reduced pressure to afford 3.65 g of white solid.

¹H NMR (400 MHz, CDCl₃) δ 7.56 (dd, *J* = 9.0, 2.2 Hz, 1H), 6.73 (dd, *J* = 8.9, 7.3 Hz, 1H), 4.03 (d, *J* = 2.3 Hz, 3H), 3.93 (s, 3H), 2.60 (s, 3H).

¹³C NMR (100 MHz, CDCl₃) δ 197.12 (d, *J* = 2.6 Hz), 152.51 (d, *J* = 9.3 Hz), 148.81 (d, *J =* 8.8 Hz), 145.43 (d, *J* = 246.6 Hz), 125.16 (d, *J =* 4.4 Hz), 107.06 (d, *J=* 1.4 Hz), 61.62 (d, *J* = 7.4 Hz), 56.42, 31.06.

¹⁹F NMR (376 MHz, DMSO-*d₆*) δ -152.69.

### Synthesis of intermediate 1-5

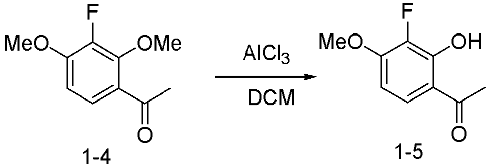

Intermediate 1-4 (6.25 g, 31.53 mmol) was dissolved in 150 mL of dichloromethane and aluminum trichloride (6.3 g, 47.30 mmol) was added at around 0°C. The reaction was stirred at room temperature. Upon TLC confirmation of reaction completion, the reaction was quenched with water and concentrated hydrochloric acid. The mixture was separated, and the organic phase was washed with water and dried over anhydrous sodium sulfate. The solid was filtered off, and the solvent in the filtrate was evaporated under reduced pressure to afford 5.48 g of a white solid.

¹H NMR (400 MHz, DMSO-*d₆*) δ 12.32 (s, 1H), 7.75 (dd, *J =* 9.1, 2.1 Hz, 1H), 6.79 (dd, *J =* 9.1, 7.5 Hz, 1H), 3.94 (s, 3H), 2.61 (s, 3H).

¹³C NMR (100 MHz, *DMSO-d₆*) δ 204.21 (d, *J* = 2.2 Hz), 153.44 (d, *J* = 7.4 Hz), 150.70 (d, *J* = 9.8 Hz), 140.04 (d, *J* = 241.7 Hz), 128. 04 (d, *J =* 4.1 Hz), 115.74 (d, *J* = 1.8 Hz), 104.35, 56.99, 27.37.

¹⁹F NMR (376 MHz, DMSO-_{d6}) δ -161.60.

### Synthesis of Intermediate 1-6

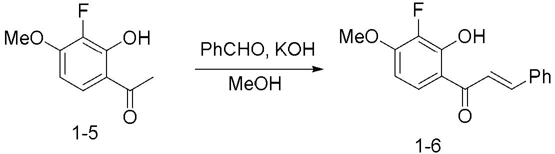

Intermediate 1-5 (5.48 g, 29.76 mmol) was dissolved in 70 mL of methanol, and potassium hydroxide (5.84 g, 104.16 mmol) and benzaldehyde (3.16 g, 29.76 mmol) were added. The reaction was stirred under reflux. Upon TLC confirmation of reaction completion, the pH was adjusted to approximately 3 with concentrated hydrochloric acid. The mixture was then subjected to vacuum filtration, and the filter cake was dried to afford 10.75 g of a yellow solid.

¹H NMR (400 MHz, DMSO-*d₆*) δ 13.07 (s, 1H), 8.21 (dd, *J =* 9.2, 1.9 Hz, 1H), 8.04 (d, *J* = 15.5 Hz, 1H), 7.97-7.90 (m, 2H), 7.86 (d, *J* = 15.5 Hz, 1H), 7.54-7.45 (m, 3H), 6. 85 (dd, *J =* 9.1, 7.5 Hz, 1H), 3.98 (s, 3H).

¹³C NMR (100 MHz, DMSO-*d6*) δ 193.05 (d, *J* = 2.3 Hz), 153.75 (d, *J* = 7.4 Hz), 152.03 (d, *J* = 9.8 Hz), 145.36, 140.22 (d, *J* = 241.8 Hz), 134.86, 131.50, 129.72, 129.42, 127.73 (d, *J* = 4.1 Hz), 121.57, 115.99 (d, *J* = 2.0 Hz), 104.45, 57.13.

¹⁹F NMR (376 MHz, DMSO-*d₆*) δ -161.20.

### Synthesis of Intermediate 1-7

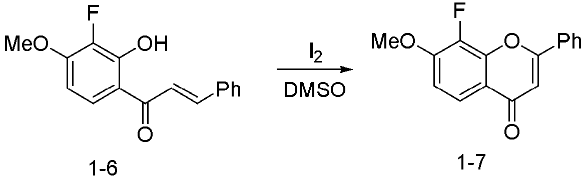

Intermediate 1-6 (10.75 g, 39.48 mmol) was dissolved in 70 mL of dimethyl sulfoxide. 1.5 g iodine (5.92 mmol) was added, and the mixture was stirred at 110°C. Upon TLC confirmation of reaction completion, saturated sodium thiosulfate solution was added to precipitate a solid. Vacuum filtration was then performed, and the filter cake was dried to afford 2.8 g of a yellow solid.

¹H NMR (400 MHz, DMSO-*d₆*) δ 8.43-7.16 (m, 7H), 6.99 (s, 1H), 4.01 (s, 3H).

¹³C NMR (100 MHz, DMSO-*d₆*) δ 176.31, 162.50, 151.55, 145.38, 140.10 (d, *J* = 248.8 Hz), 132.32, 131.32, 129.63, 126.64, 120.73, 118.23, 111.58, 107.20, 57.36.

¹⁹F NMR (376 MHz, DMSO-*d₆*) δ -157.47.

### Synthesis of Compound S1

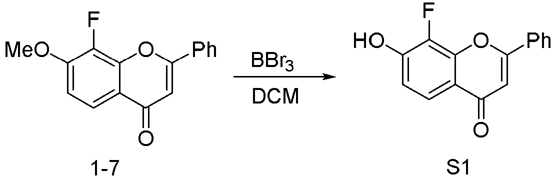

Intermediate 1-7 (1.64 g, 6.1 mmol) was dissolved in 100 mL of dichloromethane. Boron tribromide (5.2 g, 20.76 mmol) was added, and the mixture was stirred at room temperature. Upon TLC confirmation of reaction completion, the reaction was quenched with water, resulting in the precipitation of a solid. Vacuum filtration was then performed, and the filter cake was dried to afford 1.16 g of a white solid.

¹H NMR (400 MHz, DMSO-*d₆*) δ 11.26 (s, 1H), 8.08-8.01 (m, 2H), 7.70 (dd, *J* = 8.9, 1.9 Hz, 1H), 7.65-7.56 (m, 3H), 7.11 (dd, *J* = 8.9, 7.4 Hz, 1H), 6.96 (s, 1H).

¹³C NMR (100 MHz, DMSO-*d₆*) δ 176.32 (d, *J* = 2.6 Hz), 162.21, 150.32 (d, *J* = 8.4 Hz), 146.18 (d, *J* = 8.3 Hz), 139.42 (d, *J* = 245.4 Hz), 132.20, 131.46, 129.61, 126.58, 120.61 (d, *J* = 4.1 Hz), 117.18, 115.83 (d, *J* = 2.1 Hz), 107.23.

¹⁹F NMR (376 MHz, DMSO-*d⁶*) δ -159.34.

### Example 2 Synthesis of Compound S2

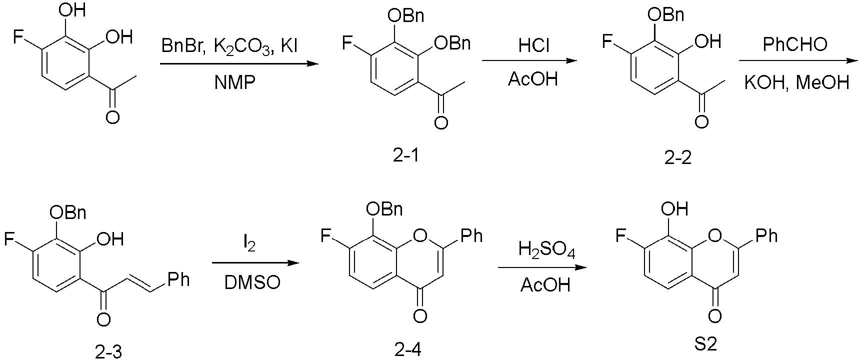

### Synthesis of Intermediate 2-1

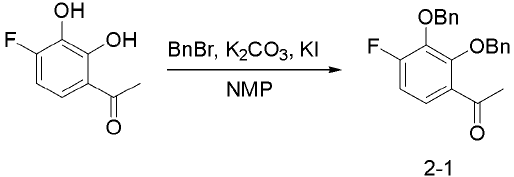

Starting material 1-(4-fluoro-2,3-dihydroxyphenyl)ethan-1-one (85.07 g, 500 mmol) was dissolved in 500 mL of N-methylpyrrolidone. Potassium carbonate (241.86 g), potassium iodide (12.45 g), and water (120 mL) were added, followed by benzyl bromide (299.31 g). The reaction proceeded at 30°C. Upon TLC confirmation of reaction completion, 2.5 L water was added to quench the reaction, resulting in the precipitation of a solid. Vacuum filtration was then performed, and the filter cake was washed with ethanol and dried to afford 148.80 g of a yellow solid.

### Synthesis of Intermediate 2-2

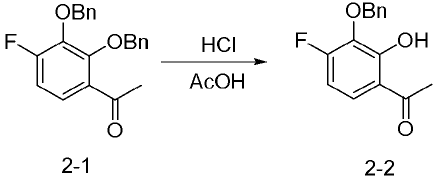

Intermediate 2-1 (148.80 g, 425 mmol) was dissolved in 420 mL of acetic acid, 42 mL of concentrated hydrochloric acid was added, and the mixture was stirred at room temperature. Upon confirmation of reaction completion by TLC, vacuum filtration was directly performed, and the filter cake was washed with ethanol, then dried to afford 99.48 g of a white solid.

### Synthesis of Intermediate 2-3

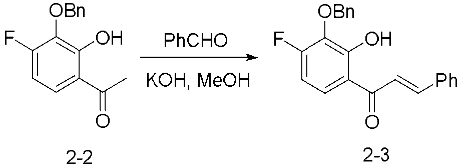

Intermediate 2-2 (99.48 g, 382.5 mmol) was dissolved in 2.4 L of methanol, then 145.55 g of potassium hydroxide and 64.40 g of benzaldehyde were added. The reaction was carried out under reflux. Upon TLC confirmation of reaction completion, the reaction solution was cooled to room temperature, and 196 mL of acetic acid was added to quench the reaction, resulting in the precipitation of a solid. The mixture was filtered under reduced pressure, and the filter cake was washed with methanol and dried to afford 123.83 g of a yellow solid.

### Synthesis of Intermediate S2

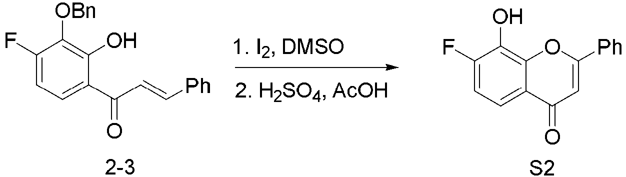

Intermediate 2-3 (123.83 g, 355.7 mmol) was dissolved in 500 mL of dimethyl sulfoxide. 9.86 g iodine was added, and the mixture was stirred at 110°C. Upon confirmation of reaction completion by TLC, the reaction mixture was cooled to room temperature and the reaction was quenched with saturated sodium thiosulfate solution, resulting in the precipitation of a solid. Vacuum filtration was performed, and the filter cake was dried to afford 102.19 g of a white solid.

The obtained solid was dissolved in 1.03 L of acetic acid, and 45.13 mL of concentrated sulfuric acid was added. The mixture was stirred at 80°C. Upon TLC confirmation of reaction completion, reaction solution was cooled to room temperature and directly filtered under reduced pressure. The filter cake was washed sequentially with acetic acid and water, and then recrystallized using a methanol/tetrahydrofuran/acetonitrile system. The solid was filtered and dried to afford 45.36 g of a yellow solid.

ES-MS m/z [M+1]⁺: 257.1.

### Example 3 Synthesis of Compound S3

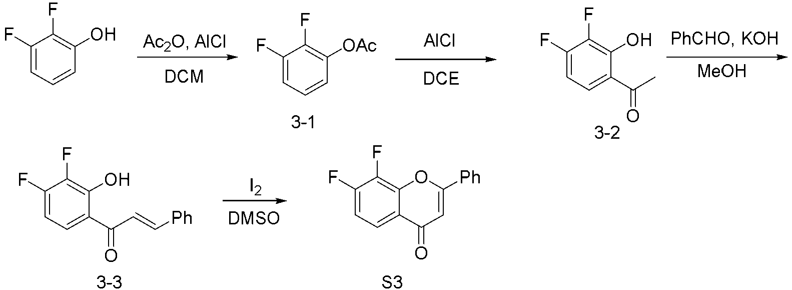

### Synthesis of Intermediate 3-1

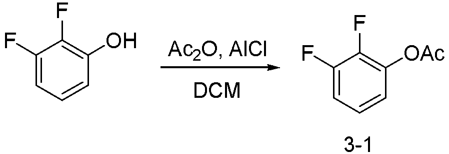

Starting material 2,3-difluorophenol (26 g, 199.86 mmol) was dissolved in 200 mL of dichloromethane. 53.3 g of aluminum trichloride was added at approximately 0°C, followed by 22.44 g of acetic anhydride. The reaction was then continued with stirring at 0°C. Upon TLC confirmation of reaction completion, the reaction was quenched with 200 mL water. The mixture was separated, and the organic phase was washed with water, and then dried over anhydrous sodium sulfate. The filtrate was concentrated under reduced pressure to afford 33.86 g of a liquid.

¹H NMR (400 MHz, DMSO-*d₆*) δ 7.30 (dddd, *J* = 10.3, 8.7, 7.0, 1.7 Hz, 1H), 7.22 (tdd, *J* = 8.3, 5.7, 2.0 Hz, 1H), 7.12 (ddt, *J* = 8.2, 6.5, 1.8 Hz, 1H), 2.34 (s, 3H).

¹³C NMR (100 MHz, DMSO-*d₆*) δ 168.25, 150.82 (dd, *J* = 246.8, 10.7 Hz), 143.01 (dd, *J* = 249.2, 14.2 Hz), 139.53 (dd, *J=* 9.9, 2.3 Hz), 124.58 (dd, *J* = 8.2, 4.9 Hz), 119.91 (d, *J=* 3.3 Hz), 115.13 (d, *J =* 17.1 Hz), 20.18.

¹⁹F NMR (376 MHz, DMSO-*d₆*) δ -137.56 (d, *J* = 21.1 Hz), -153.00 (d, *J* = 21.1 Hz).

### Synthesis of Intermediate 3-3

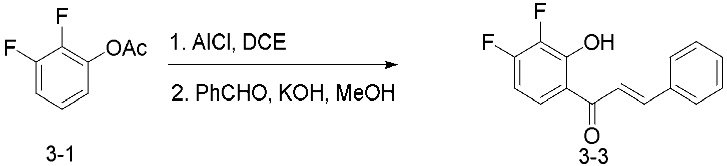

Intermediate 3-1 (33.86 g, 196.70 mmol) was dissolved in 170 mL of 1,2-dichloroethane. Aluminum trichloride (26.3 g) was added at room temperature, and the mixture was then heated to reflux. Upon TLC confirmation of reaction completion, the reaction was quenched with 170 mL water, and then 170 mL of 2 M hydrochloric acid was added. The mixture was separated, and the organic phase was washed with water and dried over anhydrous sodium sulfate. The filtrate was concentrated under reduced pressure to afford 25 g of a solid.

The obtained solid (25 g, 145.2 mmol) was dissolved in 250 mL of methanol, 28.5 g of potassium hydroxide and 15.4 g of benzaldehyde were added, and then the mixture was heated to reflux. Upon TLC confirmation of reaction completion, the reaction mixture was cooled to room temperature and the reaction was quenched with 45 mL of concentrated hydrochloric acid, followed by addition of 80 mL of water, resulting in the precipitation of a solid. The precipitate was filtered under reduced pressure, and the filter cake was dried to afford 35.3 g of a yellow solid.

¹H NMR (400 MHz, DMSO-*d₆*) δ 12.94 (s, 1H), 8.19 (ddd, *J* = 9.2, 6.0, 2.2 Hz, 1H), 8.00 (d, *J* = 15.6 Hz, 1H), 7.95-7.91 (m, 2H), 7.87 (d, *J* = 15.5 Hz, 1H), 7.54-7.44 (m, 3H), 7.16-7.03 (m, 1H).

¹³C NMR (100 MHz, DMSO-*d₆*) δ 193.10 (d, *J =* 2.0 Hz), 154.27 (dd, *J =* 254.0, 9.5 Hz), 152.28 (dd, *J =* 7.8, 3.7 Hz), 146.16, 139.66 (dd, *J* = 244.8, 14.1 Hz), 134.71, 131.71, 129.80, 129.45, 127.39 (dd, *J* = 10.1, 4.1 Hz), 122.03, 120.02, 107.83 (d, *J* = 18.1 Hz).

¹⁹F NMR (376 MHz, DMSO-*d₆*) δ -127.07 (d, *J* = 20.9 Hz), -161.51 (d, *J* = 20.8 Hz).

### Synthesis of Compound S3

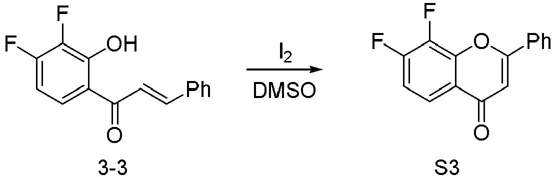

Intermediate 3-3 (35.3 g, 135.6 mmol) was dissolved in 300 mL of dimethyl sulfoxide. 5.2 g of iodine was added, and the mixture was stirred at 110°C. Upon TLC confirmation of reaction completion, the reaction mixture was cooled to room temperature and the reaction was quenched with saturated sodium thiosulfate solution, resulting in the precipitation of a solid. The precipitate was filtered under reduced pressure, and the filter cake was dried to afford 23 g of a white solid.

¹H NMR (400 MHz, DMSO-*d₆*) δ 8.13-8.02 (m, 2H), 7.88 (ddd, *J* = 9.1, 5.7, 2.2 Hz, 1H), 7.70-7.53 (m, 4H), 7.13 (s, 1H).

¹³C NMR (101 MHz, DMSO-*d₆*) δ 176.00 (d, *J* = 2.2 Hz), 163.05, 152.95 (dd, *J* = 252.1, 9.2 Hz), 145.94 (dd, *J* = 8.2, 4.7 Hz), 139.61 (dd, *J* = 253.2, 15.3 Hz), 132.61, 130.92, 129.69, 126.73, 121.69 (d, *J* = 2.0 Hz), 121.16 (dd, *J* = 9.3, 4.5 Hz), 114.78 (d, *J* = 18.8 Hz), 107.62.

¹⁹F NMR (376 MHz, DMSO-*d6*) δ -129.89 (d, *J* = 20.5 Hz), -157.60 (d, *J* = 20.5 Hz).

### Example 4 Synthesis of Compound S4

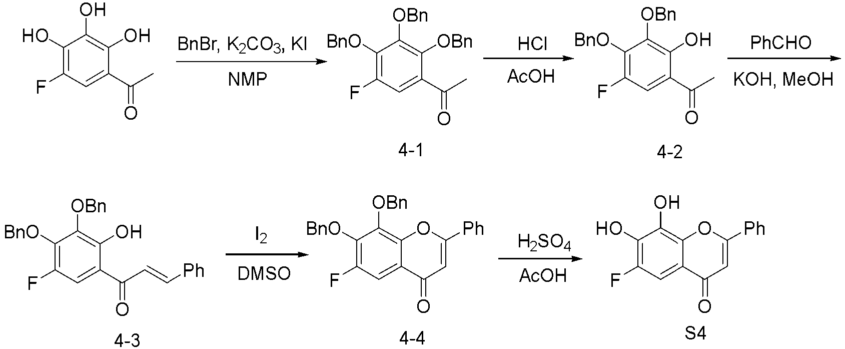

### Synthesis of Intermediate 4-1

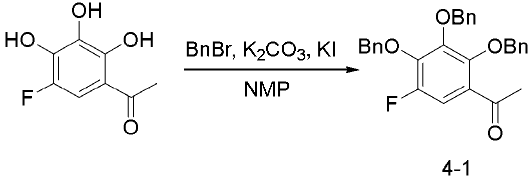

Starting material 1-(5-fluoro-2,3,4-trihydroxyphenyl)ethan-1-one (93.01 g, 500 mmol) was dissolved in 500 mL of N-methylpyrrolidone. Potassium carbonate (241.86 g), potassium iodide (12.45 g), and water (120 mL) were added, followed by benzyl bromide (299.31 g). The reaction proceeded at 30°C. Upon TLC confirmation of reaction completion, 2.5 L water was added to quench the reaction, resulting in the precipitation of a solid. The precipitate was filtered under reduced pressure, and the filter cake was washed with ethanol and dried to afford 196.15 g of a yellow solid.

### Synthesis of Intermediate 4-2

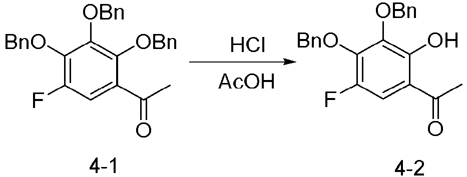

Intermediate 4-1 (196.15 g, 430 mmol) was dissolved in 420 mL of acetic acid. 42 mL of concentrated hydrochloric acid was added, and the mixture was stirred at room temperature. Upon confirmation of reaction completion by TLC, the mixture was filtered directly under reduced pressure. The filter cake was washed with ethanol and dried to afford 133.82 g of a white solid.

### Synthesis of Intermediate 4-3

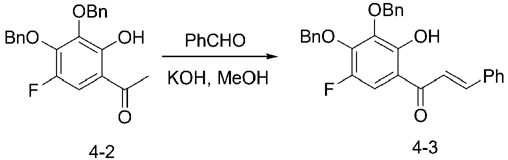

Intermediate 4-2 (133.82 g, 365.5 mmol) was dissolved in 2.4 L of methanol. Potassium hydroxide (145.55 g) and benzaldehyde (64.40 g) were added, and the mixture was heated to reflux. Upon TLC confirmation of reaction completion, the mixture was cooled to room temperature. Acetic acid (196 mL) was added to quench the reaction, resulting in the precipitation of a solid. The precipitate was filtered under reduced pressure. The filter cake was washed with methanol and dried to afford 149.39 g of a yellow solid.

### Synthesis of Intermediate S4

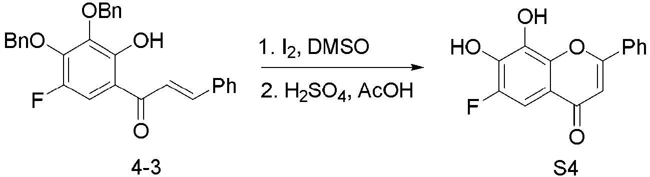

Intermediate 4-3 (149.39 g, 328.9 mmol) was dissolved in 500 mL of dimethyl sulfoxide. 9.86 g of iodine was added, and the mixture was stirred at 110°C. After TLC confirmed reaction completion, the reaction mixture was cooled to room temperature and the reaction was quenched with saturated sodium thiosulfate solution, resulting in the precipitation of a solid. The precipitate was filtered under reduced pressure, and dried to afford 123.43 g of a white solid.

The obtained solid was dissolved in 1.03 L of acetic acid and 45.13 mL of concentrated sulfuric acid was added. The reaction was carried out with stirring at 80°C. Upon TLC confirmation of reaction completion, the reaction mixture was cooled to room temperature and directly filtered under reduced pressure. The filter cake was washed successively with acetic acid and water, then recrystallized from a methanol/tetrahydrofuran/acetonitrile system. The precipitate was filtered and dried to afford 48.27 g of a yellow solid.

ES-MS m/z [M+1]⁺: 273.1.

### Example 5 Synthesis of Compound S5

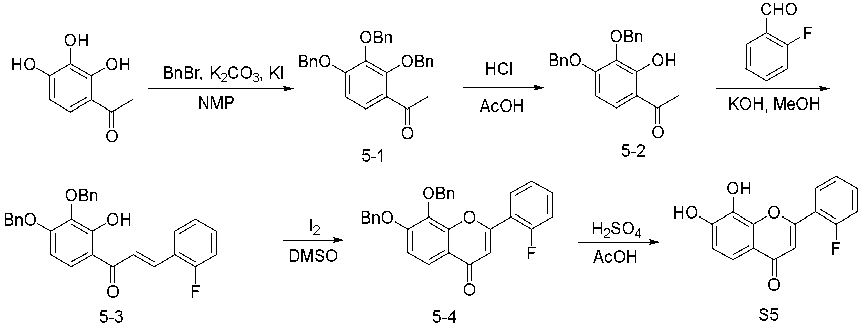

### Synthesis of Intermediate 5-1

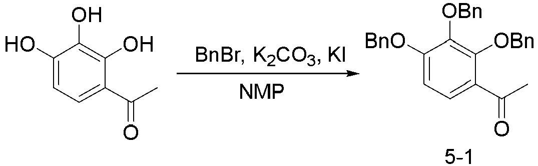

2,3,4-Trihydroxyacetophenone (84.07 g, 500 mmol) was dissolved in 500 mL of N-methylpyrrolidone. 241.86 g of potassium carbonate, 12.45 g of potassium iodide, and 120 mL of water were added, followed by 299.31 g of benzyl bromide. The reaction proceeded at 30°C. Upon TLC confirmation of reaction completion, 2.5 L of water was added to quench the reaction, resulting in the precipitation of a solid. The precipitate was filtered under reduced pressure, washed with ethanol, and dried to afford 184.58 g of a yellow solid.

¹H NMR (400 MHz, CDCl₃) δ 7.50 (d, *J* = 8.9 Hz, 1H), 7.46-7.31 (m, 12H), 7.31-7.25 (m, 3H), 6.81 (d, *J =* 8.9 Hz, 1H), 5.15 (s, 4H), 5.05 (s, 2H), 2.52 (s, 3H).

### Synthesis of Intermediate 5-2

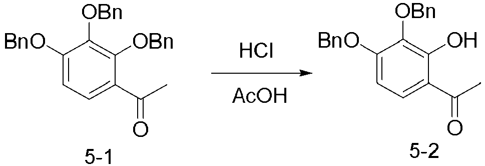

Intermediate 5-1 (184.58 g, 420.91 mmol) was dissolved in 420 mL of acetic acid. 42 mL of concentrated hydrochloric acid was added, and the mixture was stirred at room temperature. Upon TLC confirmation of reaction completion, the reaction mixture was filtered directly under reduced pressure. The filter cake was washed with ethanol and dried to afford 129. 12 g of a white solid.

¹H NMR (400 MHz, DMSO-*d₆*) δ 12.57 (s, 1H), 7.70 (d, *J* = 9.1 Hz, 1H), 7.48-7.38 (m, 6H), 7.38-7.28 (m, 4H), 6.79 (d, *J =* 9.1 Hz, 1H), 5.25 (s, 2H), 4.97 (s, 2H), 2.58 (s, 3H).

### Synthesis of Intermediate 5-3

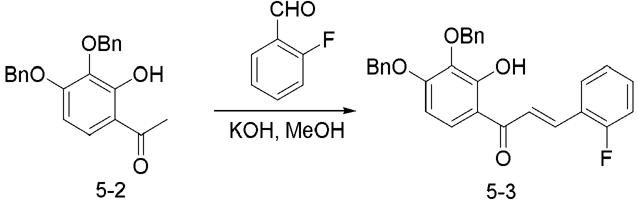

Intermediate 5-2 (129.12 g, 370.61 mmol) was dissolved in 2.4 L of methanol. Potassium hydroxide (145.55 g) and o-fluorobenzaldehyde (64.40 g) were added, and the reaction was carried out under reflux. Upon TLC confirmation of reaction completion, the reaction mixture was cooled to room temperature. Acetic acid (196 mL) was added to quench the reaction, resulting in the precipitation of a solid. The precipitate was filtered under reduced pressure, washed with methanol, and dried to afford 122.76 g of a yellow solid.

¹H NMR (400 MHz, DMSO-*d₆*) δ 13.19 (s, 1H), 8.20-8.12 (m, 1H), 8.11-8.05 (m, 2H), 7.94 (d, *J* = 15.5 Hz, 1H), 7.57-7.28 (m, 13H), 6.83 (d, *J=* 9.1 Hz, 1H), 5.28 (s, 2H), 5.02 (s, 2H).

¹³C NMR (101 MHz, DMSO-*d₆*) δ 192.56, 161.43 (d, *J* = 251.6 Hz), 158.38, 138.05, 136.86, 135.57, 133.33 (d, *J =* 8.8 Hz), 129.64 (d, *J =* 2.2 Hz), 128.93, 128.75, 128.64, 128.53, 128.44, 128.28, 128.18, 127.89, 125.38 (d, *J* = 3.3 Hz), 123.87, 122.68 (d, *J* = 11.2 Hz), 121.14, 116.54 (d, *J* = 21.7 Hz), 115.76, 105.35, 74.39, 70.65.

¹⁹ F NMR (376 MHz, DMSO-*d₆*) δ -116.02.

### Synthesis of Compound S5

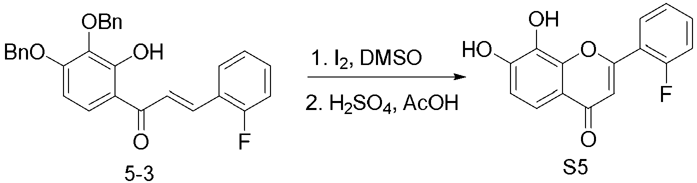

Intermediate 5-3 (117.26 g, 259.10 mmol) was dissolved in 500 mL of dimethyl sulfoxide, and 9.86 g of iodine was added. The reaction was carried out with stirring at 110°C. Upon confirmation of reaction completion by TLC, the reaction mixture was cooled to room temperature and the reaction was quenched with saturated sodium thiosulfate solution, resulting in the precipitation of a solid. After vacuum filtration, the filter cake was dried to afford 98. 07 g of white solid.

The obtained solid was dissolved in 1.03 L of acetic acid, and 45.13 mL of concentrated sulfuric acid was added. The reaction was carried out with stirring at 80°C. Upon TLC confirmation of reaction completion, the reaction mixture was cooled to room temperature and filtered directly under reduced pressure. The filter cake was sequentially washed with acetic acid and water, then recrystallized from a methanol/tetrahydrofuran/acetonitrile system. The solid was filtered and dried to afford 27.02 g of a yellow solid.

¹H NMR (400 MHz, DMSO-*d₆*) δ 10.41 (s, 1H), 9.48 (s, 1H), 7.44 (d, *J* = 8.4 Hz, 1H), 7.32-7.15 (m, 4H), 6.99 (d, *J* = 8.8 Hz, 1H), 6.54 (s, 1H).

¹³C NMR (100 MHz, DMSO-*d₆*) δ 177.13, 160.16 (d, *J* = 253.1 Hz), 157.88 (d, *J* = 3.6 Hz), 151.14, 147.29, 133.59, 130.06, 125.47 (d, *J* = 3.4 Hz), 121.08, 117.26 (d, *J = 4.6* Hz), 117.21, 115.66, 114.72, 112.78, 111.32.

¹⁹F NMR (376 MHz, DMSO-*d₆*) δ -112.04.

### Example 6 Synthesis of Compound S6

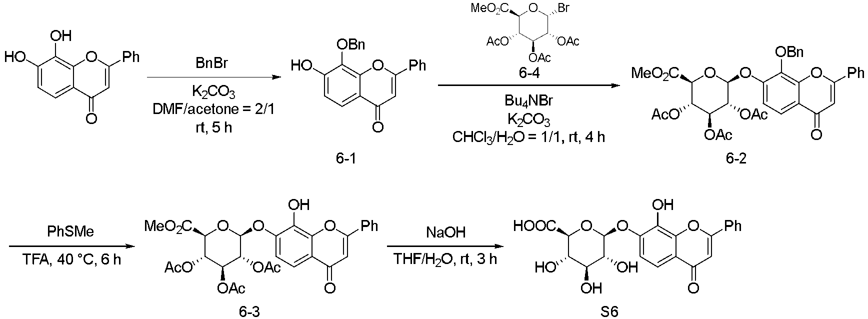

### Synthesis of Intermediate 6-1

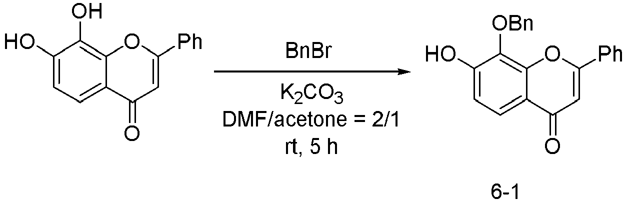

Starting material 7,8-DHF was dissolved in a mixed solvent of DMF and acetone (2:1). 3 equivalents of K₂CO₃ were added, followed by the dropwise addition of 1.1 equivalents of BnBr at room temperature under stirring. The reaction progress was monitored by TLC. The starting material could not be completely consumed, with the major byproduct being the dibenzyl-protected compound. After 5 hours, the reaction was completed. The crude product was obtained via extraction, drying, filtration, and concentration. The product was finally purified by column chromatography to afford the white solid product 6-1 in 39% yield.

### Synthesis of Intermediate 6-2

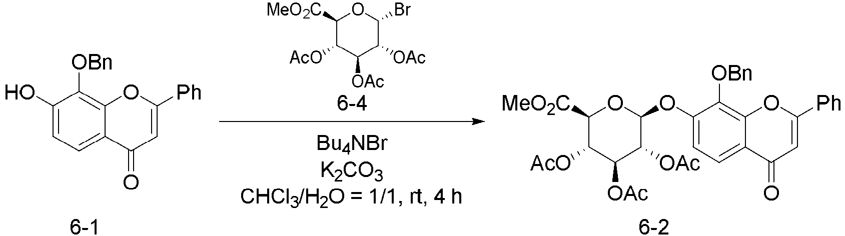

1 equivalent of starting material 6-1, 1.2 equivalents of Bu₄NBr, 5 equivalents of K₂CO₃, and 3 equivalents of 6-4 were dissolved in a mixed solvent of CHCl₃ and H₂O (1:1). The reaction was then carried out at room temperature for 4 hours, with reaction progress monitored by TLC. After the reaction was completed, the crude product was obtained by extraction, drying, filtration, and concentration. Finally, the product was purified by column chromatography to afford the white solid product 6-2 in 93% yield.

### Synthesis of Intermediate 6-3

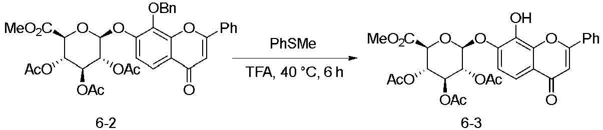

1 equivalent of starting material 6-2 was dissolved in a mixture of PhSMe (5 equiv), TFA (5 equiv), and DCM. The reaction was then carried out at 40°C for 6 hours, with progress monitored by TLC. After the reaction was completed, a saturated aqueous NaHCO₃ solution was added to adjust the pH to weakly alkaline. The crude product was obtained by extraction, drying, filtration, and concentration. Finally, the product was purified by column chromatography to afford the white solid product 6-3 in 71% yield.

### Synthesis of Compound S6

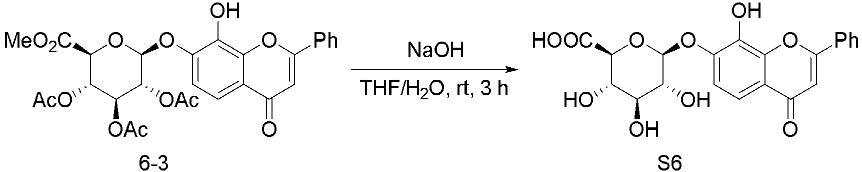

1 equivalent of starting material 6-3 was dissolved in a mixed solvent of THF and H₂O (2:1). 6 equivalents of NaOH were then added, and the reaction was carried out at room temperature for 3 hours, with reaction progress monitored by TLC. After the reaction was completed, excess acetic acid was added to adjust the pH to acidic. The crude product then was obtained by concentration, and finally purified by preparative column chromatography to afford the final product S6 in 80% yield.

¹H NMR (400 MHz, MeOH-*d₄*) δ 3.52-3.68 (m, 3H) 4.05 (d, J = 9.63 Hz, 1H) 5.11 (d, J = 7.75 Hz, 1H) 6.88 (s, 1H) 7.34 (d, J=9.01 Hz, 1H) 7.54-7.67 (m, 4H) 8.11 (dd, J=7.63, 2.00 Hz, 2H).

¹³C NMR (100 MHz, MeOH-*d₄*) δ 177.97, 170.02, 163.34, 146.97, 145.91, 136.78, 131.57, 131.50, 128.94, 126.33, 121.26, 116.11, 114.27, 105.88, 100.66, 72.52, 71.10, 68.83, 53.17.

### Example 7 Synthesis of Compound S7

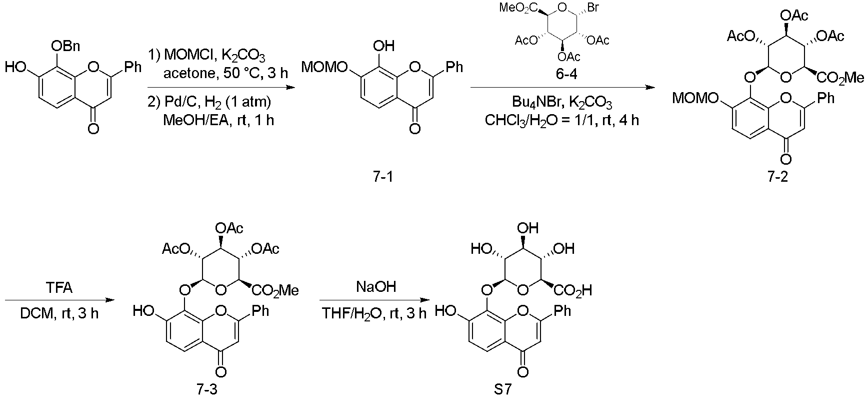

### Synthesis of Intermediate 7-1

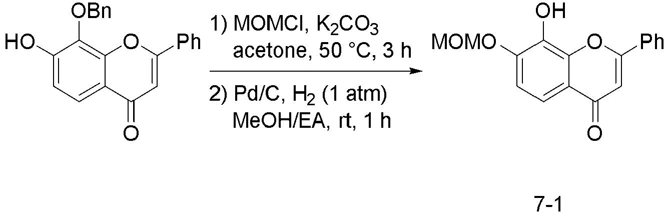

Starting material 6-1 was dissolved in acetone. 2 equivalents of K₂CO₃ were added, followed by 1.2 equivalents of MOMCl under stirring at room temperature. The reaction progress was monitored by TLC. After the reaction was completed, the mixture was acidified with dilute hydrochloric acid, and then extracted three times with EA. The organic phase was concentrated by rotary evaporation, and purified by column chromatography. The purified product from the previous step was placed in a reaction vessel along with 10 mol% Pd/C. The atmosphere was replaced with hydrogen three times, then a mixture of MeOH/EA (10/1) was added. The reaction was stirred at room temperature for 1 hour, with progress monitored by TLC. After reaction was completed, the mixture was directly filtered, concentrated, and purified by column chromatography to yield the white solid product 7-1 in a total two-step yield of 90%.

### Synthesis of Intermediate 7-2

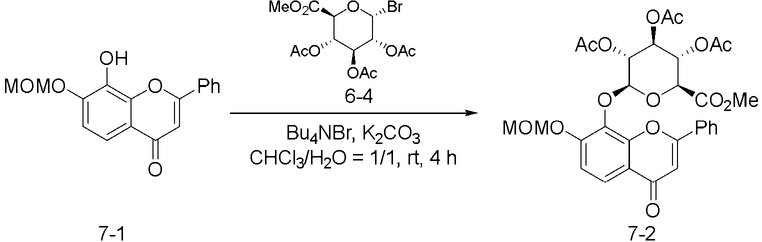

1 equivalent of starting material 7-1, 1.2 equivalents of Bu₄NBr, 5 equivalents of K₂CO₃ and 3 equivalents of 6-4 were dissolved in a mixed solvent of CHCl₃ and H₂O (1:1). The reaction was then carried out at room temperature for 4 hours, with progress monitored by TLC. After reaction completion, the crude product was obtained by extraction, drying, filtration, and concentration. Finally, the product was purified by column chromatography to yield the white solid product 7-2 in 95% yield.

### Synthesis of Intermediate 7-3

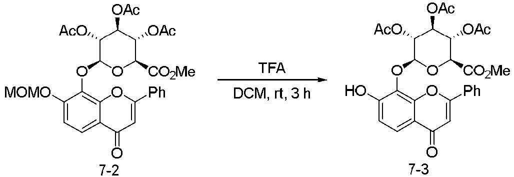

1 equivalent of starting material 7-2 was dissolved in DCM, followed by the addition of 10 equivalents of TFA. The reaction was then carried out at room temperature for 3 hours, with progress monitored by TLC. After the reaction was completed, a saturated aqueous NaHCO₃ solution was added to adjust the pH to weakly alkaline. The crude product was then obtained by extraction, drying, filtration, and concentration. Finally, the product was purified by column chromatography to yield the white solid product 7-3 in 70% yield.

### Synthesis of Compound S7

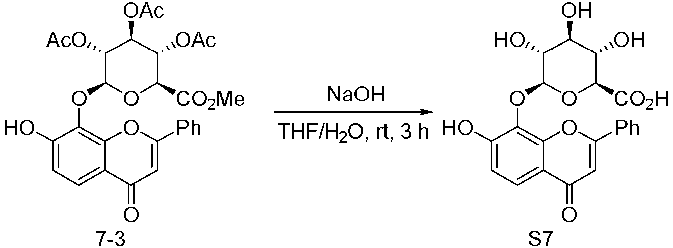

1 equivalent of starting material 7-3 was dissolved in a mixed solvent of THF and H₂O (2:1), followed by the addition of 6 equivalents of NaOH. The reaction was carried out at room temperature for 3 hours, with reaction progress monitored by TLC. Upon completion, excess acetic acid was added to adjust the pH to acidic conditions. The crude product was obtained by concentration, then purified via preparative column chromatography to yield the final product in 83% yield.

¹H NMR (400 MHz, DMSO-*d₆*) δ 10.87 (s, 1H), 8.18-8.16 (m, 2H), 7.73 (d, *J=* 8.8 Hz, 1H), 7.62-7.55 (m, 3H), 7.05 (d, *J =* 8.8 Hz, 1H), 6.94 (s, 1H), 5.61 (d, *J =* 8.0 Hz, 1H), 5.51-5.46 (m, 1H), 5.29-5.24 (m, 1H), 5.17-5.12 (m, 1H), 4.56 (d, *J* = 10.0 Hz, 1H).

¹³C NMR (100 MHz, DMSO-*d₆*) δ 176.81, 170.06, 162.61, 155.70, 150.82, 132.06, 131.73, 131.34, 129.41, 127.04, 122.06, 117.19, 115.37, 106.75, 101.17, 71.71, 71.68, 71.65, 69.57, 52.82.

### Example 8 Synthesis of Compound S8

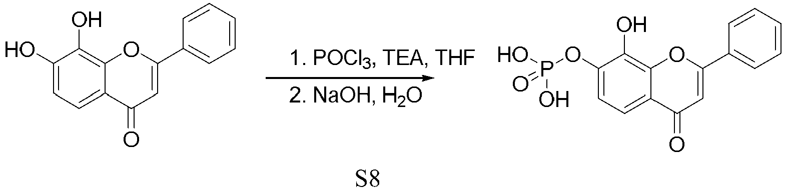

### Synthesis of Compound S8

1 equivalent of 7,8-dihydroxyflavone was dissolved in THF. 1 equivalent of trimethylamine was added, followed by 1 equivalent of phosphorous oxychloride. The mixture was stirred at room temperature for 1 hour, and then water and 2 equivalents of NaOH were added. The reaction was continued for 1 hour, then the pH was adjusted to 1-2 with 1 M hydrochloric acid. The mixture was then filtered, and the solid was washed with a small amount of water and dried to afford a product in 73% yield.

ES-MS m/z [M+1]⁺: 335.0.

### Example 9 Synthesis of Compound S9

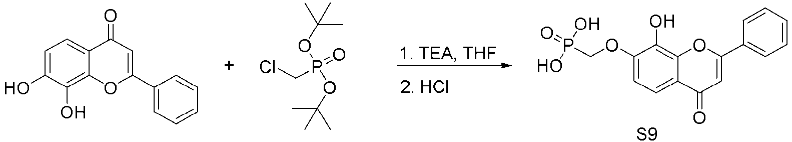

### Synthesis of Compound S9

1 equivalent of 7,8-dihydroxyflavone was dissolved in THF. 1 equivalent of trimethylamine was added, followed by the addition of 1 equivalent of tert-butyl chloromethylphosphonate. The mixture was stirred at room temperature for 2 hours. After the reaction was completed, the pH was adjusted to below 1 with concentrated hydrochloric acid. After an additional 6 hours of stirring, the mixture was filtered. The solid was washed with a small amount of THF and dried to afford a product in 67% yield.

ES-MS m/z [M+1]⁺: 365.0.

### Example 10 Synthesis of Compound S10

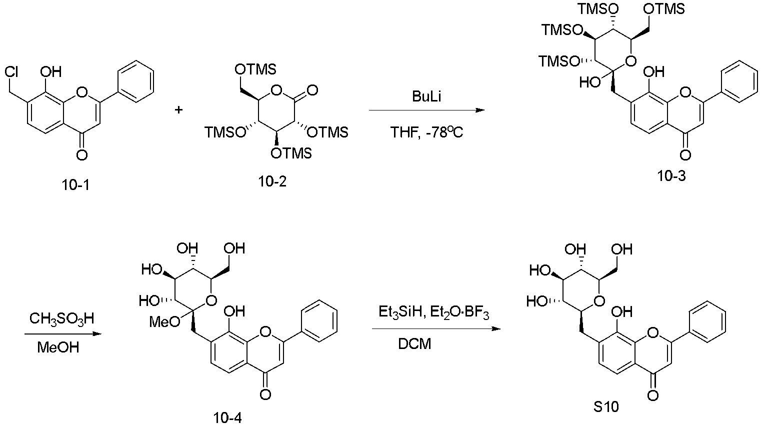

### Synthesis of Compound 10-3

1 equivalent of compound 10-1 was added to a 3 L three-necked flask and dissolved in THF. The mixture was stirred under a nitrogen atmosphere, and cooled to -78 °C. A solution of 2.5 equivalents of n-butyllithium in THF was slowly added. After addition, stirring was continued at -78°C for 0.5 hour. Then, a solution of 1.5 equivalents of 10-2 in THF was added dropwise and the reaction was stirred for 1 hour, then warmed to room temperature. An aqueous solution of 3 equivalents of acetic acid was added dropwise to quench the reaction. The reaction mixture was extracted with EA. The organic phase was dried over anhydrous sodium sulfate, and subsequently concentrated to obtain S10-3.

### Synthesis of Compound 10-4

The obtained compound 10-3 was dissolved in methanol, followed by the addition of 8 equivalents of methanesulfonic acid. The mixture was stirred at 40 °C for 2 hours. Methanol was then removed by distillation under reduced pressure. The residue was dissolved in ethyl acetate. The solution was sequentially washed with saturated sodium bicarbonate and saturated sodium chloride solutions, dried over anhydrous sodium sulfate, and filtered. The filtrate was distilled under reduced pressure to remove the solvent. Toluene and cyclohexane were added to the resulting brown solid, slurried for 2 hours, and then vacuum filtered. The filter cake was dried to yield a white solid in 53% yield.

### Synthesis of Compound S10

Compound 10-4 was added to a 3 L three-necked flask and dissolved in dichloromethane. 3 equivalents of triethylsilane were added under stirring, and the mixture was cooled to -78 °C under nitrogen atmosphere. 3 equivalents of boron trifluoride were added slowly, and the reaction was warmed to 0 °C and carried out for 2 hours. The reaction mixture was poured into saturated sodium bicarbonate solution and sequentially washed with saturated sodium chloride solution, 5% hydrochloric acid, and saturated sodium chloride solution. The organic phase was dried over anhydrous sodium sulfate, filtered, and concentrated. The concentrate was recrystallized from methanol, filtered, and dried to afford a white solid in 65% yield.

ES-MS m/z [M+1]⁺: 415.1.

### Example 11 Synthesis of Compound S11

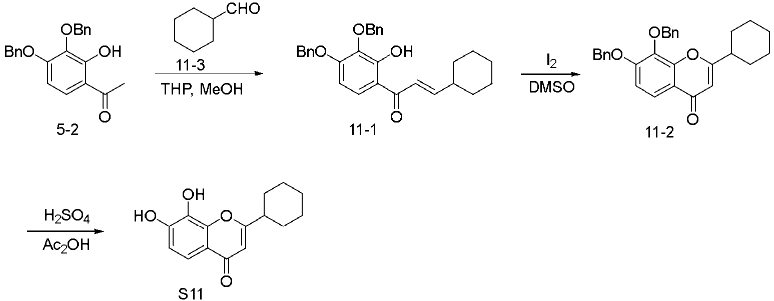

### Synthesis of Compound 11-1

1 equivalent of compound 5-2 was added to a 3 L three-necked flask and dissolved in methanol. 1 equivalent of pyrrolidine was added, and the reaction was carried out with stirring at room temperature for 1 h. Then 1.2 equivalents of compound 11-3 were added and the reaction mixture was heated to reflux. After stirring for 8 hours, the mixture was cooled to room temperature and the reaction was quenched with 2 equivalents of acetic acid. The mixture was stirred for an additional 0.5 hour, followed by suction filtration. The solid was washed with methanol and dried to afford a yellow solid in 85% yield.

### Synthesis of Compound 11-2

1 equivalent of compound 11-1 was added to a 3L three-necked flask and dissolved in dimethyl sulfoxide. 0.15 equivalents of iodine was added, and the reaction was carried out with stirring at 110 °C for 24 hours. After the reaction was completed, the reaction mixture was cooled to room temperature and the reaction was quenched with saturated sodium thiosulfate solution. The mixture was stirred for an additional 0.5 hour, followed by suction filtration. The solid was washed with methanol and dried to afford a white solid in 75% yield.

### Synthesis of Compound S11

1 equivalent of compound 11-2 was added to a 3 L three-necked flask and dissolved in acetic acid. 4 equivalents of concentrated sulfuric acid were added and the reaction was carried out with stirring at 80 °C for 24 hours. After the reaction was completed, the mixture was cooled to room temperature and filtered by suction. The solid was washed sequentially with water and methanol to obtain a yellow solid in 87% yield.

¹H NMR (400 MHz, CDCl₃) δ 10.20 (s, 1H), 9.28 (s, 1H), 7.33 (d, *J* = 8.7 Hz, 1H), 6.89 (d, *J* = 8.7 Hz, 1H), 5.98 (s, 1H), 2.55 (tt, *J* = 11.6, 8.3, 3.4 Hz, 1H), 2.00-1.92 (m, 2H), 1.84-1.73 (m, 2H), 1.72-1.63 (m, 1H), 1.54-1.41 (m, 2H), 1.40-1.14 (m, 3H).

¹³C NMR (100 MHz, CDCl₃) δ 182.13, 177.40, 155.11, 151.92, 138.12, 121.96, 120.05, 118.88, 111.45, 47.07, 35.06, 30.59, 30.51.

ES-MS m/z [M-1]⁻: 259.30.

### Example 12 Synthesis of Compound S12

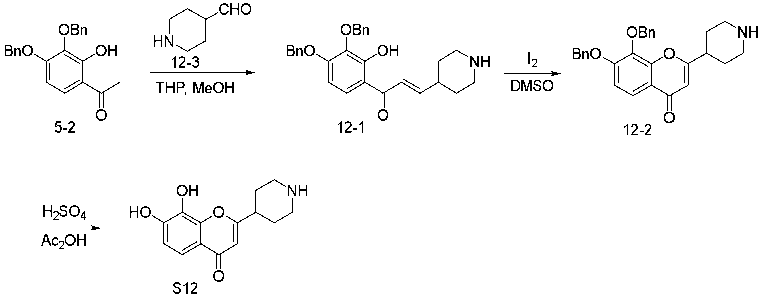

### Synthesis of Compound 12-1

1 equivalent of compound 5-2 was added to a 3 L three-necked flask and dissolved in methanol. 1 equivalent of pyrrolidine was added and the mixture was stirred at room temperature for 1 h. Then 1.2 equivalents of 12-3 were added and the mixture was heated to reflux. After stirring for 8 hours, the reaction mixture was cooled to room temperature and the reaction was quenched with 2 equivalents of acetic acid. The mixture was stirred for an additional 0.5 hour, followed by suction filtration. The solid was washed with methanol and dried to afford a yellow solid in 80% yield.

### Synthesis of Compound 12-2

1 equivalent of 12-1 was added to a 3 L three-necked flask and dissolved in dimethyl sulfoxide. 0.15 equivalents of iodine was added, and the mixture was stirred at 110 °C for 24 hours. After completion of the reaction, the mixture was cooled to room temperature and the reaction was quenched with saturated sodium thiosulfate solution. The mixture was stirred for an additional 0.5 hour, followed by suction filtration. The solid was washed with methanol and dried to afford a white solid in 77% yield.

### Synthesis of Compound S12

1 equivalent of compound 12-2 was added to a 3L three-necked flask and dissolved in acetic acid. 4 equivalents of concentrated sulfuric acid were added, and the mixture was stirred at 80 °C for 24 hours. After completion of the reaction, the mixture was cooled to room temperature, followed by suction filtration. The solid was washed successively with water and methanol to obtain a yellow solid in 84% yield.

¹H NMR (400 MHz, DMSO-*d₆*) δ 10.37 (s, 1H), 9.34 (s, 1H), 8.66 (d, J = 11.5 Hz, 1H), 8.38 (d, J = 11.1 Hz, 1H), 7.35 (d, J = 8.7 Hz, 1H), 6.93 (d, J = 8.6 Hz, 1H), 6.06 (s, 1H), 3.49-3.34 (m, 2H), 3.12-2.93 (m, 3H), 2.24-2.10 (m, 2H), 2.01-1.81 (m, 2H).

¹³C NMR (100 MHz, DMSO-*d₆*) δ 177.2, 169.6, 150.5, 147.1, 133.4, 117.2, 115.3, 114.3, 107.4, 43.3, 37.7, 26.2.

ES-MS m/z [M+1]⁺: 261.90.

### Example 13 Synthesis of Compound S13

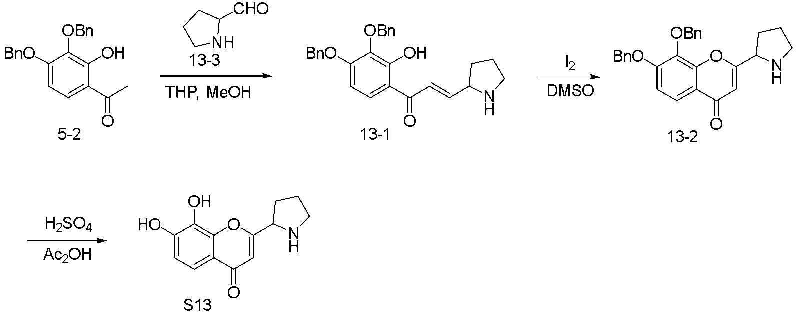

### Synthesis of Compound 13-1

1 equivalent of compound 5-2 was added to a 3 L three-necked flask and dissolved in methanol. 1 equivalent of pyrrolidine was added, and the mixture was stirred at room temperature for 1 h. Then 1.2 equivalents of 13-3 were added and the mixture was heated to reflux. After stirring for 8 hours, the mixture was cooled to room temperature and the reaction was quenched with 2 equivalents of acetic acid. The mixture was stirred for an additional 0.5 hour, followed by suction filtration. The solid was washed with methanol and dried to afford a yellow solid in 83% yield.

### Synthesis of Compound 13-2

1 equivalent of compound 13-1 was added to a 3 L three-necked flask and dissolved in dimethyl sulfoxide. 0.15 equivalents of iodine was added, and the mixture was stirred at 110 °C for 24 hours. After completion of the reaction, the mixture was cooled to room temperature and the reaction was quenched with saturated sodium thiosulfate solution. The mixture was stirred for an additional 0.5 hour, followed by suction filtration. The solid was washed with methanol and dried to afford a white solid in 76% yield.

### Synthesis of Compound S13

1 equivalent of compound 13-2 was added to a 3 L three-necked flask and dissolved in acetic acid. 4 equivalents of concentrated sulfuric acid were added, and the mixture was stirred at 80 °C for 24 hours. After completion of the reaction, the mixture was cooled to room temperature, followed by suction filtration. The solid was washed sequentially with water and methanol to obtain a yellow solid in 82% yield.

ES-MS m/z [M+1]⁺: 248.1.

### Example 14 Synthesis of Compound S14

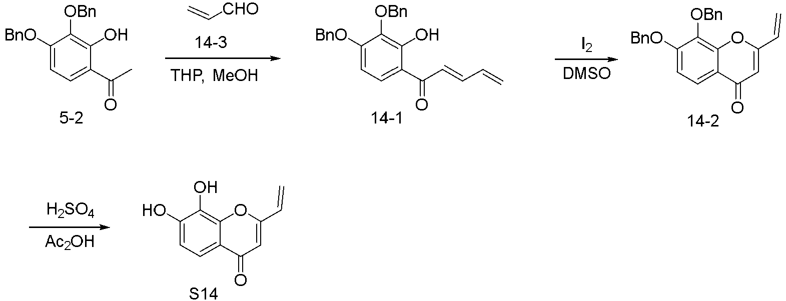

### Synthesis of Compound 14-1

1 equivalent of compound 5-2 was added to a 3L three-necked flask and dissolved in methanol. 1 equivalent of pyrrolidine was added, and the mixture was stirred at room temperature for 1 hour. Then 1.2 equivalents of 14-3 was added and the mixture was heated to reflux. After stirring for 8 hours, the reaction mixture was cooled to room temperature and the reaction was quenched with 2 equivalents of acetic acid. The mixture was stirred for an additional 0.5 hour, followed by suction filtration. The solid was washed with methanol and dried to obtain a yellow solid in 78% yield.

### Synthesis of Compound 14-2

1 equivalent of compound 14-1 was added to a 3 L three-necked flask and dissolved in dimethyl sulfoxide. 0.15 equivalents of iodine was added, and the mixture was stirred at 110 °C for 24 hours. After completion of the reaction, the reaction mixture was cooled to room temperature and the reaction was quenched with saturated sodium thiosulfate solution. After stirring for an additional 0.5 hour, the mixture was filtered under suction. The solid was washed with methanol and dried to afford a white solid in 83% yield.

### Synthesis of Compound S14

1 equivalent of 14-2 was added to a 3 L three-necked flask and dissolved in acetic acid. 4 equivalents of concentrated sulfuric acid were added, and the mixture was stirred at 80 °C for 24 hours. After completion of the reaction, the mixture was cooled to room temperature and filtered under suction. The solid was washed sequentially with water and methanol to obtain a yellow solid in 86% yield.

ES-MS m/z [M+1]⁺: 205.0.

### Example 15 Synthesis of Compound S15

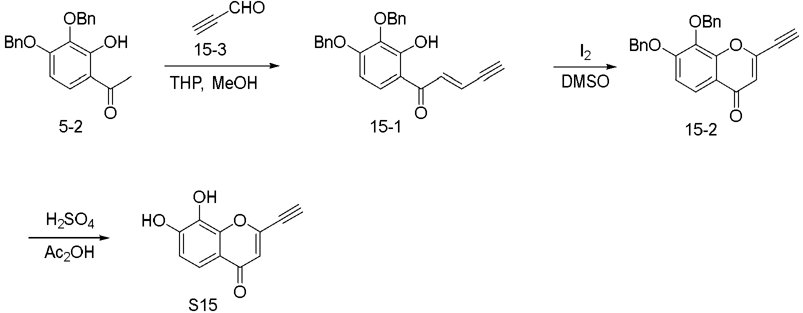

### Synthesis of Compound 15-1

1 equivalent of compound 5-2 was added to a 3 L three-necked flask and dissolved in methanol. 1 equivalent of pyrrolidine was added, and the mixture was stirred at room temperature for 1 h. Then 1.2 equivalents of 15-3 were added and the mixture was heated to reflux. After stirring for 8 hours, the reaction mixture was cooled to room temperature and the reaction was quenched with 2 equivalents of acetic acid. The mixture was stirred for an additional 0.5 hour, then filtered under suction. The solid was washed with methanol and dried to obtain a yellow solid in 85% yield.

### Synthesis of Compound 15-2

1 equivalent of 15-1 was added to a 3 L three-necked flask and dissolved in dimethyl sulfoxide. 0.15 equivalents of iodine was added, and the mixture was stirred at 110 °C for 24 hours. After completion of the reaction, the reaction mixture was cooled to room temperature and the reaction was quenched with saturated sodium thiosulfate solution. After stirring for an additional 0.5 hour, the mixture was filtered under suction. The solid was washed with methanol and dried to afford a white solid in 80% yield.

### Synthesis of Compound S15

1 equivalent of compound 15-2 was added to a 3L three-necked flask and dissolved in acetic acid. 4 equivalents of concentrated sulfuric acid were added, and the mixture was stirred at 80 °C for 24 hours. After completion of the reaction, the reaction mixture was cooled to room temperature and filtered under vacuum. The solid was washed successively with water and methanol to obtain a yellow solid in 81% yield.

ES-MS m/z [M+1]⁺: 203.0.

### Example 16 Synthesis of Compound S16

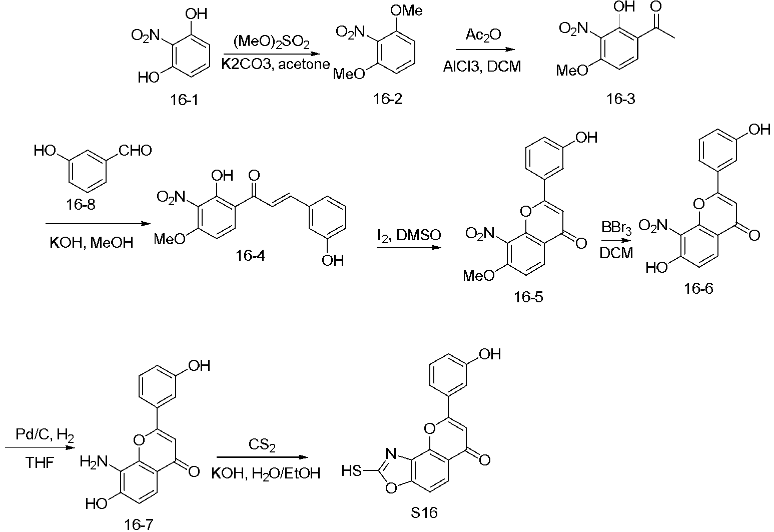

### Synthesis of Compound 16-2

23.27 g of compound 16-1 was added to a 500 mL two-necked flask and dissolved in 150 mL acetone. 103.65 g potassium carbonate and 56.76 g dimethyl sulfate were added, and the mixture was heated to reflux. After 18 h of reaction, the reaction mixture was poured into 1 L of water, stirred for an additional 0.5 hour, and then filtered under suction. The filter cake was dried to afford a white solid in 88% yield.

### Synthesis of Compound 16-3

24 g of compound 16-2 was added to a 1 L two-necked flask and dissolved in 300 mL of dichloromethane. After the mixture was cooled to 0 °C, 34.94 g of aluminum trichloride was added with stirring. Subsequently, 14.71 g of acetic anhydride was added dropwise slowly. After the addition was completed, the mixture was heated to reflux and reacted overnight. After completion of reaction, the mixture was cooled to 0 °C, and the reaction was quenched with 65 mL of concentrated hydrochloric acid. Then 300 mL of water and 200 mL of dichloromethane were added, and the mixture was stirred for an additional 0.5 h. The organic and aqueous phases were separated. The organic phase was washed with water, and then concentrated to obtain a brown solid in 61% yield.

### Synthesis of Compound 16-4

9 g of compound 16-3 was added to a 500 mL two-necked flask and dissolved in 150 mL methanol. 16.74 g of potassium hydroxide and 7. 29 g of compound 16-8 were added, and the mixture was heated to reflux for 7 h for reaction. After cooling to room temperature, the reaction was quenched with 20 mL acetic acid. The reaction mixture was poured into 400 mL of isopropyl ether, stirred at room temperature for 0.5 hour, and then filtered under suction. The solid was dried to obtain a yellow solid in 69% yield.

### Synthesis of Compound 16-5

9.2 g of compound 16-4 was added to a 500 mL two-necked flask and dissolved in 60 mL dimethyl sulfoxide. 1.11 g iodine was added, and the mixture was heated to 110 °C and reacted overnight with stirring. After completion of the reaction, the mixture was cooled to room temperature, and the reaction was quenched with 60 mL of saturated sodium thiosulfate solution. The mixture was filtered under suction, and the filter cake was dried to yield a yellow solid in 61% yield.

### Synthesis of Compound 16-6

2.0 g of compound 16-5 was added to a 100 mL two-necked flask and dissolved in 20 mL of dichloromethane. 6.4 g of boron tribromide was added, and the mixture was heated to reflux for 4 hours. After the reaction was completed, it was quenched with 50 mL of water. Then the mixture was filtered under suction and the filter cake was dried to obtain a yellow solid in 72% yield.

### Synthesis of Compound 16-7

1.30 g of compound 16-6 was added to a 100 mL two-necked flask and dissolved in 20 mL of tetrahydrofuran. 200 mg of 10% Pd/C was added, and the mixture stirred overnight at room temperature under 1 atm of hydrogen gas. The mixture was filtered under suction, and the filtrate was concentrated to obtain a yellow solid in 92% yield.

### Synthesis of Compound 16

1 g of compound 16-7 was added to a 50 mL two-necked flask and dissolved in 15 mL of ethanol. 3 mL of water, 760 mg of carbon disulfide, and 560 mg of potassium hydroxide were added sequentially. The mixture was heated to reflux for 3 h. After completion of the reaction, the reaction mixture was poured into warm water at 60-70 °C, followed by the addition of 2 mL of acetic acid, and then the mixture was cooled to 0 °C. After being allowed to stand and cool overnight, the mixture was filtered under suction, and the filter cake was dried to afford a white solid in 81% yield.

ES-MS m/z [M+1]⁺: 312.0.

### Example 17 Synthesis of Compound S17

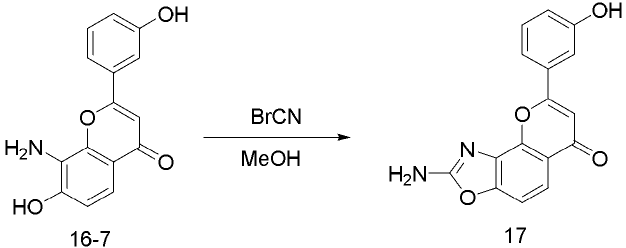

### Synthesis of Compound 17

1 equivalent of compound 16-7 was added to a 100 mL two-necked flask and dissolved in methanol. 1.1 equivalents of cyanobromide were added, and the mixture was stirred at room temperature overnight. After completion of the reaction, the pH of the mixture was adjusted to 7 with saturated sodium carbonate solution. Then methanol was removed by distillation under reduced pressure, and the residue was dissolved in ethyl acetate. The mixture was washed sequentially with water and saturated sodium chloride solution, and then dried over anhydrous sodium sulfate. After filtration, the organic solvent was removed by distillation under reduced pressure to obtain a white solid in 83% yield.

ES-MS m/z [M+1]⁺: 295.1.

### Example 18 Synthesis of Compound S18

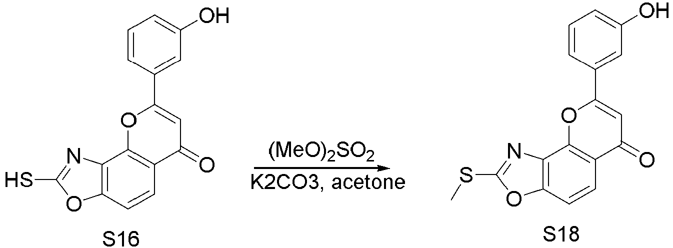

### Synthesis of Compound 18

1 equivalent of compound 16 was added to a 500 mL two-necked flask and dissolved in 150 mL acetone. 1.5 equivalents potassium carbonate and 1 equivalent dimethyl sulfate were added, and the mixture was heated to reflux. After 18 hours reaction, the reaction mixture was poured into 1 L of water, stirred for an additional 0.5 hour, and then filtered under suction. The filter cake was dried to afford a white solid in 89% yield.

ES-MS m/z [M+1]⁺: 326.0.

### Example 19 Synthesis of Compound S19

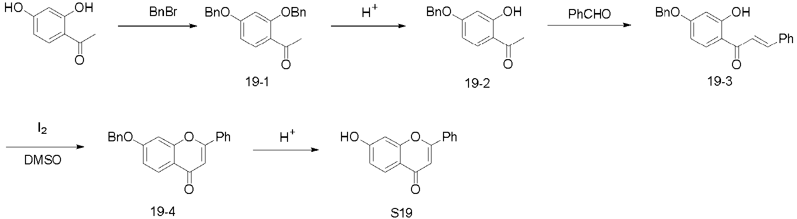

### Synthesis of Intermediate 19-1

To a 2 L two-necked flask charged with 2,4 - dihydroxyacetophenone (50 g, 328.63 mmol) was added 330 mL of NMP. Under magnetic stirring at room temperature, K₂CO₃ (99.92 g, 722.98 mmol, 2.2 eq), KI (8.18 g, 49.29 mmol, 0.15 eq.), 80 mL of water and benzyl bromide (123.66 g, 722.98 mmol, 2.2 eq.) were added sequentially. The mixture was stirred at 30 °C for 2 hours, and the reaction was monitored by TLC (developing solvent: EA : PE = 1 : 3). After completion of the reaction, water (1.3 L) was added to the reaction system, and the mixture was stirred for another 20 min, resulting in the precipitation of a large amount of solid. The solid was collected by suction filtration, and the filter cake was washed once with ethanol (330 mL) and dried at room temperature to afford intermediate 19-1 (100.72 g, yellow solid) in 92 % yield.

### Synthesis of Intermediate 19-2

To a 500 mL two-necked flask charged with intermediate 19-1 (100.72 g, 303.01 mmol) was added 300 mL of acetic acid. Under magnetic stirring at room temperature, 30 mL of concentrated hydrochloric acid was added, and the mixture was stirred at 45 °C for 18h. The reaction progress was monitored by TLC (developing solvent: DCM:PE = 2:1). Upon completion of the reaction, the mixture was cooled to room temperature, then further cooled to 15 °C and stirred at this temperature for 30 minutes. The mixture was filtered under suction. The filter cake was washed once with 100 mL of ethanol and dried in an oven at 60 °C to afford intermediate 19-2 (43.23 g, white solid) in 59% yield.

### Synthesis of Intermediate 19-3

To a 3 L two-necked flask charged with intermediate 19-2 (43.23 g, 178.43 mmol) was added 900 mL of methanol. Under magnetic stirring at room temperature, KOH (70.08 g, 1.25 mol, 7.0 eq.) and benzaldehyde (26.51 g, 249.81 mmol, 1.4 eq.) were added, and the mixture was stirred under reflux for 18 hours. The reaction progress was monitored by TLC (developing solvent: DCM:PE = 1:1). Upon completion of the reaction, the mixture was cooled to room temperature, and 100 mL of acetic acid was added to the system to quench the reaction. The mixture was filtered under suction. The filter cake was washed once with 100 mL of methanol and dried in an oven at 80 °C to afford intermediate 19-3 (47.97 g, yellow solid) in 81% yield.

### Synthesis of Intermediate 19-4

To a 2 L two-necked flask charged with intermediate 19-3 (47.97 g, 145.20 mmol) was added 300 mL of dimethyl sulfoxide. Under magnetic stirring at room temperature, iodine (5.53 g, 21.78 mmol, 0.15 eq.) was added, and the mixture was stirred at 110°C for 24h. The reaction progress was monitored by TLC (developing solvent: EA:PE = 1 : 3). Upon completion of the reaction, the mixture was cooled to room temperature. 22 g of sodium thiosulfate pentahydrate solid was dissolved in 600 mL of water to prepare a solution, which was then added to the reaction system. The mixture was further stirred at room temperature for an additional 1 hour, and then filtered under suction. The filter cake was dried in an oven at 80 °C to afford intermediate 19-4 (47.66 g, yellow solid) in 99% yield.

### Synthesis of Compound S19

To a 1 L two-necked flask charged with intermediate 19-4 (46.02 g, 140.15 mmol) was added 500 mL of acetic acid. Under magnetic stirring at room temperature, 54.98 g of concentrated sulfuric acid was added, and the mixture was stirred at 80 °C for 17h. The reaction progress was monitored by TLC (developing solvent: EA:PE = 1 : 3). Upon completion of the reaction, the mixture was cooled to room temperature, and then filtered under suction. The filter cake was washed with 300 mL of water and subsequently dried in an oven at 80 °C to afford Compound S19 (25.14 g, yellow solid) in 75% yield.

¹H NMR (400 MHz, DMSO-*d₆*) δ 10.88 (s, 1H), 8.11-7.98 (m, 2H), 7.90 (d, *J* = 8.7 Hz, 1H), 7.65-7.48 (m, 3H), 7.01 (d, *J* = 2.3 Hz, 1H), 6.94 (dd, *J* = 8.7, 2.3 Hz, 1H), 6.89 (s, 1H).

### Example 20 Synthesis of Compound S20

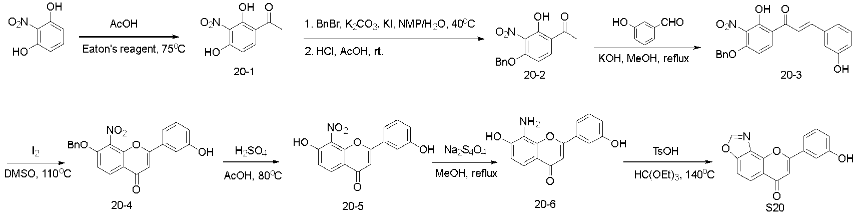

### Synthesis of Intermediate 20-1

To a 500 mL two-necked flask charged with 2-nitroresorcinol (20 g, 118.94 mmol) was added Eaton's reagent (245.18 g, 1.03 mol, 8.0 eq.). Under stirring at room temperature, acetic acid (11.61 g, 193.41 mmol, 1.5 eq.) was added to the system, and the mixture was stirred at 75 °C, with reaction progress monitored by TLC (developing solvent: EA: PE = 1 : 3, v/v). After stirring for 3 hours, the temperature was lowered to room temperature. 500 mL of water was measured and added to a 2 L beaker, which was then placed in a 0 °C cooling bath. The reaction mixture was then poured into the water and extracted with 250 mL of ethyl acetate. The organic phase was concentrated under reduced pressure to afford intermediate 20-1 (23.23 g, black oil) in 91% yield.

### Synthesis of Intermediate 20-2

To a 500 mL single-necked flask charged with intermediate 20-1 (23.23 g, 118 mmol) was added140 mL of NMP. Under stirring at 40 °C, K₂CO₃ (56.94 g, 412 mmol, 3.5 eq.), KI (2.94 g, 17 mmol, 0.15 eq.) and 30 mL of water were added to the system. Benzyl bromide (70.54 g, 412 mmol, 3.5 eq.) was added at 40 °C, and the mixture was stirred at the same temperature. After stirring for 2 hours, the temperature was lowered to room temperature. 600 mL of water was measured and added to a 2 L beaker, and the reaction mixture was poured into the water. The mixture was extracted twice with 250 mL of ethyl acetate each time. The combined organic phases were concentrated under reduced pressure to obtain 63.83 g of a black oil. 100 mL of acetic acid was added to the obtained black oil, and the mixture was stirred at room temperature. Subsequently, 20 mL of concentrated hydrochloric acid was added to the system, and stirring was continued at room temperature for 21 hours, followed directly by suction filtration. The filter cake was dried in an oven at 80 °C for 4 hours to afford intermediate 20-2 (14.08 g, reddish-brown solid) in 42% yield.

### Synthesis of Intermediate 20-3

To a two-necked flask charged with 220 mL of methanol was added KOH (16.5 g, 294 mmol, 7.0 eq.). Under stirring at room temperature, intermediate 20-2 (12 g, 42 mmol) and 3-hydroxybenzaldehyde (7.1 g, 58 mmol, 1.4 eq.) were added to the system, and then the mixture was heated to reflux. The reaction progress was monitored by TLC (developing solvent: DCM: PE = 5:1, v/v). After stirring for 21 hours, the temperature was lowered to room temperature. 30 mL of isopropyl ether was added to the reaction system, and then 21.2 g of acetic acid was added to quench the reaction. Stirring was continued at room temperature for 30 minutes, followed by suction filtration. The filter cake was rinsed once with 20 mL of methanol and dried in an oven at 80 °C for 2 hours to afford intermediate 20-3 (8.18 g, yellow solid) in 50% yield.

### Synthesis of Intermediate 20-4

To a 250 mL two-necked flask charged with intermediate 20-3 (8.18 g, 21 mmol) was added 70 mL of DMSO. The mixture was stirred at room temperature, and iodine (0.80 g, 3.14 mmol, 0.15 eq.) was added to the system before heating the mixture to 110 °C. After stirring for 17 hours, the temperature was lowered to room temperature. 1.56 g of sodium thiosulfate pentahydrate was weighed and placed in a 250 mL beaker, then dissolved in 150 mL of water to prepare a sodium thiosulfate solution. 35 mL of isopropyl ether was added to the reaction system, followed by the prepared sodium thiosulfate solution to quench the reaction. Stirring was continued at room temperature for 30 minutes, followed by filtration under reduced pressure. The filter cake was dried in an oven at 80 °C for 4 h to afford intermediate 20-4 (5.45 g, yellow solid) in 67% yield.

### Synthesis of Intermediate 20-5

To a two-necked flask charged with intermediate 20-4 (5 g, 13 mmol) was added 60 mL of glacial acetic acid, and the mixture was stirred at room temperature. Concentrated sulfuric acid (5.03 g, 51 mmol, 4.0 eq.) was added to the system and the mixture was heated to 80 °C. After stirring for 24 hours, the temperature was lowered to room temperature, followed by filtration under reduced pressure. The filter cake was rinsed once with 40 mL of water and dried in an oven at 80 °C for 4 h to afford intermediate 20-5 (2.50 g, yellow solid) in 65% yield.

### Synthesis of Intermediate 20-6

To a three-necked flask charged with intermediate 20-5 (2.5 g, 8.35 mmol) was added 40 mL of methanol, and the mixture was stirred at room temperature. Sodium hydrosulfite (8.72 g, 50 mmol, 6.0 eq.) was added and the mixture was heated to reflux. The reaction progress was monitored by TLC (developing solvent: EA). After stirring for 2 hours, the temperature was lowered to room temperature, and the mixture was concentrated under reduced pressure. 50 mL of water was added to the concentrate, and the mixture was slurried for 20 minutes, followed by suction filtration. The filter cake was dried in an oven at 80 °C for 2 h to afford intermediate 20-6 (270 mg, yellow solid).

### Synthesis of Compound S20

To a two-necked flask charged with intermediate 20-6 (3.5 g, 12.9 mmol) was added 30 mL of triethyl orthoformate, and the mixture was stirred at room temperature. p-Toluenesulfonic acid (67.0 mg, 0.39 mmol, 0.03 eq.) was added and the mixture was heated 140 °C for reaction. The reaction progress was monitored by TLC (developing solvent: EA:TEA = 10:1, v/v). After stirring for 19 hours, the temperature was lowered to room temperature, and the mixture was concentrated under reduced pressure. The concentrate was purified by silica gel column chromatography (eluent: EA:TEA = 10:1) to afford compound S20 (170 mg, yellow solid) in 5% yield.

¹H NMR (400 MHz, DMSO-*d*₆) δ 9.98 (s, 1H), 9.03 (s, 1H), 8.09 (d, J = 8.7 Hz, 1H), 7.90 (d, J = 8.7 Hz, 1H), 7.59-7.48 (m, 2H), 7.41 (t, J = 7.9 Hz, 1H), 7.08-6.97 (m, 2H).

¹³C NMR (100 MHz, DMSO-*d*₆) δ 176.9, 162.5, 158.4, 155.9, 154.0, 148.2, 132.5, 130.8, 129.5, 122.8, 120.8, 119.4, 117.6, 113.1, 110.0, 107.9.

ES-MS m/z [M+1]⁺: 280.0.

### Example 21 Synthesis of Compound S21

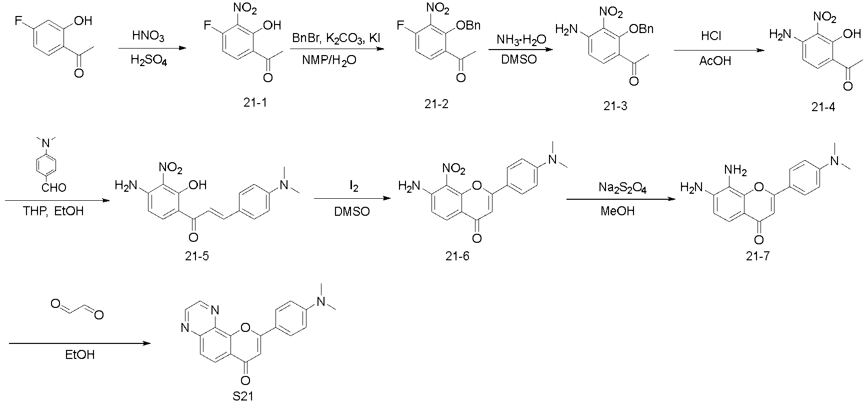

### Synthesis of Intermediate 21-1

To a two-necked flask charged with 50 g of 4-fluoro-2-hydroxyacetophenone (324 mmol) was added 200 mL of concentrated sulfuric acid. The mixture was cooled to -10 °C, and 35 mL of concentrated nitric acid was added dropwise slowly over approximately 1 h. Upon completion of the concentrated nitric acid addition, the reaction solution was added dropwise into 1 L of water over about 1 h. After the addition of water, the mixture was extracted with ethyl acetate. The organic phase was concentrated under reduced pressure, and the concentrate was purified by silica gel column chromatography (EA:PE=5:1) to afford intermediate 21-1 (33 g, yellow solid) in 51% yield.

### Synthesis of Intermediate 21-2

To a two-necked flask charged with 33 g of intermediate 21-1 (165.71 mmol) was added 200 mL of NMP. Under stirring at room temperature, 45.80 g of K₂CO₃ (331.43 mmol, 2.0 eq.), 1.38 g of KI (8.29 mmol, 0.05 eq.) and 50 mL of water were added. The mixture was then heated to 45 °C, and 42.52 g of benzyl bromide (248.57 mmol, 1.5 eq.) was added thereto. Stirring was continued at 45 °C for reaction. After stirring for 4 hours, the reaction was quenched with 800 mL of water. The mixture was extracted twice with ethyl acetate. The combined organic phases were washed twice with water and then concentrated under reduced pressure to afford intermediate 21-2 (45.44 g, reddish-brown liquid).

¹H NMR (400 MHz, DMSO-*d*₆) δ 8.07 (dd, J = 9.0, 6.5 Hz, 1H), 7.54 (t, J = 9.0 Hz, 1H), 7.42-7.38 (m, 3H), 7.38-7.34 (m, 2H), 7.33-7.30 (m, 1H), 5.06 (s, 2H), 2.61 (s, 3H).

¹³C NMR (100 MHz, DMSO-*d*₆) δ 197.4, 156.5, 153.9, 150.5, 150.5, 143.0, 135.5, 134.5, 134.4, 131.0, 131.0, 129.3, 129.0, 129.0, 128.5, 126.9, 113.3, 113.1, 79.2, 63.3, 30.5.

### Synthesis of Intermediate 21-3

To a two-necked flask charged with 45.44 g of intermediate 21-2 (157.10 mmol) was added 450 mL of DMSO. Under stirring at room temperature, 75 mL of ammonia solution was added. The mixture was then stirred at 50 °C for 16 hours, after which the reaction was quenched with 450 mL of water. The mixture was extracted with ethyl acetate, and the organic phase was concentrated under reduced pressure. The concentrate was purified by silica gel column chromatography (EA:PE=2:1) to afford intermediate 21-3 (6.6 g, reddish-brown solid) in 15% yield.

### Synthesis of Intermediate 21-4

To a two-necked flask charged with 9 g of intermediate 21-3 (31.40 mmol) was added 20 mL of glacial acetic acid. Under stirring at room temperature, 4 mL of concentrated hydrochloric acid was added, and stirring was continued at room temperature for 24 h. The pH was adjusted to 7-8 with saturated Na₂CO₃. The mixture was extracted twice with ethyl acetate, and the combined organic phases were concentrated under reduced pressure to afford intermediate 21-4 (6.95 g, off-white solid).

### Synthesis of Intermediate 21-5

To a two-necked flask charged with 6.17 g of intermediate 21-4 (31.45 mmol) was added 60 mL of ethanol. Under stirring at room temperature, 3.36 g of pyrrolidine (47.18 mmol, 1.5 eq.) and 5.16 g of 4-(dimethylamino) benzaldehyde (34.59 mmol, 1.1 eq.) were added. The mixture was then heated to reflux and stirred for 19 h for reaction. After the reaction was completed, the mixture was cooled to room temperature, and ethanol was removed by concentration under reduced pressure. The concentrate was purified by silica gel column chromatography (PE: EA = 4:1) to afford intermediate 21-5 (2.5 g, red solid) in 24% yield.

### Synthesis of Intermediate 21-6

To a two-necked flask charged with 2.5 g of intermediate 21-5 (7.64 mmol) was added 25 mL of DMSO. Under stirring at room temperature, 291 mg of iodine (1.15 mmol, 0.15 eq.) was added. The mixture was stirred at 110 °C for 14 h and subsequently cooled to room temperature. 1 g of sodium thiosulfate pentahydrate solid was dissolved in 70 mL of water to prepare a solution. The reaction mixture was poured into the prepared sodium thiosulfate solution, and stirring was continued for 10 minutes. The mixture was then filtered under suction, and the filter cake was dried in an oven at 80 °C to afford intermediate 21-6 (3.87 g, brown solid).

### Synthesis of Intermediate 21-7

To a two-necked flask charged with 2.48 g of intermediate 21-6 (7.63 mmol) was added 250 mL of methanol. Under stirring at room temperature, 10.62 g of sodium hydrosulfite (61 mmol, 8.0 eq.) was added. The mixture was then heated to reflux and reacted for 16 h and subsequently cooled to room temperature. Methanol was removed by concentration under reduced pressure, and 100 mL of water was added to the residue for slurrying. The mixture was filtered under reduced pressure, and the filter cake was dried at 80 °C to afford intermediate 21-7 (4.67 g, reddish-brown solid).

### Synthesis of Compound S21

A 100 mL two-necked flask charged with 2 g of intermediate 21-7 (3.4 mmol) was added 20 mL of ethanol. Under stirring at room temperature, 2 mL of 40% glyoxal aqueous solution was added, and the mixture was stirred at 80 °C for reaction. The reaction progress was monitored by LC-MS. After stirring for 18 hours, the mixture was cooled to room temperature, and the reaction was quenched with 40 mL of water. The mixture was filtered under suction, and the filtrate was extracted three times with ethyl acetate. The combined organic phases were concentrated under reduced pressure, and the resulting concentrate was purified by silica gel column chromatography (eluent: EA:PE = 1:1) to afford compound S21 (110 mg, reddish-brown solid) in 5% yield.

¹H NMR (400 MHz, DMSO-*d*₆) δ 9.20 (dd, *J* = 9.6, 1.8 Hz, 2H), 8.33 (d, *J* = 8.9 Hz, 1H), 8.12-8.00 (m, 3H), 7.08 (s, 1H), 6.89 (d, *J =* 8.6 Hz, 2H), 3.06 (s, 6H).

ESI-MS m/z [M+1]⁺: 318.0.

### Example 22 Synthesis of Compound S22

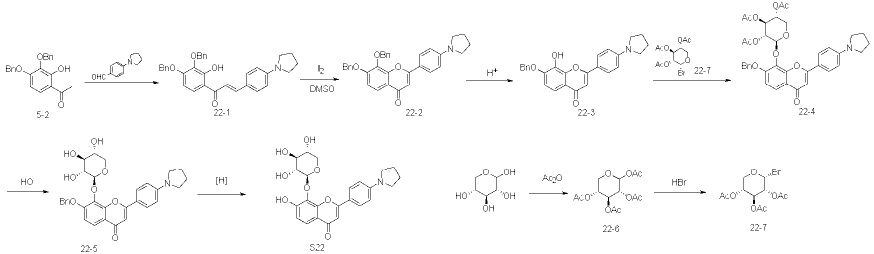

### Synthesis of Intermediate 22-1

To a 500 mL three-necked flask charged with 30 g of compound 5-2 (86 mmol, 1 eq.), 300 mL of ethanol was added. Under stirring at room temperature, 9.17 g of pyrrolidine (129 mmol, 1.5 eq.) and 16.60 g of 4-(pyrrolidin-1-yl) benzaldehyde (95 mmol, 1.1 eq.) were added. The reaction mixture was then subjected to magnetic stirring under reflux for 22 hours. The reaction progress was monitored by TLC. After the reaction mixture was cooled to room temperature, 45 mL of isopropyl ether and 15.5 g of glacial acetic acid were added to quench the reaction. Following an additional 20 minutes of stirring, the mixture was filtered under suction. The obtained filter cake was dried in an oven at 80 °C to afford intermediate 22-1 (39.84 g, yellow solid) in 92% yield.

### Synthesis of Intermediate 22-2

To a 500 mL two-necked flask loaded with 23.56 g of intermediate 22-1 (46.60 mmol), 100 mL of DMSO was added, followed by the introduction of 1.77 g of iodine (6.99 mmol, 0.15 eq.). The mixture was stirred magnetically at 110 °C for 5 hours, and the reaction process was monitored by TLC. A solution was prepared by dissolving 6.94 g of sodium thiosulfate pentahydrate in 300 mL of water for subsequent use. To the reaction mixture, 200 mL of DCM was added for dilution. The diluted reaction solution was poured into the pre-prepared sodium thiosulfate solution to quench the reaction. After liquid separation, the organic phase was washed once with 200 mL of water, concentrated under reduced pressure, and then purified by silica gel column chromatography (eluent: EA:PE = 1:3) to afford intermediate 22-2 (5.21 g, yellow solid) in 22% yield.

### Synthesis of Intermediate 22-3

To a 250 mL single-necked flask charged with 7.40 g of intermediate 22-2 (14.69 mmol), 20 mL of trifluoroacetic acid was added. The reaction was carried out with magnetic stirring at 40 °C for 3 hours, and then quenched by the addition of 100 mL of water, resulting in the precipitation of a black oil. The black oil was subjected to silica gel column chromatography (eluent: EA:PE = 1:3) to give intermediate 22-3 (1.07 g, yellow solid) in 18% yield.

### Synthesis of Intermediate 22-4

To a 100 mL single-necked flask charged with 1.07 g of intermediate 22-3, 30 mL of chloroform and 30 mL of water were added. Subsequently, 1.0 g of TBAB (3.11 mmol, 1.2 eq.), 1.79 g of K₂CO₃ (12.95 mmol, 5.0 eq.) and 2.64 g of intermediate 22-7 (7.77 mmol, 3.0 eq.) were sequentially added into the mixture. The reaction was allowed to proceed with magnetic stirring at room temperature for 20 hours. After liquid separation, the organic phase was concentrated under reduced pressure, and the resulting concentrate was purified by silica gel column chromatography (eluent: EA:PE = 1:3) to afford intermediate 22-4 (1.05 g, yellow solid) in 60.3% yield.

### Synthesis of Intermediate 22-5

To a 250 mL two-necked flask loaded with 1.05 g of intermediate 22-4 (1.56 mmol), 30 mL of methanol and 15 mL of water were added, followed by the introduction of 499.32 mg of NaOH (12.48 mmol, 8.0 eq.). The reaction was stirred magnetically at room temperature for 2 hours, and then quenched by adding 60 mL of water and 4 mL of glacial acetic acid. The mixture was extracted with 30 mL of ethyl acetate, and the aqueous phase was re-extracted with another 30 mL of ethyl acetate. The combined organic phases were concentrated under reduced pressure to yield intermediate 22-5 (1.11 g, yellow solid).

### Synthesis of Intermediate 22-6

To a 250 mL two-necked flask charged with 10 g of (3R,4S,5R)-oxane-2,3,4,5-tetrol (66.60 mmol), 50 mL of pyridine was added. The flask was placed in a cold well maintained at 0 °C, and the mixture was stirred magnetically. A total of 50 mL of acetic anhydride was slowly added dropwise to the reaction system over approximately 2 hours. The reaction was then continued with stirring at 0 °C for 19 hours. The reaction mixture was slowly poured into 200 mL of water to quench the reaction, and the resulting mixture was extracted with 200 mL of MTBE. After liquid separation, the organic phase was washed once with 200 mL of water, followed by a wash with 100 mL of 50% acetic acid solution. The organic phase was further washed with saturated copper sulfate solution until the aqueous phase no longer turned deep blue. Finally, the organic phase was washed once with 50 mL of water, dried over anhydrous sodium sulfate, and filtered. The filtrate was concentrated under reduced pressure to afford intermediate 22-6 (21.58 g, white solid) in 100% yield.

### Synthesis of Intermediate 22-7

To a 250 mL two-necked flask charged with 10 g of intermediate 22-6 (31.42 mmol), 100 mL of DCM was added, followed by the addition of 60 mL of 33% HBr in acetic acid (248 mmol, 8.0 eq.). The reaction was carried out with magnetic stirring at room temperature for 2 hours. 500 mL of water was placed in a 1 L beaker, which was then placed in a cold well at 0 °C and stirred magnetically. The reaction mixture was slowly poured into the stirred water to quench the reaction. After liquid separation, the organic phase was washed once with 100 mL of water, once with 50 mL of saturated sodium bicarbonate solution, and once more with 100 mL of saturated sodium chloride solution. The organic phase was dried over anhydrous sodium sulfate, filtered, and concentrated under reduced pressure to give intermediate 22-7 (7.03 g, white solid) in 66% yield.

### Synthesis of Compound S22

To a 100 mL two-necked flask loaded with 1.11 g of intermediate 22-5 (2.03 mmol), 550 mg of 10% Pd/C was added. The flask was evacuated and purged with hydrogen gas for three cycles. 50 mL of THF was measured out with a syringe and then added to the system, and the reaction was allowed to proceed with magnetic stirring at room temperature for 23 hours. The mixture was filtered under suction, and the filtrate was concentrated under reduced pressure. The resulting concentrate was purified by silica gel column chromatography (eluent: DCM: MeOH = 30:1) to afford compound S22 (160 mg, yellow solid) in 17% yield.

ES-MS m/z [M+1]⁺: 456.1.

### Example 23 Synthesis of Compound S23

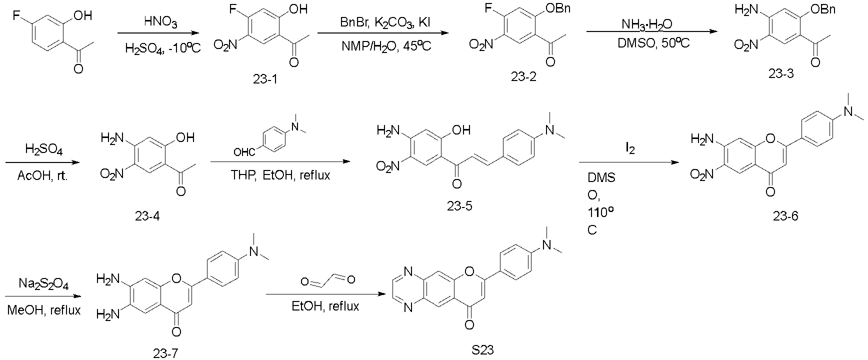

### Synthesis of Intermediate 23-1

To a 2 L two-necked flask charged with 50.88 g of 1-(4-fluoro-2-hydroxyphenyl) ethen-1-one (330.09 mmol), 205 mL of concentrated sulfuric acid was added. Under stirring at -10 °C, 34 mL of concentrated nitric acid was added dropwise over 1 hour. The reaction progress was monitored by TLC (developing solvent: EA: PE=1:3). After completion of nitric acid addition, stirring was continued for 10 minutes. Subsequently, 1 L of water was slowly added dropwise to quench the reaction. The mixture was extracted with 500 mL of ethyl acetate, followed by liquid separation. The organic phase was concentrated under reduced pressure to afford intermediate 23-1 (60 g, black liquid).

### Synthesis of Intermediate 23-2

To a 2 L two-necked flask loaded with 60 g of intermediate 23-1 (300 mmol), 360 mL of NMP was added. Under stirring at 45 °C, 82.92 g of K₂CO₃ (600 mmol, 2.0 eq.), 7.47 g of KI (45 mmol, 0.15 eq.), 80 mL of water and 76.91 g of benzyl bromide (450 mmol, 1.5 eq.) were sequentially added. The reaction mixture was then stirred at 45 °C for 17 hours. 1.5 L of water was placed in a 5 L beaker, and the reaction mixture was poured into the stirred water. The mixture was extracted twice with 500 mL of EA each time. The combined organic phases were washed three times with 500 mL of water each time, then concentrated under reduced pressure. To the resulting concentrate, 100 mL of methanol was added, and the mixture was slurried at room temperature, followed by filtration under suction. The filter cake was dried in an oven at 80 °C for 2 hours to yield intermediate 23-2 (18.0 g, yellow solid) in 21% yield.

### Synthesis of Intermediate 23-3

To a 500 mL two-necked flask charged with 10 g of intermediate 23-2 (34.57 mmol), 100 mL of DMSO was added. Under stirring at room temperature, 10 mL of ammonia solution was introduced, and the reaction was carried out with stirring at 50 °C for 2 hours. The reaction was quenched by adding 100 mL of water, resulting in the precipitation of a large amount of yellow solid. The solid was collected by filtration, and the filter cake was dried in an oven at 80 °C for 4 hours to obtain intermediate 23-3 (9.43 g, yellow solid) in 95% yield.

### Synthesis of Intermediate 23-4

To a 100 mL single-necked flask charged with 9.43 g of intermediate 23-3 (32.94 mmol), 40 mL of acetic acid was added. Under stirring at room temperature, 10 mL of concentrated sulfuric acid was added, and the reaction was allowed to proceed with stirring at room temperature. The reaction process was monitored by LC-MS. After 21 hours of stirring, the pH of the reaction mixture was adjusted to 6-7 with saturated NaHCO₃ solution. The mixture was filtered, and the filter cake was dried in an oven at 80 °C for 3 hours to give intermediate 23-4 (5.69 g, yellow solid) in 88% yield.

### Synthesis of Intermediate 23-5

To a 250 mL two-necked flask loaded with 4.45 g of intermediate 23-4 (22.69 mmol), 45 mL of ethanol was added. Under stirring at room temperature, 2.42 g of pyrrolidine (34.03 mmol, 1.5 eq.) and 3.72 g of 4-(dimethylamino) benzaldehyde (24.95 mmol, 1.1 eq.) were sequentially added. The mixture was then stirred under reflux for 18 hours, and subsequently cooled to room temperature. 10 mL of isopropyl ether was added to the reaction mixture, followed by the addition of 3 g of acetic acid to quench the reaction. The mixture was filtered, and the filter cake was dried in an oven at 80 °C for 5 hours to afford intermediate 23-5 (5.92 g, reddish-brown solid) in 65% yield.

¹H NMR (400 MHz, DMSO-*d*₆) δ 13.81 (s, 1H), 8.93 (s, 1H), 7.89 (s, 2H), 7.84-7.75 (m, 3H), 7.68 (d, *J =* 15.1 Hz, 1H), 6.75 (d, *J* = 9.0 Hz, 2H), 6.32 (s, 1H), 3.03 (s, 6H).

### Synthesis of Intermediate 23-6

To a 200 mL two-necked flask charged with 4.63 g of intermediate 23-5 (14.14 mmol), 40 mL of DMSO was added. Under stirring at room temperature, 538.33 mg of iodine (2.12 mmol, 0.15 eq.) was added, and the reaction was carried out with stirring at 110 °C. The reaction progress was monitored by LC-MS. After 7 hours of stirring, the mixture was cooled to room temperature. A solution was prepared by dissolving 2 g of sodium thiosulfate pentahydrate in 100 mL of water. The reaction solution was poured into the prepared sodium thiosulfate solution to quench the reaction, followed by stirring at room temperature for 1 hour. The mixture was filtered, and the filter cake was washed once with 20 mL of methanol, then dried in an oven at 80 °C for 5 hours to yield intermediate 23-6 (5.12 g, brown solid) in 100% yield.

### Synthesis of Intermediate 23-7

To a 1 L two-necked flask charged with 4 g of intermediate 23-6 (12.29 mmol), 400 mL of methanol was added. Under stirring at room temperature, 17.13 g of sodium hydrosulfite (98.39 mmol, 8.0 eq.) was added, and the mixture was stirred under reflux. The reaction process was monitored by LC-MS. After 20 hours of stirring, the mixture was cooled to room temperature, then concentrated under reduced pressure. To the concentrate, 100 mL of water was added, and the mixture was slurried at room temperature, followed by filtration under suction. The filter cake was dried in an oven at 80 °C for 2 hours to obtain intermediate 23-7 (3.46 g, black solid) in 93% yield.

### Synthesis of Compound S23

To a 100 mL two-necked flask loaded with 1 g of intermediate 23-7 (3.4 mmol), 10 mL of ethanol was added. Under stirring at room temperature, 1 mL of 40% aqueous glyoxal solution was added, and the reaction was carried out with stirring at 80 °C. The reaction progress was monitored by LC-MS. After 17 hours of stirring, the mixture was cooled to room temperature, and the reaction was quenched by adding 20 mL of water, resulting in the precipitation of a black solid. The mixture was filtered under suction, and the filtrate was extracted three times with ethyl acetate. The combined organic phases were concentrated under reduced pressure to obtan crude product, which was then purified by silica gel column chromatography (eluent: EA:PE = 1:1) to afford compound S23 (90 mg, reddish-brown solid) in 8% yield.

¹H NMR (400 MHz, DMSO-*d*₆) δ 9.09 (d, *J* = 1.7 Hz, 1H), 9.05 (d, *J* = 1.7 Hz, 1H), 8.68 (s, 1H), 8.46 (s, 1H), 8.06 (d, *J* = 9.1 Hz, 2H), 6.95 (s, 1H), 6.86 (d, *J* = 9.2 Hz, 2H), 3.07 (s, 7H).

ES-MS m/z [M+1]⁺: 318.0.

### Example 24 Synthesis of Compound S24

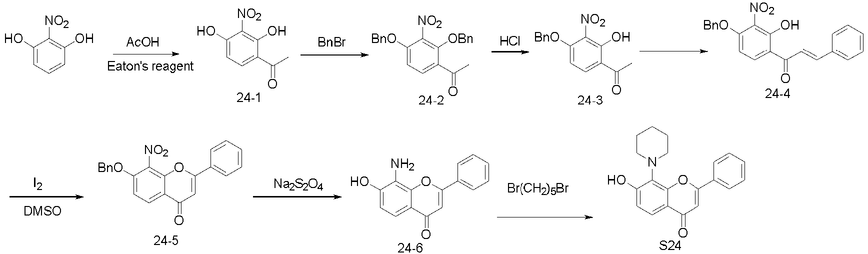

### Synthesis of Intermediate 24-1

To a 500 mL two-necked flask charged with 20 g of 2-nitrobenzene-1,3-diol (128.94 mmol), 245.18 g of Eaton's reagent was added, followed by the introduction of 11.61 g of glacial acetic acid (193.41 mmol, 1.5 eq.) under stirring at room temperature. The reaction mixture was then stirred at 80 °C for 1.5 hours and subsequently cooled to room temperature. At 0 °C, the reaction mixture was slowly poured into 500 mL of water, and 500 mL of DCM was added thereafter. The mixture was filtered under suction, and the filtrate was subjected to liquid separation. The organic phase was washed once with 300 mL of water and concentrated under reduced pressure to afford intermediate 24-1 (27.43 g). The resulting concentrate was used directly in the next step without further purification.

### Synthesis of Intermediate 24-2

Intermediate 24-1 (128.94 mmol) was transferred to a 1 L two-necked flask, followed by the addition of 130 mL of NMP. Under stirring at room temperature, 53.46 g of K₂CO₃ (386.82 mmol, 3.0 eq.), 3.21 g of KI (19.34 mmol, 0.15 eq.) and 30 mL of water were added sequentially, followed by the addition of 55.13 g of benzyl bromide (322.35 mmol, 2.5 eq.). The reaction mixture was stirred at 40 °C for 1 hour and then cooled to room temperature. The reaction was quenched by adding 500 mL of water, and the mixture was extracted with 250 mL of ethyl acetate. After liquid separation, the aqueous phase was re-extracted with another 250 mL of ethyl acetate. After another liquid separation, the combined organic phases were concentrated under reduced pressure to yield intermediate 24-2 as a black oil (69.94 g). The resulting oil was used directly in the subsequent reaction without purification.

### Synthesis of Intermediate 24-3

Intermediate 24-2 (128.94 mmol) was transferred to a 500 mL two-necked flask, followed by the addition of 150 mL of glacial acetic acid. Under stirring at room temperature, 30 mL of concentrated hydrochloric acid was added, and the reaction was allowed to proceed with stirring at room temperature for 20 hours. Then the mixture was filtered under suction to obtain a viscous solid. The solid was dissolved in 500 mL of DCM, followed by a wash with 300 mL of water. The organic phase was washed three times with 200 mL of water each time, and then concentrated under reduced pressure to give intermediate 24-3 as a yellow oil (60.97 g). The oil was used directly in the next step without further purification.

### Synthesis of Intermediate 24-4

Intermediate 24-3 (128.94 mmol) was transferred to a 1 L two-necked flask, followed by the addition 500 mL of methanol. Under stirring at room temperature, 50.64 g of KOH (902.58 mmol, 7.0 eq.) and 15.05 g of benzaldehyde (141.83 mmol, 1.1 eq.) were added. The reaction mixture was stirred under reflux for 20 hours and then cooled to room temperature. The reaction was quenched by adding 65 g of glacial acetic acid, and stirring was continued at room temperature for 30 minutes. The mixture was filtered under suction, and the filtered solid was dried at 80 °C to afford intermediate 24-4 (11.90 g, yellow solid), with an overall yield of 24% over four steps.

### Synthesis of Intermediate 24-5

To a 250 mL two-necked flask charged with 6.0 g of intermediate 24-4 (15.98 mmol), 40 mL of DMSO was added, followed by the introduction of 608.38 mg of iodine (2.4 mmol, 0.15 eq.) under stirring at room temperature. The reaction mixture was stirred at 110 °C for 1.5 hours and then cooled to room temperature. A solution was prepared by dissolving 2 g of sodium thiosulfate pentahydrate in 150 mL of water. The prepared sodium thiosulfate solution was added to the reaction mixture to quench the reaction, and stirring was continued for 20 minutes. The mixture was filtered under suction, and the filtered solid was dried at 80 °C to yield intermediate 24-5 (3.97 g, yellow solid) in 66% yield.

### Synthesis of Intermediate 24-6

To a 250 mL two-necked flask charged with 3.97 g of intermediate 24-5 (3.97 mmol), 50 mL of methanol was added, followed by the introduction of 11.10 g of sodium hydrosulfite (63.78 mmol, 6.0 eq.) under stirring at room temperature. The reaction mixture was stirred under reflux for 2 hours and then cooled to room temperature, followed by concentration under reduced pressure. The concentrate was slurried with 100 mL of water, and filtered under suction. The resulting solid was dried at 80 °C to afford intermediate 24-6 (2.79 g, yellow solid) in 74% yield.

### Synthesis of Compound S24

To a 100 mL two-necked flask charged with 1 g of intermediate 24-6 (3.95 mmol), 30 mL of acetonitrile was added. Under stirring at room temperature, 1.99 g of K₂CO₃ (7.90 mmol, 2.0 eq.), 1.31 g of KI (7.90 mmol, 2.0 eq.) and 907.94 mg of GRK-022-003-4 (3.95 mmol, 1.0 eq.) were added. The reaction mixture was stirred under reflux for 17 hours and then cooled to room temperature, followed by concentration under reduced pressure. The concentrate was dissolved in 100 mL of water and 50 mL of EA, and the mixture was subjected to liquid separation. The organic phase was concentrated under reduced pressure, and the resulting concentrate was purified by silica gel column chromatography (eluent: EA: PE = 1:4) to afford compound S24 (120 mg, white solid) in 9% yield.

¹H NMR (400 MHz, DMSO-*d*₆) δ 9.79 (s, 1H), 8.22-8.12 (m, 2H), 7.78 (d, *J =* 8.7 Hz, 1H), 7.72-7.64 (m, 3H), 7.06 (d, *J* = 8.7 Hz, 1H), 6.97 (s, 1H), 3.28-3.16 (m, 4H), 1.85-1.61 (m, 6H).

ES-MS m/z [M+1]⁺: 322.12.

### Example 25 Synthesis of Compound S25

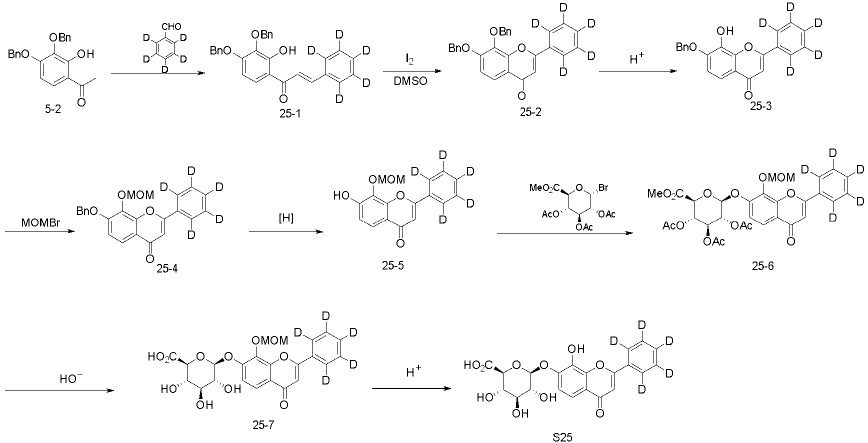

### Synthesis of Intermediate 25-1

To a 500 mL two-necked flask charged with 14.10 g of intermediate 5-2 (40.18 mmol), 180 mL of methanol was added. Under stirring at room temperature, 15.78 g of KOH (281.29 mmol, 7.0 eq) and 4.91 g of 5-deuteriobenzaldehyde (44.20 mmol, 1.1 eq) were added. The reaction mixture was refluxed for 17 hours. After the reaction was completed, the reaction mixture was cooled to room temperature, and 27 mL of glacial acetic acid was added to quench the reaction, followed by continuous stirring for 1 hour. The mixture was filtered under suction, and the obtained solid was dried in an oven at 50 °C to afford intermediate 25-1 (16.15 g, yellow solid) in 91% yield.

### Synthesis of Intermediate 25-2

To a 250 mL two-necked flask loaded with 16.15 g of intermediate 25-1 (36.58 mmol), 80 mL of DMSO and 1.39 g of iodine (5.49 mmol, 0.15 eq) were added. The reaction mixture was then magnetically stirred at 110 °C for 18 hours. After completion of the reaction, the mixture was cooled to room temperature. A solution was prepared by dissolving 6.0 g of sodium thiosulfate pentahydrate in 160 mL of water for subsequent use. The prepared sodium thiosulfate solution was added to the cooled reaction mixture to quench the reaction. After further stirring for 30 minutes, the mixture was filtered under suction. The filter cake was rinsed once with 20 mL of methanol and then dried in an oven at 50 °C to yield intermediate 25-2 (14.84 g, pale yellow solid) in 92% yield.

### Synthesis of Intermediate 25-3

To a 250 mL two-necked flask charged with 14.84 g of intermediate 25-2 (33.76 mmol), 60 mL of TFA was added. The reaction was carried out with magnetic stirring at 40 °C for 3 hours, with progress monitored by LC-MS. After completion of the reaction, it was quenched by adding 150 mL of water and further stirred overnight, resulting in the precipitation of a large amount of solid. The mixture was filtered under suction, and the obtained solid was transferred to a 250 mL two-necked flask. 60 mL of methanol was added, and the mixture was slurried under reflux for 30 minutes. After completion of slurrying, the mixture was cooled to room temperature, and then filtered under suction. The obtained solid was dried in an oven at 50 °C to give intermediate 25-3 (7.02 g, yellow solid) in 59% yield.

### Synthesis of Intermediate 25-4

To a 250 mL two-necked flask charged with 7.02 g of intermediate 25-3 (20.09 mmol), 30 mL of DMF was added. Under stirring at room temperature, 1.61 g of NaH (40.18 mmol, 2.0 eq) and 3.01 g of MOMBr (24.11 mmol, 1.2 eq) were sequentially added. The reaction was performed with magnetic stirring at room temperature for 17 hours, with progress monitored by LC-MS. Upon completion, the reaction was quenched by adding 7.2 g of glacial acetic acid. 100 mL of water was added, and the mixture was stirred for another 10 minutes, leading to the precipitation of a large amount of solid. The mixture was filtered under suction, and the obtained solid was dried at 80 °C to afford intermediate 25-4 (7.02 g, yellow solid) in 89% yield.

### Synthesis of Intermediate 25-5

To a 250 mL two-necked flask loaded with 7.02 g of intermediate 25-4 (17.84 mmol), 2.8 g of 10% Pd/C was added. The flask was evacuated and purged with hydrogen gas for three cycles. Subsequently, 40 mL of tetrahydrofuran was added, and the reaction was carried out with magnetic stirring at room temperature for 18 hours, with progress monitored by LC-MS. The mixture was then filtered under suction, and the filtrate was concentrated under reduced pressure. The resulting concentrate was purified by silica gel column chromatography (eluent: EA: PE = 1:1) to obtain intermediate 25-5 (4.41 g, white solid) in 81% yield.

### Synthesis of Intermediate 25-6

To a 250 mL two-necked flask charged with 2.0 g of intermediate 25-5 (6.59 mmol), 50 mL of chloroform was added. Under stirring at room temperature, 7.85 g of (2R,3R,4S,5S,6S)-2-bromo-6-(methoxycarbonyl) tetrahydro-2H-pyran-3,4,5-triacetate (19.77 mmol, 3.0 eq), 2.55 g of TBAB (7.91 mmol, 1.2 eq), 50 mL of water and 4.55 g of K₂CO₃ (32.95 mmol, 5.0 eq) were sequentially added. The reaction was continued with magnetic stirring at room temperature for 22 hours, and its progress was monitored by LC-MS. The mixture was then subjected to direct liquid separation, and the organic phase was concentrated under reduced pressure. The resulting concentrate was purified by silica gel column chromatography to yield intermediate 25-6 (3.2 g, yellow solid) in 80% yield.

### Synthesis of Intermediate 25-7

To a 200 mL two-necked flask charged with 3.20 g of intermediate 25-6 (5.16 mmol), 30 mL of methanol and 15 mL of water were added, followed by the introduction of 1.65 g of NaOH (41.28 mmol, 4.0 eq). The reaction was carried out with magnetic stirring at room temperature for 1 hour, and its progress was monitored by LC-MS. 45 mL of water was added to the system, followed by the addition of 5.0 g of glacial acetic acid to quench the reaction. The mixture was extracted with 60 mL of EA, and after liquid separation, the aqueous phase was concentrated under reduced pressure to obtain intermediate 25-7 as a concentrate, which was used directly in the subsequent step without further purification.

### Synthesis of Compound S25

To a 100 mL single-necked flask loaded with 2.47 g of intermediate 25-7 (5.16 mmol), 10 mL of TFA was added. The reaction was performed with magnetic stirring at room temperature for 1 hour, and its progress was monitored by LC-MS. 70 mL of MTBE was added to the system, resulting in the precipitation of a white solid. The mixture was filtered under suction, and the filtrate was concentrated under reduced pressure. The filter cake was dried in an oven at 50 °C. The resulting concentrate and the dried filter cake was purified by silica gel column chromatography to afford compound S25 (1.46 g, white solid) in 65% yield.

ES-MS m/z [M+1]⁺: 436.02.

### Example 26 Synthesis of Compound S26

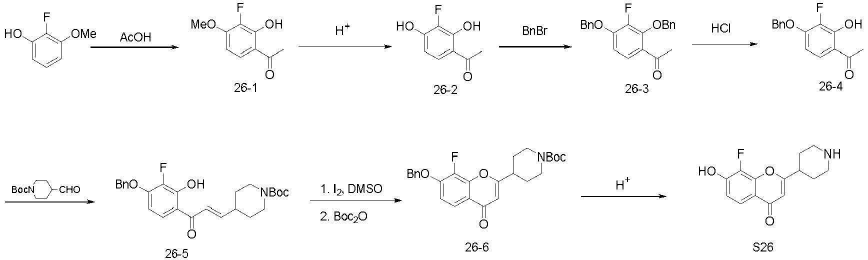

### Synthesis of Intermediate 26-1

To a 1 L two-necked flask charged with 20 g of 2-fluoro-3-methoxyphenol (141 mmol), 400 mL of glacial acetic acid was added. The mixture was Under magnetic stirring at room temperature, 191 g of ZnCl₂ (1.41 mol, 10.0 eq.) was added. The reaction mixture was then heated to reflux, and stirred for 23 hours, with the reaction progress monitored by LC-MS. After the reaction was completed, the mixture was cooled to room temperature and concentrated under reduced pressure to remove acetic acid. The resulting concentrate was quenched by adding 400 mL of water, followed by extraction with 400 mL of ethyl acetate. The separated organic phase was washed twice with saturated sodium bicarbonate solution and concentrated under reduced pressure to afford intermediate 26-1 (21.54 g, reddish-brown solid) in 83% yield.

### Synthesis of Intermediate 26-2

To a 500 mL two-necked flask loaded with 20 g of intermediate 26-1 (110 mmol), 200 mL of dichloroethane was added. Under magnetic stirring at 0 °C, 85.2 g of AlCl₃ (640 mmol, 6.0 eq.) was slowly added, followed by 50.2 g of ethane-1,2-dithiol (530 mmol, 5.0 eq.). The reaction mixture was heated to reflux and stirred for 1 hour, with the reaction progress monitored by LC-MS. After the reaction was completed, the mixture was cooled to -10 °C, and then 100 mL of concentrated hydrochloric acid was slowly added dropwise. Upon completion of the acid addition, the mixture was filtered under reduced pressure. The filtrate was subjected to liquid separation, and the aqueous phase was re-extracted with 200 mL of dichloromethane. The two organic phases were combined. The filter cake was slurried with 300 mL of methanol for 1 hour and then filtered under suction to obtain a filtrate. The filtrate was combined with the organic phases, followed by concentration under reduced pressure. The concentrate was purified by silica gel column chromatography (eluent: EA: PE = 1:5) to yield intermediate 26-2 (9.45 g, yellow solid) in 51% yield.

### Synthesis of Intermediate 26-3

To a 500 mL two-necked flask charged with 9.45 g of intermediate 26-2 (55.5 mmol), 60 mL of NMP was added. Under magnetic stirring at room temperature, 19.2 g of K₂CO₃ (140 mmol, 2.5 eq.), 1.38 g of KI (8.33 mmol, 0.15 eq.), 15 mL of water and 23.7 g of benzyl bromide (140 mmol, 2.5 eq.) were sequentially added. The mixture was stirred at 40 °C for 1 hour, the progress monitored by LC-MS. After the reaction was completed, the reaction was quenched by adding 300 mL of water, followed by extraction twice with 150 mL of EA each time. The combined organic phases were washed twice with water, concentrated under reduced pressure, and then purified by silica gel column chromatography (eluent: EA:PE = 1:5) to obtain intermediate 26-3 (7.42 g, yellow solid) in 38% yield.

### Synthesis of Intermediate 26-4

To a 250 mL two-necked flask charged with 7.32 g of intermediate 26-3 (20.90 mmol), 200 mL of glacial acetic acid was added. Under magnetic stirring at room temperature, 20 mL of concentrated hydrochloric acid was incorporated. The mixture was stirred at room temperature for 22 hours, with its progress monitored by LC-MS. After the reaction was completed, the reaction mixture was directly filtered under suction. The filter cake was dried in an oven at 80 °C to afford intermediate 26-4 (3.01 g, white solid) in 55% yield.

### Synthesis of Intermediate 26-5

To a 100 mL two-necked flask loaded with 2.9 g of intermediate 26-4 (11.1 mmol), 30 mL of ethanol was added. Under magnetic stirring at room temperature, 1.98 g of pyrrolidine (27.9 mmol, 2.5 eq.) and 3.56 g of 1-tert-butoxycarbonyl piperidine-4-carbaldehyde (16.7 mmol, 1.5 eq.) were added successively. The reaction mixture was heated to reflux, and stirred for 20 hours, with the progress monitored by LC-MS, after which heating was stopped. When the system was cooled to room temperature, the reaction mixture was directly concentrated under reduced pressure. The resulting concentrate was purified by silica gel column chromatography (eluent: EA:PE = 5:1) to yield intermediate 26-5 (4.91 g, yellow oil) in 97% yield.

### Synthesis of Intermediate 26-6

To a 100 mL single-necked flask charged with 2.5 g of intermediate 26-5 (5.49 mmol), 25 mL of DMSO and 2.79 g of iodine (10.98 mmol, 2.0 eq.) were added. The reaction was carried out at 110 °C for 1 hour, with its progress monitored by LC-MS, after which heating was stopped. When the system was cooled to room temperature, 2.39 g of Boc₂O (10.98 mmol, 2.0 eq.) and 1.11 g of TEA (10.98 mmol, 2.0 eq.) were added, and stirring was continued at room temperature for another 17 hours. After the reaction was completed, 50 mL of saturated sodium thiosulfate solution and 50 mL of EA were added to the system. The mixture was subjected to liquid separation, and the organic phase was concentrated under reduced pressure. The concentrate was purified by silica gel column chromatography (eluent: EA:PE = 1:3) to obtain intermediate 26-6 (570 mg, yellow solid) in 23% yield.

### Synthesis of Compound S26

To a 50 mL single-necked flask charged with 50 mg of intermediate 26-6 (0.11 mmol), 2 mL of TFA was added. The mixture was stirred at 80 °C for 2 hours, with the progress monitored by LC-MS, after which heating was stopped. When the system was cooled to room temperature, the reaction mixture was directly concentrated under reduced pressure. The resulting concentrate was adjusted to pH 7-8 with saturated sodium bicarbonate solution, then concentrated under reduced pressure again. The residue was purified by Pre-TLC (developing solvent: methanol) to afford compound S26 (20 mg, yellow solid) in 69% yield.

¹H NMR (400 MHz, DMSO-*d*₆) δ 7.29 (d, *J* = 9.0 Hz, 1H), 6.45 (t, *J* = 8.5 Hz, 1H), 5.75 (s, 1H), 3.14-2.94 (m, 2H), 2.69-2.53 (m, 3H), 1.83 (dd, *J* = 13.2, 3.4 Hz, 2H), 1.51 (qd, *J* = 12.3, 4.0 Hz, 2H).

ES-MS m/z [M+1]⁺: 264.20.

### Example 27 Synthesis of Compound S27

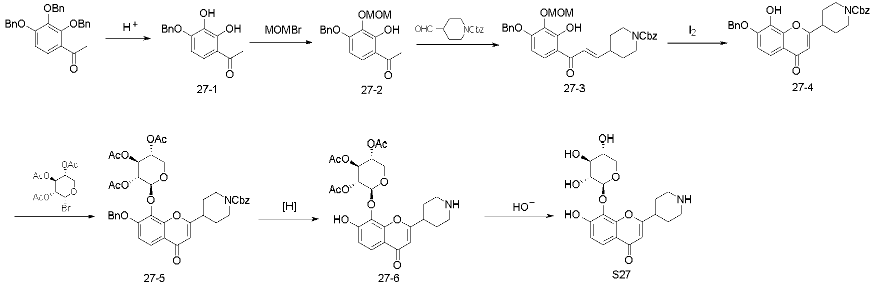

### Synthesis of Intermediate 27-1

To a 500 mL two-necked flask charged with 41.35 g of 1-(2,3,4-tris(benzyloxy) phenyl) ethen-1-one (94.30 mmol), 100 mL of dichloromethane was added. Under magnetic stirring at room temperature, 118.58 g of trifluoroacetic acid (1.04 mol, 11.0 eq.) was added. The mixture was stirred at 30 °C for 3 hours. The reaction was quenched by adding 300 mL of water, and the mixture was subjected to liquid separation. The organic phase was washed once with 200 mL of water, separated again, and the organic phase was concentrated under reduced pressure. To the resulting concentrate, 100 mL of isopropyl ether was added, and the mixture was slurried at room temperature for 17 hours. The mixture was filtered under suction, and the obtained solid was dried at 50 °C to afford intermediate 27-1 (11.42 g, yellow solid) in 47% yield.

### Synthesis of Intermediate 27-2

To a 500 mL two-necked flask loaded with 11.42 g of intermediate 27-1 (44.22 mmol), 100 mL of dichloromethane was added. Under magnetic stirring at -10 °C, 9.33 g of N,N-diisopropylethylamine (75.18 mmol, 1.7 eq.) was added. While the temperature was controlled below 0 °C, 9.39 g of MOMBr (75.18 mmol, 1.7 eq.) was slowly added dropwise. After the completion of dropwise addition, the reaction mixture was stirred for 2 hours while the temperature was maintained below 10 °C, with the reaction progress monitored by LC-MS. After the reaction was completed, the system was quenched by adding 200 mL of water. The aqueous phase was discarded after liquid separation, and the organic phase was washed once more with 200 mL of water, separated, and concentrated under reduced pressure to yield intermediate 27-2 (14.32 g, yellow solid).

### Synthesis of Intermediate 27-3

To a 50 mL two-necked flask containing 2.0 g of intermediate 27-2 (6.6 mmol), 20 mL of ethanol was added. Under magnetic stirring at room temperature, 1.17 g of pyrrolidine (16.5 mmol, 2.5 eq.) and 2.45 g of benzyl 4-formylpiperidine-1-carboxylate (9.9 mmol, 1.5 eq.) were added. The reaction mixture was then stirred under reflux for 3 hours, with the progress monitored by LC-MS. After the reaction was completed, the mixture was cooled to room temperature, and the reaction mixture was concentrated under reduced pressure. The resulting concentrate was purified by column chromatography (eluent: EA: PE = 1:2) to obtain intermediate 27-3 (2.61 g, yellow oil) in 74% yield.

### Synthesis of Intermediate 27-4

To a 100 mL two-necked flask charged with 2.61 g of intermediate 27-3 (4.91 mmol), 40 mL of DMSO was added. Under magnetic stirring at room temperature, 373.86 mg of iodine (1.47 mmol, 0.3 eq.) was added. The mixture was stirred at 110 °C for 1 hour, with the progress monitored by LC-MS. After the reaction was completed, the mixture was cooled to room temperature, and the reaction was quenched by adding 120 mL of water, followed by extraction with 60 mL of EA. After liquid separation, the organic phase was washed once with 20 mL of saturated sodium thiosulfate solution, separated again, and concentrated under reduced pressure. The concentrate was purified by silica gel column chromatography (eluent: EA:PE = 1:3) to yield intermediate 27-4 (1.50 g, yellow solid) in 63% yield.

### Synthesis of Intermediate 27-5

To a 100 mL two-necked flask loaded with 1 g of intermediate 27-4 (2.18 mmol), 20 mL of chloroform was added. Under magnetic stirring at room temperature, 2.22 g of intermediate 22-7 (6.54 mmol, 3.0 eq.), 843.35 mg of TBAB (2.62 mmol, 1.2 eq.), 20 mL of water and 1.30 g of K₂CO₃ (10.9 mmol, 5.0 eq.) were sequentially added. The reaction was continued with stirring at room temperature for 18 hours, and the progress was monitored by LC-MS. The reaction mixture was subjected to liquid separation, and the organic phase was concentrated under reduced pressure. The resulting concentrate was purified by silica gel column chromatography (eluent: EA:PE = 1:1) to afford intermediate 27-5 (1.25 g, yellow solid) in 82% yield.

### Synthesis of Compound S27

To a 50 mL two-necked flask charged with 160 mg of Intermediate 27-5 (0.22 mmol), 16 mg of Pd/C was added, and the system was purged with hydrogen gas for three cycles. Subsequently, 15 mL of THF was added, and the reaction mixture was stirred at room temperature for 24 hours. The reaction progress was monitored by LC-MS. After the reaction was completed, the catalyst was removed by filtration under suction. To the filtrate, 10 mL of water and 10 mL of methanol were added, and 35.2 mg of NaOH (0.88 mmol, 4.0 eq.) was added under stirring at room temperature. Stirring was continued for 6 hours at room temperature, and the reaction was then quenched by adding 52.8 mg of AcOH. The quenched solution was concentrated under reduced pressure, and the residue was purified by Pre-TLC (eluent: MeOH: DCM = 10:1) to obtain compound S27 (31 mg, white solid) in 37% yield.

¹H NMR (400 MHz, Methanol-*d₄*) δ 7.59 (d, *J* = 9.0 Hz, 1H), 6.72 (d, *J* = 9.0 Hz, 1H), 5.98 (s, 1H), 5.17-5.06 (m, 1H), 4. 54 (d, *J =* 7.4 Hz, 1H), 3.98-3.85 (m, 1H), 3.62-3.53 (m, 1H), 3.50 (dd, *J =* 9.0*,* 7.5 Hz, 1H), 3.38 (d, *J =* 8.8 Hz, 1H), 3.22 (dd, *J* = 11.5, 10.1 Hz, 1H), 3.18-3.10 (m, 2H), 2.83-2.66 (m, 3H), 2.11-2.04 (m, 1H), 2.02-1.94 (m, 1H), 1.81-1.68 (m, 2H).

ES-MS m/z [M+1]⁺: 394.10.

### Example 28 Synthesis of Compound S28

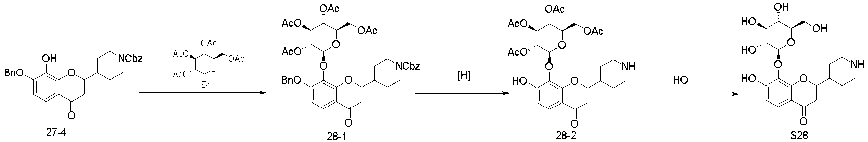

### Synthesis of Intermediate 28-1

To a 50 mL two-necked flask charged with 500 mg of intermediate 27-4 (1.03 mmol), 10 mL of chloroform was added. Under magnetic stirring at room temperature, 1.27 g of (2R, 3R, 4S, 5R, 6R)-2-(acetoxymethyl)-6-bromotetrahydro-2H-pyran-3,4,5- triyl triacetate (3.09 mmol, 3.0 eq.), 399.75 mg of TBAB (1.24 mmol, 1.2 eq.), 10 mL of water and 711.73 mg of K₂CO₃ (5.15 mmol, 5.0 eq.) were added sequentially. The reaction was continued with magnetic stirring at room temperature, and its progress was monitored by LC-MS. After 27 hours of stirring, the mixture was subjected to liquid separation. The organic phase was concentrated under reduced pressure, and the resulting concentrate was purified by silica gel column chromatography (eluent: EA: PE = 1:1) to afford intermediate 28-1 (750 mg, yellow solid) in 89% yield.

### Synthesis of Intermediate 28-2

To a 100 mL single-necked flask loaded with 750 mg of intermediate 28-1 (0.92 mmol), 10 mL of methanol and 75 mg of 10% Pd/C were added. The flask was fitted with a hydrogen balloon, and the system was purged with hydrogen gas for three cycles. The reaction was then carried out with magnetic stirring at room temperature, and its progress was monitored by LC-MS. After 23 hours of stirring, the reaction mixture was directly filtered under reduced pressure. The filtrate was concentrated under reduced pressure to yield intermediate 28-2 (380 mg, yellow solid) in 70% yield.

### Synthesis of Compound S28

To a 100 mL single-necked flask charged with 380 mg of intermediate 28-2 (0.75 mmol), 10 mL of methanol and 5 mL of water were added. Under magnetic stirring at room temperature, 120 mg of NaOH (3.0 mmol, 4.0 eq.) was added. The reaction was performed at room temperature for 2 hours, and its progress was monitored by LC-MS. After the reaction was completed, 180 mg of glacial acetic acid was added to quench the reaction. The reaction mixture was directly concentrated under reduced pressure, and the resulting concentrate was purified by silica gel column chromatography (eluent: methanol) to obtain compound S28 (290 mg, yellow solid) in 91% yield.

¹H NMR (400 MHz, Methanol-*d₄*) δ 7.62 (d, *J* = 9.0 Hz, 1H), 6.76 (d, *J* = 9.0 Hz, 1H), 6.04 (s, 1H), 4.61 (d, *J* = 7.7 Hz, 1H), 3.86 (dd, *J* = 12.0, 2.3 Hz, 1H), 3.77 (dd, *J* = 12.0, 4.7 Hz, 1H), 3.58-3.50 (m, 1H), 3.48-3.34 (m, 4H), 2.93 (td, *J* = 12.0, 3.7 Hz, 3H), 2.21-1.92 (m, 4H).

ES-MS m/z [M+1]⁺: 424.20.

### Example 29 Synthesis of Compound S29

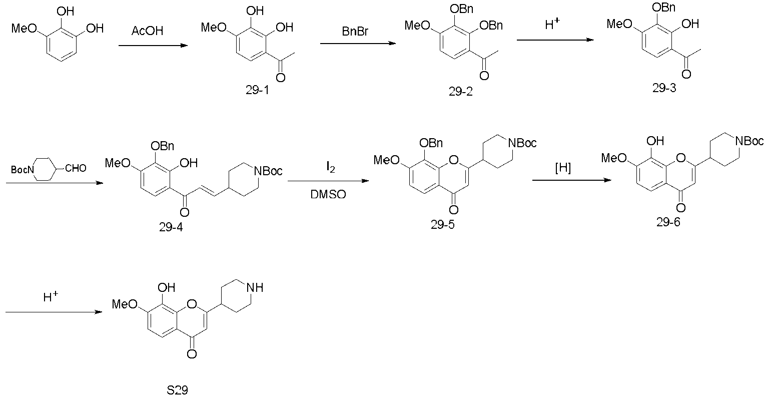

### Synthesis of Intermediate 29-1

To a 1 L two-necked flask charged with 20 g of 3-methoxybenzene-1,2-diol (142.71 mmol) was added 400 mL of glacial acetic acid. Under stirring at room temperature, 194.88 g of ZnCl₂ (1.43 mol, 10.0 eq.) was added, and the mixture was heated to reflux and stirred for 20 hours. The reaction process was monitored by LC-MS. Upon completion of the reaction, the reaction mixture was cooled to room temperature, and concentrated under reduced pressure to remove acetic acid. After 500 mL of water was added to the concentrate, the mixture was extracted with 500 mL of EA. After liquid separation, the organic phase was washed sequentially with 500 mL of water, 200 mL of saturated sodium bicarbonate solution and 200 mL of water. Liquid separation was conducted again, and the organic phase was concentrated under reduced pressure to obtain intermediate 29-1 (23.83 g, yellow solid) in 91% yield.

### Synthesis of Intermediate 29-2

To a 1 L two-necked flask charged with 23.83 g of intermediate 29-1 (130.80 mmol) was added 150 mL of NMP. Under stirring at room temperature, 54.23 g of K₂CO₃ (329.4 mmol, 3.0 eq.), 3.26 g of KI (19.62 mmol, 0.15 eq.), 36 mL of water and 45.27 g of BnBr (274.68 mmol, 2.1 eq.) were added in sequence. The mixture was stirred at 40 °C for 1.5 hours. The reaction process was monitored by LC-MS. Upon completion of the reaction, the reaction mixture was cooled to room temperature, and 600 mL of water was added to the mixture to quench the reaction. Then the mixture was extracted with 200 mL of EA, and after liquid separation, the aqueous phase was extracted once more with 200 mL of EA. The two organic phases were combined and washed once with 200 mL of water. After liquid separation, the organic phase was concentrated under reduced pressure to obtain intermediate 29-2 (42.25 g, yellow oil) in 95% yield.

### Synthesis of Intermediate 29-3

To a 500 mL two-necked flask charged with 45.5 g of intermediate 29-2 (124.85 mmol) was added 150 mL of glacial acetic acid and 15 mL of concentrated hydrochloric acid. The mixture was stirred at room temperature for 19 hours. The reaction process was monitored by LC-MS. Upon completion of the reaction, 400 mL of water was added to quench the reaction, and the mixture was then extracted with 200 mL of ethyl acetate. After liquid separation, the organic phase was washed once with 100 mL of saturated sodium bicarbonate solution. Liquid separation was carried out again, and the organic phase was concentrated under reduced pressure. The resulting concentrate was purified by silica gel column chromatography (eluent: EA: PE = 1:3) to obtain intermediate 29-3 (17.03 g, yellow oil) in 50% yield.

### Synthesis of Intermediate 29-4

To a 250 mL two-necked flask charged with 7.0 g (25.71 mmol) of intermediate 29-3 was added 60 mL of ethanol. Under stirring at room temperature, 8.22 g (38.56 mmol, 1.5 eq.) of 1-tert-butoxycarbonyl piperidine-4-carbaldehyde and 4.57 g (64.27 mmol, 2.5 eq.) of pyrrolidine were added. The mixture was then stirred under reflux for 18 hours. The reaction was monitored by LC-MS. When the reaction was completed, the reaction mixture was cooled to room temperature, and was concentrated directly. The residue was dissolved in 100 mL of ethyl acetate and washed twice with water (100 mL each time). The aqueous phase was discarded, and the organic phase was concentrated under reduced pressure. The concentrate was purified by silica gel column chromatography (eluent: EA:PE = 1:5) to afford intermediate 29-4 (10.45 g, yellow oil) in 87% yield.

### Synthesis of Intermediate 29-5

To a 100 mL two-necked flask charged with 3.0 g of intermediate 29-4 (6.42 mmol) was added 20 mL of DMSO. Under stirring at room temperature, 324.88 mg of iodine (1.28 mmol, 0.2 eq.) was added. The mixture was stirred at room temperature for 20 hours. The reaction process was monitored by LC-MS. Upon completion of the reaction, the reaction mixture was cooled to room temperature, and 1.66 g of DIEA and 2.10 g of Boc₂O were added. The mixture was stirred at room temperature for another 40 min. 1 g of solid sodium thiosulfate pentahydrate was dissolved in 80 mL of water to prepare a solution. 50 mL of ethyl acetate and the prepared sodium thiosulfate solution were added to quench the reaction. After liquid separation, the organic phase was concentrated under reduced pressure. The resulting concentrate was purified by silica gel column chromatography (eluent: EA:PE = 1:3) to obtain intermediate 29-5 (650 mg, yellow oil) in 22% yield.

### Synthesis of Intermediate 29-6

To a 50 mL two-necked flask charged with 650 mg of intermediate 29-5 (1.40 mmol) was added 65 mg of 10% Pd/C, and then the system was purged with hydrogen for three times. 10 mL of THF was added, and the mixture was stirred for 17 hours. The reaction process was monitored by LC-MS. Upon completion of the reaction, the Pd/C was removed by vacuum filtration. The filtrate was concentrated under reduced pressure to obtain intermediate 29-6 (530 mg, white foamy solid).

### Synthesis of Compound S29

To a 100 mL two-necked flask charged with 530 mg of intermediate 29-6 (1.41 mmol) was added 5 mL of THF and 13 mL of TFA. The mixture was stirred at room temperature for 24 hours. The reaction process was monitored by LC-MS. Upon completion of the reaction, the reaction mixture was directly concentrated under reduced pressure. 5 mL of water and 5 mL of THF were added to the concentrate, followed by the addition of 250 mg of NaOH. Then the mixture was stirred at room temperature for 18 hours, followed by the addition of 375 mg of glacial acetic acid to quench the reaction. The reaction mixture was concentrated under reduced pressure, and the resulting concentrate was purified by preparative liquid chromatography to obtain compound S29 (60.9 mg, white solid) in 47% yield.

¹H NMR (400 MHz, Methanol-*d₄*) δ 7.60 (d, *J* = 8.9 Hz, 1H), 7.17 (d, *J* = 8.9 Hz, 1H), 6.18 (s, 1H), 4.00 (s, 3H), 3.62-3.50 (m, 2H), 3.22-3.04 (m, 3H), 2.31 (d, *J* = 14.1 Hz, 2H), 2.14-1.97 (m, 2H).

ES-MS m/z [M+1]⁺: 276.10.

### Example 30 Synthesis of Compound S30

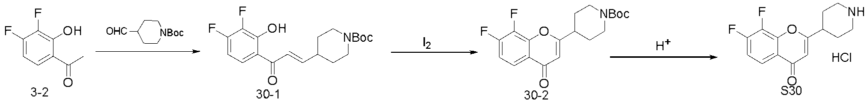

### Synthesis of Intermediate 30-1

To a 100 mL two-necked flask charged with 2 g of intermediate 3-2 (11.62 mmol) was added 20 mL of ethanol. Under stirring at room temperature, 1.24 g of pyrrolidine (17.43 mmol, 1.5 eq.) and 2.73 g of 1-tert-butoxycarbonyl piperidine-4-carbaldehyde (12.78 mmol, 1.1 eq.) were added. The mixture was stirred under reflux for 2 hours. The reaction process was monitored by LC-MS. Upon completion of the reaction, the mixture was cooled to room temperature and concentrated under reduced pressure. The crude product was purified by silica gel column chromatography (eluent: EA:PE = 1:3) to afford intermediate 30-1 (800 mg, yellow oil) in 19% yield.

### Synthesis of Intermediate 30-2

To a 50 mL two-necked flask charged with 400 mg of intermediate 30-1 (1.09 mmol) was added 20 mL of DMSO. Under stirring at room temperature, 553.30 mg of iodine (2.18 mmol, 2.0 eq.) was incorporated. The mixture was stirred at 110 °C for 2 hours. The reaction progress was monitored by LC-MS. After the reaction was completed, the mixture was cooled to room temperature. Subsequently, 330.89 mg of triethylamine (3.27 mmol, 3.0 eq.) and 475.79 mg of Boc₂O (2.18 mmol, 2.0 eq.) were added, and the mixture was stirred at room temperature for another 40 minutes. Upon the reaction termination, 40 mL of water was added to quench the reaction. The mixture was extracted with 20 mL of EA, and the aqueous phase was discarded after liquid separation. The organic phase was washed sequentially with 10 mL of saturated sodium thiosulfate solution and 50 mL of saturated brine. After liquid separation, the organic phase was concentrated under reduced pressure. The concentrate was purified by silica gel column chromatography (eluent: EA:PE = 1:3) to afford intermediate 30-2 (40 mg, yellow oil).

### Synthesis of Compound S30

To a 10 mL single-necked flask charged with 40 mg of intermediate 30-2 (0.11 mmol) was added 2 mL of 4 M hydrogen chloride in ethyl acetate, and the mixture was carried at room temperature for 5 min. The reaction was monitored by LC-MS. When the reaction finished, the reaction mixture was concentrated under reduced pressure. The concentrate was slurried with 5 mL of ethyl acetate, and the resulting mixture was subjected to suction filtration to obtain compound S30 (30 mg, yellow solid) in 91% yield.

¹H NMR (400 MHz, Methanol-*d₄*) δ 7.93 (ddd, *J =* 9.1, 5.5, 2.3 Hz, 1H), 7.42 (ddd, *J* = 9.9, 9.1, 6.7 Hz, 1H), 6.34 (s, 1H), 3.62-3.50 (m, 2H), 3.24-3.13 (m, 3H), 2.38-2.24 (m, 2H), 2.13-1.96 (m, 2H).

ES-MS m/z [M+1]⁺: 266.10.

### Example 31 Synthesis of Compound S31

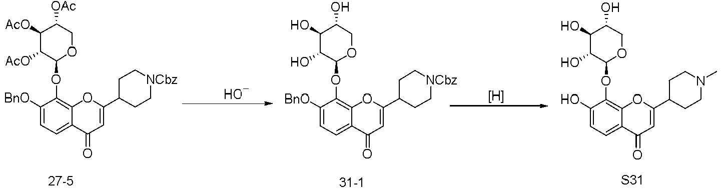

### Synthesis of Intermediate 31-1

To a 250 mL two-necked flask charged with 500 mg of intermediate 27-5 (0.67 mmol) was added 50 mL of methanol and 25 mL of water. Under magnetic stirring at 0°C, 107.2 mg of NaOH (2.68 mmol, 4.0 eq.) was added. The reaction was then allowed to proceed with stirring at room temperature for 1.5 hours. The reaction progress was monitored by LC-MS. When the reaction was completed, 160 mg of acetic acid was added to quench the reaction, followed by the addition of 150 mL of water. The mixture was filtered under suction, and the filter cake was dried in an oven at 50°C to afford intermediate 31-1 (330 mg, white solid) in 79% yield.

### Synthesis of Compound S31

To a 50 mL two-necked flask charged with 330 mg of intermediate 31-1 (0.53 mmol) was added 33 mg of 10% Pd/C, and the system was purged three times with hydrogen gas. Subsequently, 10 mL of THF and 10 mL of methanol were added, and the mixture was reacted with stirring at room temperature for 25 hours. The reaction progress was monitored by LC-MS. When the reaction was completed, the catalyst Pd/C was removed by filtration. The filtrate was concentrated under reduced pressure, and the concentrate was purified by Pre-TLC (developing solvent: methanol) to afforded compound S31 (10 mg, white solid).

¹H NMR (400 MHz, Methanol-*d₄*) δ 7.60 (d, *J* = 9.0 Hz, 1H), 6.74 (d, *J* = 9.0 Hz, 1H), 6.01 (s, 1H), 4.55 (d, *J =* 7.5 Hz, 1H), 3.97-3.89 (m, 1H), 3.56 (ddd, *J* = 10.1, 8.7, 5.3 Hz, 1H), 3.50 (dd, *J* = 9.0, 7.5 Hz, 1H), 3.37 (t, *J* = 8.9 Hz, 1H), 3.21 (dd, *J* = 11.4, 10.2 Hz, 1H), 3.06-2.95 (m, 2H), 2.70-2.57 (m, 1H), 2.32 (s, 3H), 2.24-2.10 (m, 3H), 2.08-2.00 (m, 1H), 1.94 (td, *J* = 12.3, 3.7 Hz, 1H), 1.89-1.80 (m, 1H).

ES-MS m/z [M+1]⁺: 408.10.

### Example 32 Synthesis of Compound S32

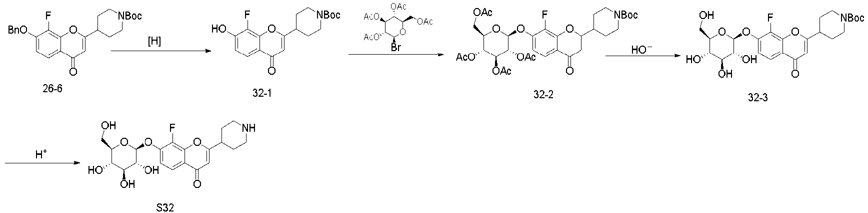

### Synthesis of Intermediate 32-1

To a 100 mL two-necked flask charged with 500 mg of intermediate 26-6(1.10 mmol) was added 500 mg of 10% Pd/C, and the system was purged three times with hydrogen gas. Subsequently, 5 mL of THF was added, and the mixture was reacted with stirring at room temperature for 4 hours. The reaction progress was monitored by LC-MS. When the reaction was completed, the Pd/C was removed by filtration under reduced pressure. The filtrate was concentrated under reduced pressure to afford intermediate 32-1 (460 mg, yellow oil).

### Synthesis of Intermediate 32-2

To a 100 mL two-necked flask charged with intermediate 32-1 (1.27 mmol) was added 20 mL of chloroform. Under stirring at room temperature, (2R,3R,4S,5R,6R)-2-(acetoxymethyl)-6-bromotetrahydro-2H-pyran-3,4,5-triyl triacetate (3.80 mmol, 3.0 eq.), TBAB (1.52 mmol, 1.2 eq.), 20 mL of water, and K₂CO₃ (6.33 mmol, 5.0 eq.) were added sequentially. The mixture was then reacted with stirring at room temperature for 16 hours. The reaction progress was monitored by LC-MS. When the reaction was completed, liquid separation was conducted, and the organic phase was concentrated under reduced pressure. The concentrate was purified by silica gel column chromatography (eluent: EA:PE = 1:1) to afford intermediate 32-2 (317 mg, yellow oil,) in 36% yield.

### Synthesis of Intermediate 32-3

To a 50 mL single-necked flask charged with Intermediate 32-2 (0.46 mmol) were added 2 mL of THF and 2 mL of water. Under stirring at room temperature, NaOH (1.84 mmol, 4.0 eq.) was added, and the mixture was reacted with stirring at room temperature for 2 hours. The reaction progress was monitored by LC-MS. When the reaction was completed, 77 mg of glacial acetic acid was added to quench the reaction. The reaction mixture was concentrated under reduced pressure, and the concentrate was purified by silica gel column chromatography to afford intermediate 32-3(181.2 mg, white solid) in 75% yield.

### Synthesis of Compound S32

To a 10 mL single-necked flask charged with intermediate 32-3 (0.35 mmol) was added 2 mL of a 4 M hydrogen chloride in ethyl acetate, and the mixture was reacted with stirring at room temperature for 10 min. The reaction progress was monitored by LC-MS. When the reaction was completed, the reaction mixture was concentrated under reduced pressure. The concentrate was slurried in 5 mL of ethyl acetate, followed by suction filtration to obtain the filter cake as compound S32 (77 mg, yellow solid) in 52% yield.

ES-MS m/z [M+1]⁺: 426.10.

### Example 33 Synthesis of Compound S33

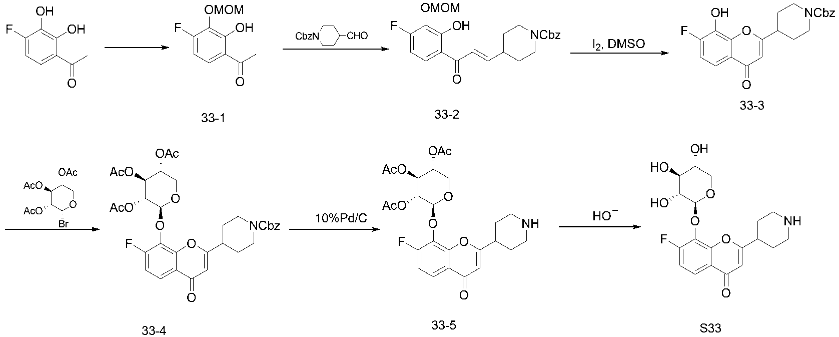

### Synthesis of Intermediate 33-1

To a 500 mL two-necked flask charged with 1-(4-fluoro-2,3-dihydroxyphenyl) ethen-1-one (40.00 mmol) was added 100 mL of dichloromethane. Under magnetic stirring at -10 °C, N,N-diisopropylethylamine (68.00 mmol, 1.7 eq.) was then added. The mixture was maintained below 0 °C while MOMBr (68.00 mmol, 1.7 eq.) was slowly added dropwise. After the completion of addition, the reaction was carried out with stirring at a temperature below 10 °C for 2 hours. The reaction process was monitored by LC-MS. Upon the completion of the reaction, 200 mL of water was added to quench the reaction. The mixture was subjected to liquid separation, and the obtained organic phase was washed with another 200 mL water, followed by a second liquid separation. The organic phase was concentrated under reduced pressure to afford intermediate 33-1 (7.28 g, yellow solid) in 85% yield.

### Synthesis of Intermediate 33-2

To a 50 mL two-necked flask charged with intermediate 33-1 (6.6 mmol) was added 20 mL of ethanol. Under magnetic stirring at room temperature, pyrrolidine (16.5 mmol, 2.5 eq.) and benzyl 4-formylpiperidine-1-carboxylate (9.9 mmol, 1.5 eq.) were added, and the reaction was carried out with stirring under reflux for 3 hours. The reaction process was monitored by LC-MS. After the reaction was completed, the mixture was cooled to room temperature. The reaction mixture was concentrated under reduced pressure, and the resulting concentrate was purified by silica gel column chromatography (eluent: EA: PE = 1:3) to afford intermediate 33-2 (2.28 g, yellow oil) in 78% yield.

### Synthesis of Intermediate 33-3

To a 100 mL two-necked flask charged with intermediate 33-2 (5.15 mmol) was added 40 mL of DMSO. Under magnetic stirring at room temperature, iodine (1.545 mmol, 0.3 eq.) was added, and the reaction was carried out with stirring at 110 °C for 1 hour. The reaction process was monitored by LC-MS. After the reaction was completed, the mixture was cooled to room temperature, and 120 mL of water was added to quench the reaction. The mixture was extracted with 60 mL of ethyl acetate, and the organic phase was separated. The obtained organic phase was washed with 20 mL of saturated sodium thiosulfate solution, followed by liquid separation. The organic phase was concentrated under reduced pressure, and the resulting concentrate was purified by silica gel column chromatography (eluent: EA: PE = 1:3) to afford intermediate 33-3 (1.45 g, yellow solid) in 71% yield.

### Synthesis of Intermediate 33-4

To a 100 mL two-necked flask charged with intermediate 33-3 (3.66 mmol) was added 20 mL of chloroform. Under magnetic stirring at room temperature, intermediate 22-7 (10.98 mmol, 3.0 eq.), TBAB (4.392 mmol, 1.2 eq.), 20 mL of water and K₂CO₃ (18.3 mmol, 5.0 eq.) were sequentially added, and the mixture was continuously stirred at room temperature for 18 hours. The reaction process was monitored by LC-MS. The reaction mixture was subjected to liquid separation, and the organic phase was concentrated under reduced pressure. The resulting concentrate was purified by silica gel column chromatography (eluent: EA: PE = 1:1) to afford intermediate 33-4 (1.92 g, yellow solid) in 80% yield.

### Synthesis of Compound S33

To a 50 mL two-necked flask charged with intermediate 33-4 (0.22 mmol) was added 16 mg of Pd/C, and the atmosphere inside the flask was replaced with hydrogen for three times. 15 mL of THF was then added, and the reaction was carried out with stirring at room temperature for 24 hours. The reaction process was monitored by LC-MS. Upon the completion of the reaction, the catalyst was removed by suction filtration to obtain a solution of intermediate 33-5. To the solution of intermediate 33-5 were added 10 mL of water and 10 mL of methanol, followed by the addition of NaOH (0.88 mmol, 4.0 eq.) under stirring at room temperature. The mixture was continuously stirred for another 6 hours. After the reaction was completed, 52.8 mg of acetic acid was added to quench the reaction. The quenched solution was concentrated under reduced pressure, and the resulting residue was purified by Pre-TLC (developing solvent: MeOH: DCM=10:1) to afford compound S33 (30 mg, white solid) in 35% yield.

ES-MS m/z [M+1]⁺: 396.10.

### Example 34 Synthesis of Compound S34

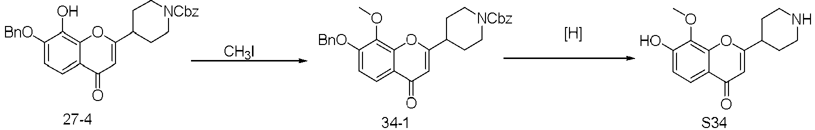

### Synthesis of Intermediate 34-1

To a 50 mL two-necked flask charged with 500 mg of intermediate 27-4 (1.03 mmol) was added 10 mL of DMF. Under magnetic stirring at room temperature, 711.73 mg of K₂CO₃ (5.15 mmol, 5.0 eq.) and iodomethane (10.3 mmol, 10.0 eq.) were successively added, and the mixture was continuously stirred at room temperature for another 6 hours. The reaction process was monitored by LC-MS. Upon the completion of the reaction, to the mixture was added 30 mL of ethyl acetate and 40 mL of water, followed by liquid separation. The organic phase was concentrated under reduced pressure to obtain a concentrate, which was purified by silica gel column chromatography (eluent: EA: PE = 1:1) to afford intermediate 34-1 (468 mg, yellow solid) in 91% yield.

### Synthesis of Compound S34

To a 100 mL single-necked flask charged with 468 mg of intermediate 34-1 (0.94 mmol) was added 10 mL of methanol and 75 mg of 10% Pd/C. A hydrogen balloon was attached to the flask, and the atmosphere inside the system was replaced with hydrogen for three times. Then the reaction was conducted with magnetic stirring at room temperature for 23 hours. The reaction process was monitored by LC-MS. After the reaction was completed, the reaction mixture was directly subjected to filtration under reduced pressure. The filtrate was concentrated under reduced pressure to obtain a concentrate, which was purified by silica gel column chromatography (eluent: EA: PE = 1:1) to afford compound S34 (168 mg, yellow solid) in 65% yield.

ES-MS m/z [M+1]⁺: 276.1.

### Example 35 Synthesis of Compound S35

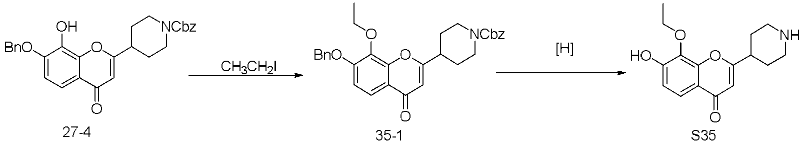

### Synthesis of Intermediate 35-1

To a 50 mL two-necked flask charged with 500 mg of intermediate 27-4 (1.03 mmol) was added 10 mL of DMF. Under magnetic stirring at room temperature, 711.73 mg of K₂CO₃ (5.15 mmol, 5.0 eq.) and iodoethane (10.3 mmol, 10.0 eq.) were successively added, and the mixture was continuously stirred at room temperature for 6 hours. The reaction process was monitored by LC-MS. Upon the completion of the reaction, 30 mL of ethyl acetate and 40 mL of water were added to the mixture, followed by liquid separation. The organic phase was concentrated under reduced pressure to obtain a concentrate, which was purified by silica gel column chromatography (eluent: EA: PE = 1:1) to afford intermediate 35-1 (449 mg, yellow solid) in 85% yield.

### Synthesis of Compound S35

To a 100 mL single-necked flask charged with 449 mg of intermediate 35-1 (0.88 mmol) was added 10 mL of methanol and 75 mg of 10% Pd/C. A hydrogen balloon was attached to the flask, and the atmosphere inside the system was replaced with hydrogen for three times. Then the reaction was conducted with magnetic stirring at room temperature for 23 hours. The reaction process was monitored by LC-MS. After the reaction was completed, the reaction mixture was directly subjected to filtration under reduced pressure. The filtrate was concentrated under reduced pressure to obtain a concentrate, which was purified by silica gel column chromatography (eluent: EA: PE = 1:1) to afford compound S35 (151 mg, yellow solid) in 60% yield.

ES-MS m/z [M+1]⁺: 290.1.

### Example 36 Synthesis of Compound S36

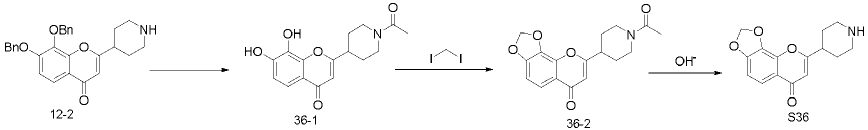

### Synthesis of Intermediate 36-1

To a 100 mL single-necked flask charged with intermediate 12-2 (5 mmol) was added 20 mL of THF, followed by the addition of Ac₂O (10 mmol, 2.0 eq.) and TEA (10 mmol, 2.0 eq.). The mixture was stirred at room temperature for 1 hour, with the reaction process monitored by LC-MS. Upon the completion of the reaction, 50 mL of EA and 30 mL of water were added, followed by the liquid separation. The organic phase was concentrated under reduced pressure, and the resulting concentrate was purified by silica gel column chromatography (eluent: EA: PE = 1:3) to afford intermediate 36-1 (1.29 g, yellow solid) in 85% yield.

### Synthesis of Intermediate 36-2

To a 100 mL single-necked flask charged with Intermediate 36-1 (4.25 mmol) was added 15 mL of DMF. Sodium bicarbonate (8.5 mmol, 2.0 eq.) and diiodomethane (8.5 mmol, 2.0 eq.) were successively added, and the mixture was stirred at 80 °C for 15 hours, with the reaction process monitored by LC-MS. Upon the completion of the reaction, 30 mL of EA and 30 mL of water were added, followed by liquid separation. The organic phase was concentrated under reduced pressure, and the resulting concentrate was purified by silica gel column chromatography (eluent: EA: PE = 1:5) to afford intermediate 36-2 (0.63 g, yellow solid) in 45% yield.

### Synthesis of Compound S36

To a 50 mL single-necked flask charged with intermediate 36-2 (1.91 mmol) was added 5 mL of methanol, followed by the addition of 3 mL of 4 M sodium hydroxide solution. The mixture was stirred at room temperature for 2 hours, with the reaction process monitored by LC-MS. Upon the completion of the reaction, 20 mL of EA and 20 mL of water were added, followed by liquid separation. The organic phase was concentrated under reduced pressure, and the resulting concentrate was purified by silica gel column chromatography (eluent: EA: PE = 1:3) to afford compound S36 (0.34 g, yellow solid) in 65% yield.

ES-MS m/z [M+1]⁺: 274.1.

### Example 37 Synthesis of Compound S37

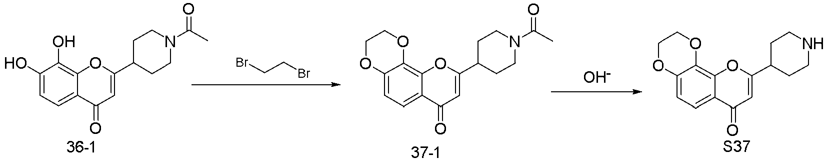

### Synthesis of Intermediate 37-1

To a 100 mL single-necked flask charged with intermediate 36-1 (4.25 mmol) was added 15 mL of DMF. Then potassium carbonate (8.5 mmol, 2.0 eq.) and 1,2-dibromoethane (8.5 mmol, 2.0 eq.) were successively added. The mixture was stirred at 80 °C for 3 hours, with the reaction process monitored by LC-MS. Upon the completion of the reaction, 30 mL of EA and 30 mL of water were added, followed by liquid separation. The organic phase was concentrated under reduced pressure, and the resulting concentrate was purified by silica gel column chromatography (eluent: EA: PE = 1:5) to afford intermediate 37-1 (1.23 g, yellow solid) in 88% yield.

### Synthesis of Compound S37

To a 50 mL single-necked flask charged with intermediate 37-1 (3.74 mmol) was added 10 mL of methanol, followed by the addition of 8 mL of 4 M sodium hydroxide solution. The mixture was stirred at room temperature for 2 hours, with the reaction process monitored by LC-MS. Upon the completion of the reaction, 30 mL of EA and 25 mL of water were added, followed by liquid separation. The organic phase was concentrated under reduced pressure, and the resulting concentrate was purified by silica gel column chromatography (eluent: EA: PE = 1:3) to afford compound S37 (0.79 g, yellow solid) in 74% yield.

ES-MS m/z [M+1]⁺: 288.1.

### Example 38 Synthesis of Compound S38

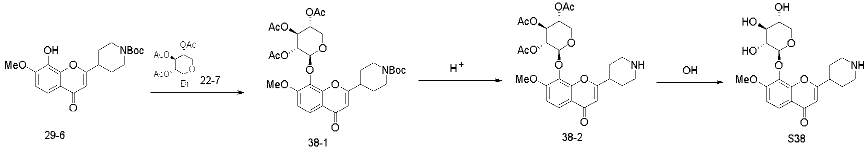

### Synthesis of Intermediate 38-1

To a 100 mL two-necked flask charged with intermediate 29-6 (1.27 mmol) was added 20 mL of chloroform. Under magnetic stirring at room temperature, (2R,3R,4S,5R,6R)-2-(acetoxymethyl)-6-bromotetrahydro-2H-pyran-3,4,5-triyl triacetate (3.80 mmol, 3.0 eq.), TBAB (1.52 mmol, 1.2 eq.), 20 mL of water and K₂CO₃ (6.33 mmol, 5.0 eq.) were successively added, and the reaction was allowed to proceed with stirring at room temperature for 16 hours. The reaction process was monitored by LC-MS. Upon the completion of the reaction, liquid separation was performed, and the organic phase was concentrated under reduced pressure. The resulting concentrate was purified by silica gel column chromatography (eluent: EA: PE = 1:1) to afford intermediate 38-1 (0.69 g, yellow oil) in 86% yield.

### Synthesis of Intermediate 38-2

To a 50 mL single-necked flask charged with intermediate 38-1 (1.09 mmol) was added 10 mL of 4 M hydrogen chloride in ethyl acetate, and the reaction was allowed to proceed with stirring at room temperature for 10 minutes. The reaction process was monitored by LC-MS. Upon the completion of the reaction, the reaction mixture was concentrated under reduced pressure. The resulting concentrate was slurried with 10 mL of ethyl acetate, followed by suction filtration to afford intermediate 38-2 (436 mg, yellow solid) in 75% yield.

### Synthesis of Compound S38

To a 50 mL single-necked flask charged with intermediate 38-2 (0.82 mmol) was added 5 mL of THF and 5 mL of water. Under magnetic stirring at room temperature, NaOH (3.28 mmol, 4.0 eq.) was added, and the reaction was allowed to proceed with stirring at room temperature for 2 hours. The reaction process was monitored by LC-MS. Upon the completion of the reaction, glacial acetic acid was added to quench the reaction. The reaction mixture was concentrated under reduced pressure, and the resulting residue was purified by silica gel column chromatography to afford compound S38 (150 mg, white solid) in 45% yield.

ES-MS m/z [M+1]⁺: 408.10.

### Example 39 Synthesis of Compound S39

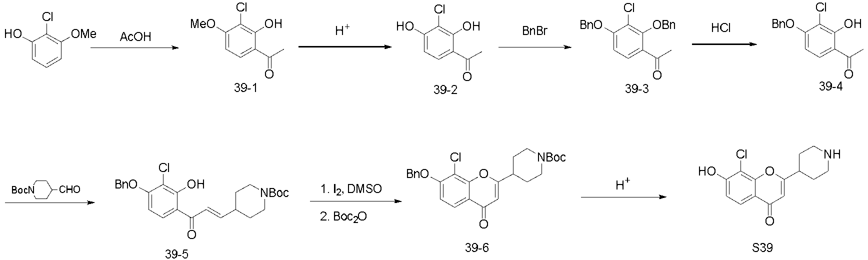

### Synthesis of Intermediate 39-1

To a 1 L two-necked flask charged with 2-chloro-3-methoxyphenol (141 mmol) was added 400 mL of glacial acetic acid. Under magnetic stirring at room temperature, ZnCl₂ (1.41 mol, 10 eq.) was slowly added, and the reaction was carried out under reflux with stirring for 23 hours. The reaction process was monitored by LC-MS. Upon the completion of the reaction, the mixture was cooled to room temperature and then concentrated under reduced pressure to remove acetic acid. 400 mL of water was added to the resulting concentrate to quench the reaction. The mixture was extracted with 400 mL of ethyl acetate, and the separated organic phase was washed twice with saturated sodium bicarbonate solution. The organic phase was concentrated under reduced pressure to afford intermediate 39-1 (22.56 g, reddish-brown solid) in 80% yield.

### Synthesis of Intermediate 39-2

To a 500 mL two-necked flask charged with intermediate 39-1 (112.8 mmol) was added 200 mL of 1,2-dichloroethane. Under magnetic stirring at 0 °C, AlCl₃ (676.8 mmol, 6.0 eq.) was slowly added, followed by the addition of ethane-1,2-dithiol (564 mmol, 5.0 eq.). The reaction was then carried out under reflux with stirring for 1 hour. The reaction process was monitored by LC-MS. Upon the completion of the reaction, the mixture was cooled to -10 °C, and 100 mL of concentrated hydrochloric acid was slowly added dropwise. After the completion of the dropwise addition, suction filtration was performed. The filtrate was subjected to liquid separation, and the aqueous phase was extracted once with 200 mL of dichloromethane. The two portions of organic phase were combined. The filter cake was slurried with 300 mL of methanol for 1 hour and then subjected to suction filtration to obtain a filtrate. The combined organic phase and filtrate were concentrated under reduced pressure, and the resulting concentrate was purified by silica gel column chromatography (eluent: EA: PE = 1:5) to afford intermediate 39-2 (13.64 g, yellow solid) in 65% yield.

### Synthesis of Intermediate 39-3

To a 500 mL two-necked flask charged with intermediate 39-2 (73.3 mmol) was added 60 mL of NMP. Under magnetic stirring at room temperature, K₂CO₃ (2.5 eq.), KI (0.15 eq.), 15 mL of water and benzyl bromide (2.5 eq.) were successively added, and the reaction was carried out with stirring at 40 °C for 1 hour. The reaction process was monitored by LC-MS. Upon the completion of the reaction, the reaction was quenched by adding 300 mL of water. The mixture was then extracted twice with 150 mL of ethyl acetate each time. The two portions of organic phase were combined and washed twice with water. The organic phase was concentrated under reduced pressure, and the resulting concentrate was purified by silica gel column chromatography (eluent: EA: PE = 1:5) to afford intermediate 39-3 (20.13 g, yellow solid) in 75% yield.

### Synthesis of Intermediate 39-4

To a 250 mL two-necked flask charged with intermediate 39-3 (54.98 mmol) was added 200 mL of glacial acetic acid. Under magnetic stirring at room temperature, 20 mL of concentrated hydrochloric acid was added, and the reaction was carried out with stirring at room temperature for 22 hours. The reaction process was monitored by LC-MS. Upon the completion of the reaction, the reaction solution was directly subjected to suction filtration. The filter cake was dried in an oven at 80 °C to afford intermediate 39-4 (7.74 g, white solid) in 51% yield.

### Synthesis of Intermediate 39-5

To a 100 mL two-necked flask charged with intermediate 39-4 (11.1 mmol) was added 30 mL of ethanol. Under magnetic stirring at room temperature, pyrrolidine (2.5 eq.) and 1-tert-butoxycarbonyl piperidine-4-carbaldehyde (1.5 eq.) were added, and the reaction was carried out under reflux. The reaction process was monitored by LC-MS. After stirring for 20 hours, heating was stopped. When the reaction mixture was cooled to room temperature, it was directly concentrated under reduced pressure. The resulting concentrate was purified by silica gel column chromatography (eluent: EA: PE = 5:1) to afford intermediate 39-5 (4.76 g, yellow oil) in 91% yield.

### Synthesis of Intermediate 39-6

To a 100 mL single-necked flask charged with intermediate 39-5 (5.49 mmol) was added 25 mL of DMSO and iodine (10.98 mmol, 2.0 eq.), and the reaction was carried out at 110 °C. The reaction process was monitored by LC-MS. After stirring for 1 hour, heating was stopped. When the reaction mixture was cooled to room temperature, Boc₂O (10.98 mmol, 2.0 eq.) and TEA (10.98 mmol, 2.0 eq.) were added, and the reaction was further maintained with stirring at room temperature for 17 hours. Upon the completion of the reaction, 50 mL of saturated sodium thiosulfate solution and 50 mL of EA were added. After liquid separation, the organic phase was concentrated under reduced pressure. The resulting concentrate was purified by silica gel column chromatography (eluent: EA: PE = 1:3) to afford intermediate 39-6 (0.67 g, yellow solid) in 26% yield.

### Synthesis of Compound S39

To a 50 mL single-necked flask charged with intermediate 39-6 (0.11 mmol) was added 2 mL of TFA, and the reaction was carried out at 80 °C. The reaction process was monitored by LC-MS. After stirring for 2 hours, heating was stopped. When the reaction mixture was cooled to room temperature, it was directly concentrated under reduced pressure. The pH of the resulting concentrate was adjusted to 7-8 with saturated sodium bicarbonate solution, followed by concentration under reduced pressure. The resulting residue was purified by Pre-TLC (developing solvent: methanol) to afford compound S39 (22 mg, yellow solid) in 72% yield.

ES-MS m/z [M+1]⁺: 280.1.

### Example 40 Synthesis of Compound S40

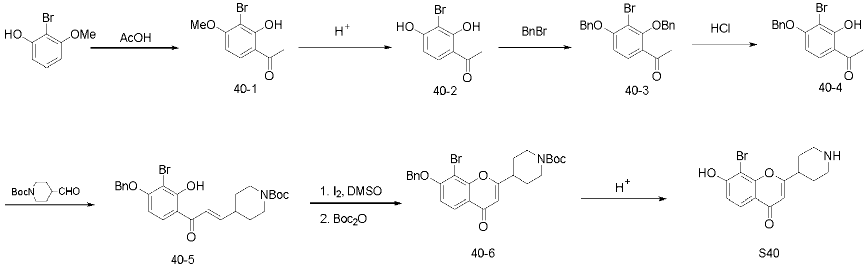

### Synthesis of Intermediate 40-1

To a 1 L two-necked flask charged with 2-bromo-3-methoxyphenol (141 mmol) was added 400 mL of glacial acetic acid. Under magnetic stirring at room temperature, ZnCl₂ (1.41 mol, 10 eq.) was slowly added, and the reaction was conducted with stirring under reflux for 23 hours. The reaction process was monitored by LC-MS. Upon the completion of the reaction, the reaction mixture was cooled to room temperature and then concentrated under reduced pressure to remove acetic acid. 400 mL of water was added to the resulting concentrate to quench the reaction, followed by extraction with 400 mL of ethyl acetate. The separated organic phase was washed twice with saturated sodium bicarbonate solution, and then was concentrated under reduced pressure to afford intermediate 40-1 (29.24 g, reddish-brown solid) in 85% yield.

### Synthesis of Intermediate 40-2

To a 500 mL two-necked flask charged with intermediate 40-1 (119.85 mmol) was added 200 mL of 1,2-dichloroethane. Under magnetic stirring at 0 °C, AlCl₃ (6.0 eq.) was slowly added, followed by the addition of ethane-1,2-dithiol (5.0 eq.). The reaction was then carried out with stirring under reflux for 1 hour. The reaction process was monitored by LC-MS. Upon the completion of the reaction, the reaction mixture was cooled to -10 °C, and 100 mL of concentrated hydrochloric acid was slowly added dropwise. After the completion of the dropwise addition, suction filtration was performed. The filtrate was subjected to liquid separation, and the aqueous phase was extracted once with 200 mL of dichloromethane. The two portions of organic phase were combined. The filter cake was slurried with 300 mL of methanol for 1 hour and then subjected to suction filtration to obtain a filtrate. The combined organic phase and filtrate were concentrated under reduced pressure, and the resulting concentrate was purified by silica gel column chromatography (eluent: EA: PE = 1:5) to afford intermediate 40-2 (19.56 g, yellow solid) in 71% yield.

### Synthesis of Intermediate 40-3

To a 500 mL two-necked flask charged with intermediate 40-2 (85.09 mmol) was added 60 mL of NMP. Under magnetic stirring at room temperature, K₂CO₃ (2.5 eq.), KI (0.15 eq.), 15 mL of water and benzyl bromide (2.5 eq.) were successively added, and the reaction was carried out with stirring at 40 °C for 1 hour. The reaction process was monitored by LC-MS. Upon the completion of the reaction, the reaction was quenched by adding 300 mL of water. The mixture was then extracted twice with 150 mL of EA each time. The two portions of organic phase were combined and washed twice with water. The organic phase was concentrated under reduced pressure, and the resulting concentrate was purified by silica gel column chromatography (eluent: EA: PE = 1:5) to afford intermediate 40-3 (24.77 g, yellow solid) in 71% yield.

### Synthesis of Intermediate 40-4

To a 250 mL two-necked flask charged with intermediate 40-3 (60.41 mmol) was added 200 mL of glacial acetic acid. Under magnetic stirring at room temperature, 20 mL of concentrated hydrochloric acid was added, and the reaction was carried out with stirring at room temperature for 22 hours. The reaction process was monitored by LC-MS. Upon the completion of the reaction, the reaction mixture was directly subjected to filtration under reduced pressure. The filter cake was dried in an oven at 80 °C to afford intermediate 40-4 (10.63 g, white solid) in 55% yield.

### Synthesis of Intermediate 40-5

To a 100 mL two-necked flask charged with intermediate 40-4 (11.1 mmol) was added 30 mL of ethanol. Under magnetic stirring at room temperature, pyrrolidine (2.5 eq.) and 1-tert-butoxycarbonyl piperidine-4-carbaldehyde (1.5 eq.) were added, and the reaction was conducted under reflux. The reaction process was monitored by LC-MS. After stirring for 20 hours, heating was stopped. When the reaction mixture was cooled to room temperature, it was directly concentrated under reduced pressure. The resulting concentrate was purified by silica gel column chromatography (eluent: EA: PE = 5:1) to afford intermediate 40-5 (5.43 g, yellow oil) in 95% yield.

### Synthesis of Intermediate 40-6

To a 100 mL single-necked flask charged with intermediate 40-5 (5.49 mmol) was added 25 mL of DMSO and iodine (10.98 mmol, 2.0 eq.), and the reaction was carried out at 110 °C. The reaction process was monitored by LC-MS. After stirring for 1 hour, heating was stopped. When the reaction mixture was cooled to room temperature, Boc₂O (10.98 mmol, 2.0 eq.) and TEA (10.98 mmol, 2.0 eq.) were added, and the reaction was further maintained with stirring at room temperature for 17 hours. Upon the completion of the reaction, 50 mL of saturated sodium thiosulfate solution and 50 mL of EA were added. After liquid separation, the organic phase was concentrated under reduced pressure. The resulting concentrate was purified by silica gel column chromatography (eluent: EA: PE = 1:3) to afford intermediate 40-6 (0.82 g, yellow solid) in 29% yield.

### Synthesis of Compound S40

To a 50 mL single-necked flask charged with intermediate 40-6 (0.11 mmol) was added 2 mL of TFA, and the reaction was carried out at 80 °C. The reaction process was monitored by LC-MS. After stirring for 2 hours, heating was stopped. When the reaction mixture was cooled to room temperature, it was directly concentrated under reduced pressure. The pH of the resulting concentrate was adjusted to 7-8 with saturated sodium bicarbonate solution, followed by concentration under reduced pressure. The resulting residue was purified by Pre-TLC (developing solvent: methanol) to afford compound S40 (25 mg, yellow solid) in 69% yield.

ES-MS m/z [M+1]⁺: 324.1.

### Example 41 Synthesis of Compound S41

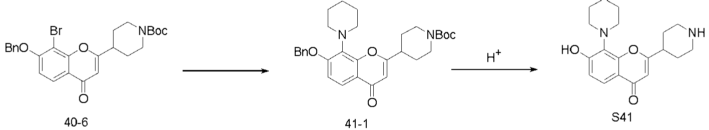

### Synthesis of Intermediate 41-1

To a 50 mL single-necked flask charged with intermediate 40-6 (2.80 mmol) and piperidine (4.20 mmol, 1.5 eq.) was added 15 mL of dioxane, and the atmosphere inside the flask was replaced with nitrogen for three times. PdCl₂(dppf) (0.28 mmol, 0.1 eq.) and cesium carbonate (5.6 mmol, 2.0 eq.) were then added. The mixture was heated to 90 °C and reacted for 20 hours. After the mixture was cooled to room temperature, filtration was performed, and the filtrate was concentrated under reduced pressure. The resulting concentrate was purified by silica gel column chromatography (eluent: EA: PE = 1:1) to afford Intermediate 41-1 (1.15 g, yellow solid) in 79% yield.

### Synthesis of Compound S41

To a 50 mL single-necked flask charged with intermediate 41-1 (0.11 mmol) was added 2 mL of TFA, and the reaction was carried out at 80 °C. The reaction process was monitored by LC-MS. After stirring for 2 hours, heating was stopped. When the reaction mixture was cooled to room temperature, it was directly concentrated under reduced pressure. The pH of the resulting concentrate was adjusted to 7-8 with saturated sodium bicarbonate solution, followed by concentration under reduced pressure. The resulting residue was purified by Pre-TLC (developing solvent: methanol) to afford compound S41 (24 mg, yellow solid) in 66% yield.

ES-MS m/z [M+1]⁺: 329.2.

### Example 42 Synthesis of Compound S42

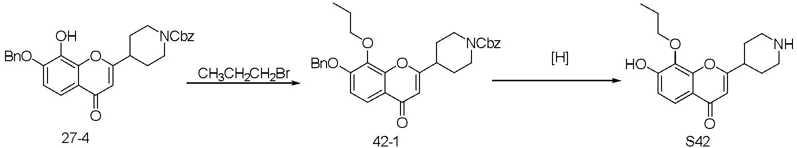

### Synthesis of Intermediate 42-1

To a 50 mL two-necked flask charged with 500 mg of Intermediate 27-4 (1.03 mmol) was added 10 mL of DMF. Under magnetic stirring at room temperature, 711.73 mg of K₂CO₃ (5.15 mmol, 5.0 eq.) and bromopropane (10.3 mmol, 10.0 eq.) were successively added, and the reaction was carried out with stirring at 80 °C for 6 hours, with the reaction process monitored by LC-MS. 30 mL of ethyl acetate and 40 mL of water were then added, followed by liquid separation. The organic phase was concentrated under reduced pressure to obtain a concentrate, which was purified by silica gel column chromatography (eluent: EA: PE = 1:1) to afford intermediate 42-1 (467 mg, yellow solid) in 86% yield.

### Synthesis of Compound S42

To a 100 mL single-necked flask charged with 467 mg of intermediate 42-1 (0.88 mmol) was added 10 mL of methanol and 75 mg of 10% Pd/C. A hydrogen balloon was attached to the flask, and the atmosphere inside the system was replaced with hydrogen for three times, followed by magnetic stirring of the reaction at room temperature for 23 hours. The reaction process was monitored by LC-MS. After the reaction was completed, the reaction mixture was directly subjected to filtration under reduced pressure. The filtrate was concentrated under reduced pressure to obtain a concentrate, which was purified by silica gel column chromatography (eluent: EA: PE = 1:1) to afford compound S42 (155 mg, yellow solid) in 58% yield.

ES-MS m/z [M+1]⁺: 304.1.

### Effect Example 1: Enhancement of Cell Survival Rates of PC-12 Neurocytes by Various Compound Under Glutamate Stimulation

### 1.1. Culture of PC-12 Cell Line and Modeling of Excitotoxic Cell Death

PC-12 cells (a neuroblastoma cell line derived from rat adrenal medulla) were cultured in DMEM/F12 complete medium until 80-90% confluency. After being digested, collected, and resuspended, the cells were seeded into 24-well plates pre-coated with coverslips at a density of 5×10⁴ cells per well. Following 26 hours of cell culture, glutamate-induced excitotoxic cell death modeling was performed. The modeling method was as follows: an appropriate amount of glutamate was weighed and dissolved in DMEM/F12 medium to prepare a stock solution; after filtration, the stock solution was diluted with the same medium, and the cells were administered with the diluted solution by medium replacement. After administration, the cells were further incubated for 24 hours.

### 1.2. Application of Small Molecules

Prior to glutamate modeling, cells incubated in the previous step were pretreated with small-molecule compounds listed in Table 1 at concentrations of 1 pM, 10 pM, 100 pM, 1 nM, 10 nM, 100 nM, 1 µM, 10 µM, and 100 µM for 30 minutes, followed by glutamate stimulation.

### 1.3 Propidium Iodide Staining

The cells treated with the compounds were stained with propidium iodide to assess cell death. After the medium was discarded, the cells were washed with PBS. Subsequently, propidium iodide/Hoechst was added. Following incubation for 30 minutes, the staining solution was removed, and the cells were washed with PBS again before being fixed and mounted on slides. Image acquisition was performed under a fluorescence microscope. The propidium iodide-positive cells and Hoechst-positive cells in the collected images were counted to calculate the cell survival rate. The survival rate was calculated using the formula: Cell survival rate = (1 - Number of Propidium Iodide-positive cells / Number of Hoechst-positive cells) × 100%. The gradient concentrations of the small molecule compounds and the corresponding normalized survival rates were imported into Prism software, and EC₅₀ values were calculated using a non-linear regression model. 7,8-dihydroxyflavone was used as the positive control drug. The specific data are shown in Table 1.

**Table 1: Dose-response relationship study of various compounds in promoting PC-12 neurocytes survival**

| **Compound** | **EC₅₀ (nM)** |
|---|---|
| 7, 8-dihydroxyflavone | 0.1590 |
| S1 | 0.01199 |
| S3 | 0.029 |
| S5 | 0.02820 |
| S6 | 0.01040 |
| S7 | 0.01110 |
| S11 | <0.001 |
| S12 | <0.001 |
| S20 | 0.0370 |

The results demonstrated that compounds S1, S3, S5, S6, S7, S11, S12, and S20 can effectively inhibit glutamate-induced excitotoxic death of PC-12 cells. Compared with the EC₅₀ values of the positive control drug 7,8-dihydroxyflavone, the EC₅₀ values of the compounds were significantly increased, exhibiting obvious activity advantages. Overall, the results showed that fluoro substitution of the hydroxyl groups or the benzene ring of 7,8-dihydroxyflavone could enhance the biological activity of the molecule, indicating that fluorine is beneficial to the activity of the compounds and further confirming that compounds such as S2 and S4 possess biological activity. Meanwhile, replacing the benzene ring with a saturated cyclohexane or the heterocyclic piperidine could both exert excellent effects in promoting neurocytes survival, demonstrating the replaceability of the benzene ring. Compounds such as S13, S14, and S15 are bioisosteres of the benzene ring and are expected to exhibit similar superior activity.

Based on the aforementioned dose-response relationship study of the compounds, the inhibitory effects of different small molecule compounds on glutamate-induced excitotoxic death of PC-12 cells were further compared at the same dose (100 nM). The specific data are shown in Table 2.

**Table 2: Study on the promoting effects of various compounds on PC-12 neurocytes survival**

| **Compound** | **Enhancement of Cell Survival Rate** |
|---|---|
| 7, 8-dihydroxyflavone | 33.58% |
| S31 | 93.32% |
| S30 | 83.78% |
| S29 | 73.51% |
| S21 | 46.17% |
| S22 | 44.34% |
| S28 | 43.06% |
| S24 | 41.01% |

The results demonstrated that each compound could effectively inhibit glutamate-induced death of PC-12 neurocytes and enhance cell survival capacity. Compared with the positive control drug 7,8-dihydroxyflavone, compounds S31, S30, S29, S21, S22, S28, and S24 exhibited a further significant enhancement in cell survival-promoting capacity, further validating the structure-activity relationship that structural modifications targeting the hydroxyl groups and benzene ring can improve cell survival.

The compounds of the present disclosure are obtained by modifying the positive control drug 7,8-dihydroxyflavone, particularly through targeted modifications at positions such as the hydroxyl groups and the benzene ring, resulting in compounds with higher biological activity and/or superior pharmacokinetic properties.

### Effect Example 2: Improvement of Pharmacokinetic Properties of Various Compounds in Rats

### 2.1 Animals, Reagents, and Instruments

| **Animals/Reagents/**Instruments | **Model/ Lot Number** | **Source** |
|---|---|---|
| **PEG-400** | BCC62966 | Sigma |
| **Tween 80** | 22129890 | Biosharp |
| **Rat** | SD/SPF grade/250-300 g/male | Vitonlihua |
| **Electronic Balance** | BCE224I-1CCN | Sartorius Scientific Instruments (Beijing) Co., Ltd. |
| **Triple Quadrupole Tandem Mass Spectrometer** | Sciex 5500 | Shimadzu Corporation, Japan |

### 2.2 Experimental Procedure

Preparation of intravenous bolus administration formulations: A graduation line for the final volume was marked on a clean and dry container. An appropriate amount of the test sample was weighed and added to the container to prepare a solution with a final concentration of 1 mg/mL.

Preparation of oral gavage administration formulations: A graduation line for the final volume was marked on a clean and dry container. An appropriate amount of the test sample was weighed and added to the container to prepare a solution with a final concentration of 3 mg/mL.

After preparation, the formulations were stored at room temperature and used on the day of preparation. A total of 2 groups of rats were set up, with 3 rats per group (half male and half female). The compounds were administered to the rats at a dose of 1 mg/kg via intravenous injection and 15 mg/kg via oral gavage, respectively. All animals were allowed free access to food and water.

Intravenous administration: Before administration (0 h) and at 0.083, 0.167, 0.5, 1.0, 2.0, 4.0, 6.0, and 24 h after administration, 0.2 mL of blood samples were collected from the jugular vein and transferred to anticoagulant tubes (pre-cooled in advance for at least 5 minutes). The tubes were gently inverted several times to fully mix the blood with the anticoagulant. The blood samples were centrifuged at 4 °C for 10 minutes to separate the plasma. 95 µ L of plasma was collected and transferred to a centrifuge tube, which was stored in a refrigerator at below -80 °C until analysis.

Single oral gavage administration: Before administration (0 h) and at 0.167, 0.5, 1.0, 2.0, 4.0, 6.0, and 24 h after administration, 0.2 mL of blood samples were collected from the jugular vein and transferred to anticoagulant tubes. The sample processing method was the same as described above.

The concentration of the compounds in plasma was determined by LC-MS/MS, and data acquisition was performed using Analyst 1.6.3 software (AB Sciex). The pharmacokinetic parameters of the rats after administration were calculated using the non-compartmental model in Phoenix WinNonlin software. The specific data are shown in Table 3.

7,8-dihydroxyflavone served as the control drug for comparing the pharmacokinetic characteristics of various compound. The specific data are presented in Table 3.

**Table 3: PK parameters of various compound**

| **Compound** | **t_{1/2} (h)** | **Cₘₐₓ(nM)** | **AUC₀₋ₜ(h·nM)** | **Oral Bioavailability F (%)** |
|---|---|---|---|---|
| 7,8-dihydroxyflavone | 3.32 | 75. 1 | 100 | 1.9 |
| S30 | **11.4** | **1264.2** | **10309.4** | **112** |
| S7 | / | **245** | **2364** | **66.9** |
| S25 | **5.38** | **3586.2** | **28, 186.2** | **52.0** |
| S29 | 2.57 | **91.3** | **331.3** | **3.38** |
| S28 | **16.7** | **174.5** | **820.3** | **2.72** |
| S12 | 1.84 | **113** | **151** | **1.9** |
| S1 | / | **94.9** | **180.9** | Not Assayed (NA) |
| S27 | 1.77 | **126.0** | **282.4** | Not Assayed (NA) |
| S26 | **4.54** | 68.1 | **252.1** | Not Assayed (NA) |

The results demonstrated that at the same oral dose, the oral bioavailability of compounds S30, S7, and S25 was significantly improved compared with the control drug 7,8-dihydroxyflavone. Correspondingly, the in vivo exposure was substantially increased, including Cₘₐₓ and AUC. The in vivo half-life was also significantly prolonged compared with that of the control drug 7,8-dihydroxyflavone. In summary, PK parameters were remarkably improved.

At the same oral dose, the oral bioavailability of compounds S29, S28, and S12 was either improved or remained substantially comparable to that of the control drug 7,8-dihydroxyflavone, with a significant increase in in vivo exposure, including Cₘₐₓ and AUC. For compounds S1, S27, and S26, the in vivo exposure was significantly increased compared with the control drug 7,8-dihydroxyflavone, including Cₘₐₓ and AUC. Additionally, the in vivo half-lives of compounds S28 and S26 were significantly prolonged.

Overall, fluoro substitution modification at the hydroxyl groups or benzene ring of 7,8-dihydroxyflavone, as well as replacement of the benzene ring with a cyclohexane or piperidine ring, all significantly improved the oral bioavailability, and/or in vivo half-life, and/or in vivo exposure of the compounds.

The compounds of the present application, while enhancing biological activity, further improve PK parameters, increase oral bioavailability, and/or prolong in vivo half-life, and/or increase in vivo exposure (plasma Cₘₐₓ and AUC). Thus, the therapeutic window of the drug and patient compliance are improved by means such as reducing the oral dose and decreasing the frequency of administration.

### Example 3: Calculation of physicochemical properties

The ClogP value of a drug refers to the logarithm of the octanol-water partition coefficient of the drug, an important parameter for assessing drug lipophilicity. The ClogP value reflects distribution tendency of a molecule between the oil phase (usually octanol, serving as the representative of the oil phase) and the aqueous phase. The ClogP values of the compounds were calculated by means of ChemBioDraw Version 14.0. A higher ClogP value indicates stronger liposolubility of the compound, with specific data shown in Table 4.

**Table 4: ClogP values of compounds**

| **Compound** | **ClogP** | **Compound** | **ClogP** | **Compound** | **ClogP** |
|---|---|---|---|---|---|
| **S12** | 0.83 | **S36** | 1.68 | **S40** | 2.00 |
| **S34** | 1.10 | **S37** | 1.64 | **S41** | 2.22 |
| **S35** | 1.63 | **S39** | 1.80 | **S42** | 2.16 |

The ClogP values of compounds S34, S35, S36, S37, S39, S40 and S41 are all higher than that of compound S12, which increases the liposolubility of the compounds, enhances their membrane permeability, facilitates their penetration across the blood-brain barrier, and thus endows the compounds with potential advantages for the treatment of diseases targeting intracerebral sites.

In summary, the compounds disclosed herein exhibit significant neuroprotective activity, possess potential neuro-regenerative effects, and/or offer pharmacokinetic advantages.

Although embodiments of the present disclosure have been illustrated and described above, it should be understood that the above embodiments are exemplary and should not be construed as limiting the scope of the disclosure. Variations, modifications, substitutions and alterations to the aforesaid embodiments may be made by those skilled in the art without departing from the scope of the present invention.

## Claims

1. A compound, wherein it is a compound as shown in formula (I), or a pharmaceutically acceptable stereoisomer, geometric isomer, tautomer, nitrogen oxide, hydrate, solvate, cocrystal, metabolite, salt, deuterated form, or prodrug thereof: 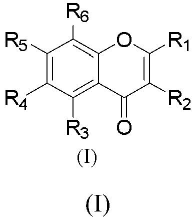 wherein,
R₁ is C₁₋₆ alkyl, C₂₋₆ alkenyl, C₂₋₆ alkynyl, aryl, C₃₋₆ cycloalkyl, or C₂₋₁₂ heterocyclyl, wherein the C₁₋₆ alkyl, C₂₋₆ alkenyl, C₂₋₆ alkynyl, aryl, C₃₋₆ cycloalkyl, and C₃₋₆ heterocyclyl are optionally substituted by one or more R₇;
R₂, R₃, R₄, R₅, R₆, and R₇ are each independently hydrogen, hydroxyl, halogen, C₁₋₆ alkyl, C₁₋₆ alkoxy, C₃₋₆ cycloalkoxy, C₁₋₆ heterocyclyl, glycosyl, 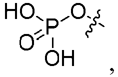 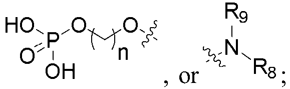 or R₄ and R₅ together with carbon atoms to which they are respectively attached form a 3-6-membered ring; or R₅ and R₆ together with carbon atoms to which they are respectively attached form a 3-6-membered ring, wherein the C₁₋₆ alkyl, C₁₋₆ alkoxy, C₃₋₆ cycloalkoxy, C₁₋₆ heterocyclyl, glycosyl, 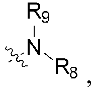 and 3-6 membered ring are optionally substituted by one or more substituents selected from hydrogen, hydroxyl, halogen, C₁₋₆ alkyl, C₁₋₆ alkoxy, C₃₋₆ cycloalkoxy, C₁₋₆ heterocyclyl, or 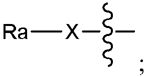
R₈ and R₉ are each independently hydrogen, C₁₋₆ alkyl, C₂₋₆ alkenyl, C₂₋₆ alkynyl, C₃₋₆ cycloalkyl, C₂₋₆ heterocyclyl, halo C₁₋₆ alkyl, aryl, or C₂₋₅ heteroaryl, or 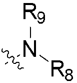 forms a 5-10 membered ring;
X is hydrogen, CH₂, S, NH, or O;
Rₐ is hydrogen, C₁₋₃ alkyl, C₂₋₄ alkenyl, halo C₁₋₃ alkyl, aryl, or C₃₋₆ heteroaryl;
n is an integer from 0 to 10;
preferably, when R₁ is aryl, the compound satisfies at least one of the following conditions:
(1) at least one of R₄, R₅, R₆, and R₇ is halogen or hydroxyl;
(2) at least one of R₅ and R₆ is glycosyl, 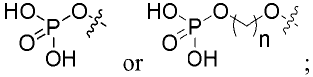 and (3) R₅ and R₆ together with carbon atoms to which they are respectively attached form 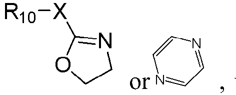 wherein X is a bond, hydrogen, CH₂, S, NH, or O, and R₁₀ is hydrogen, C₁₋₆ alkyl, C₂₋₆ alkenyl, halo C₁₋₆ alkyl, aryl, or C₂₋₅ heteroaryl.

2. The compound according to claim 1, wherein R₁ is C₁₋₆ alkyl, C₂₋₆ alkenyl, C₂₋₆ alkynyl, C₆₋₁₂ aryl, C₃₋₆ cycloalkyl, or C₂₋₅ heterocyclyl, wherein the C₁₋₆ alkyl, C₂₋₆ alkenyl, C₂₋₆ alkynyl, aryl, C₃₋₆ cycloalkyl, and C₂₋₆ heterocyclyl are optionally substituted by one or more R₇;
R₂, R₃, R₄, R₅, R₆, and R₇ are each independently hydrogen, hydroxy, halogen, C₁₋₆ alkyl, C₁₋₆ alkoxy, C₃₋₆ cycloalkoxy, glycosyl, 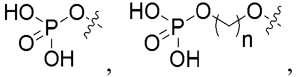 or 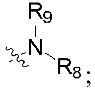 or R₅ and R₆ together with carbon atoms to which they are respectively attached form a 3-6 membered ring;
R₈ and R₉ are each independently hydrogen, C₁₋₆ alkyl, C₂₋₆ alkenyl, C₂₋₆ alkynyl, C₃₋₆ cycloalkyl, C₂₋₆ heterocyclyl, halo C₁₋₆ alkyl, aryl, or C₂₋₅ heteroaryl, or 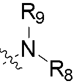 forms a 5-10 membered ring;
n is an integer from 0 to 10;
when R₁ is aryl, the compound satisfies at least one of the following conditions:
(1) at least one of R₄, R₅, R₆, and R₇ is halogen;
(2) at least one of R₅ and R₆ is a glycosyl, 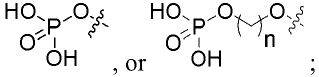 and
(3) R₅ and R₆ together with carbon atoms to which they are respectively attached form 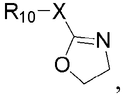 wherein X is hydrogen, CH₂, S, NH, or O; and R₁₀ is hydrogen, C₁₋₆ alkyl, C₂₋₆ alkenyl, halo C₁₋₆ alkyl, aryl, or C₂₋₅ heteroaryl.

3. The compound according to claim 1, wherein R₂ is hydrogen, and R₃ is hydrogen or OH.

4. The compound according to claim 1, wherein R₁ is aryl, and R₇ is an ortho-substituent;
R₄, R₅, and R₆ are each independently hydrogen, halogen, hydroxy, or C₁₋₆ heterocyclyl; and
R₇ is halogen, C₂₋₄ alkyl, or C₁₋₄ alkoxy.

5. The compound according to claim 4, wherein R₁ is 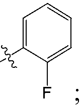
R₄ is hydrogen; and
R₅ and R₆ are each independently hydroxy or halogen.

6. The compound according to claim 1, wherein R₁ is aryl; and
at least one of R₄, R₅, and R₆ is halogen or hydroxyl, and remainder are hydrogen, hydroxyl, or C₁₋₆ heterocyclyl.

7. The compound according to claim 6, wherein R₄ is hydrogen; R₅ is hydroxyl; and R₆ is halogen; or
R₄ is hydrogen; R₅ is halogen; and R₆ is hydroxyl; or
R₄ is hydrogen; R₅ is halogen; and R₆ is halogen; or
R₄ is halogen; and R₅ and R₆ are hydroxyl; or
R₄ is hydrogen; R₅ is hydroxyl; and R₆ is piperidine.

8. The compound according to claim 1, wherein one of R₅ and R₆ is a glycosyl, 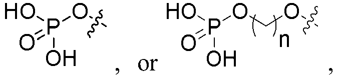 and the other is hydrogen, hydroxyl, halogen, glycosyl, 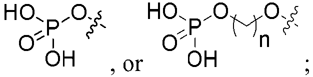
the glycosyl is tetrosyl, pentosyl, or hexosyl;
R₁ is aryl or 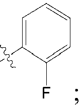
R₂ and R₃ are hydrogen;
R₄ is hydrogen or halogen; and
R₇ is hydrogen, deuterium, halogen, or a C₁₋₆ heterocyclyl.

9. The compound according to claim 8, wherein one of R₅ and R₆ is glycosyl, and the other is hydroxyl;
the glycosyl is 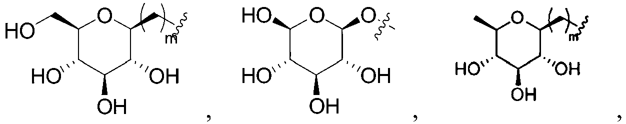 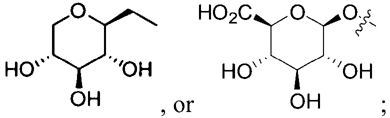
R₁ is aryl;
R₂, R₃, and R₄ are hydrogen; and
m is an integer from 0 to 10.

10. The compound according to claim 9, wherein m is 0, 1, 2, or 3.

11. The compound according to claim 1, wherein n is 0, 1, 2, or 3.

12. The compound according to claim 1, wherein R₁ is aryl;
R₂, R₃, and R₄ are each independently hydrogen, hydroxyl, C₁₋₃ alkyl, or halogen;
R₄ and R₅ together with the carbon atoms to which they are respectively attached form 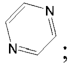 or R₅ and R₆ together with the carbon atoms to which they are respectively attached form 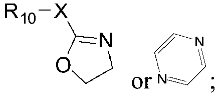
R₇ is hydroxyl or 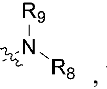 being a meta- or para-substituent;
R₈ and R₉ are each independently hydrogen, C₁₋₃ alkyl, C₂₋₄ alkenyl, C₂₋₄alkynyl, C₄₋₆ cycloalkyl, C₄₋₆ heterocyclyl, aryl, or C₂₋₆ heteroaryl, or 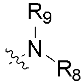 forms a 5-10 membered ring;
the 5-10 membered ring is C₄₋₆ cycloalkyl, C₄₋₆ heterocyclyl, C₈₋₁₀ fused cyclyl or C₈₋₁₀ fused heterocyclyl, and is optionally substituted by C₄₋₆ cycloalkyl or C₄₋₆ heterocyclyl;
X is hydrogen, CH₂, S, NH, or O; and
R₁₀ is hydrogen, C₁₋₃ alkyl, C₂₋₄ alkenyl, halo C₁₋₃ alkyl, aryl, or C₃₋₆ heteroaryl.

13. The compound according to claim 1 or 12, wherein 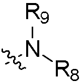 is 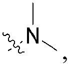 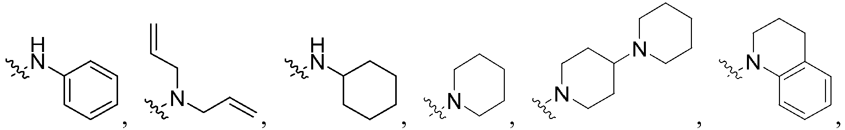 or 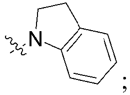 and
R₁₀ is hydrogen or C₁₋₃ alkyl.

14. The compound according to claim 12, wherein 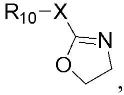 which is formed by R₅ and R₆ together with the carbon atoms to which they are respectively attached, is

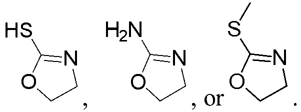

15. The compound according to claim 1, wherein R₁ is C₂₋₄ alkenyl, C₂₋₄ alkynyl, C₃₋₆ cycloalkyl, or C₄₋₆ heterocyclyl;
R₂, R₃, and R₄ are each independently hydrogen, hydroxyl, C₁₋₃ alkyl, or halogen; and
R₅ and R₆ are each independently hydrogen, halogen, or hydroxyl.

16. The compound according to claim 1, wherein R₁ is C₂₋₁₂ heterocyclyl;
R₂, R₃, R₄, R₅, R₆, and R₇ are each independently hydrogen, hydroxyl, halogen, C₁₋₆ alkyl, C₁₋₆ alkoxy, C₃₋₆ cycloalkoxy, C₁₋₆ heterocyclyl, glycosyl, 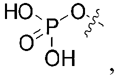 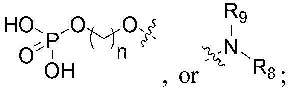 or R₄ and R₅ together with the carbon atoms to which they are respectively attached form a 3-6-membered ring; or R₅ and R₆ together with the carbon atoms to which they are respectively attached form a 3-6-membered ring;
R₈ and R₉ are each independently hydrogen, C₁₋₆ alkyl, C₂₋₆ alkenyl, C₂₋₆ alkynyl, C₃₋₆ cycloalkyl, C₂₋₆ heterocyclyl, halo C₁₋₆ alkyl, aryl, or C₂₋₅ heteroaryl, or 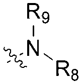 forms a 5-10-membered ring; and
n is an integer from 0 to 10.

17. The compound according to claim 1, wherein R₁ is C₂₋₆ heterocyclyl;
R₂, R₃, and R₄ are each independently hydrogen, hydroxyl, C₁₋₃ alkyl, or halogen;
R₅ and R₆ are each independently hydrogen, hydroxyl, halogen, glycosyl, C₁₋₆ heterocyclyl, or a C₁₋₆ alkoxy; or R₅ and R₆ together with the carbon atoms to which they are respectively attached form 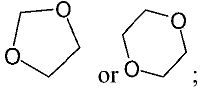
wherein the glycosyl is

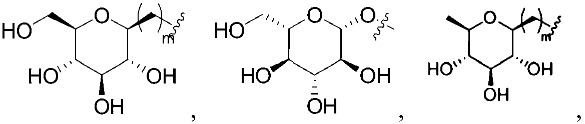

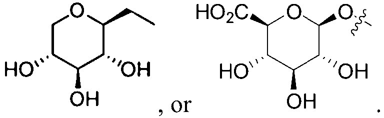

18. The compound according to claim 1, wherein R₁ is 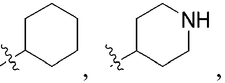 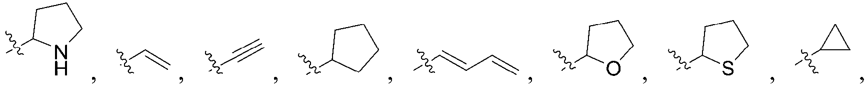 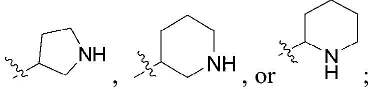
R₂, R₃, and R₄ are each independently hydrogen, hydroxyl, or halogen; and
at least one of R₅ and R₆ is hydroxyl, and the other is hydroxyl or halogen.

19. The compound according to claim 1, wherein the compound is a compound as shown in formula (I), or a stereoisomer, geometric isomer, tautomer, nitrogen oxide, hydrate, solvate, metabolite, pharmaceutically acceptable salt, or prodrug thereof:

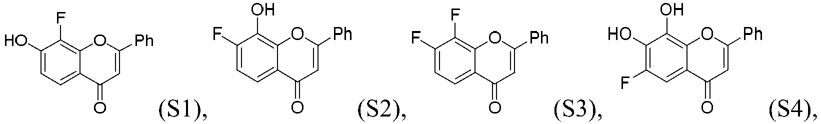

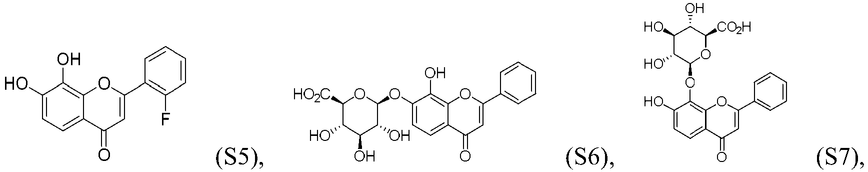

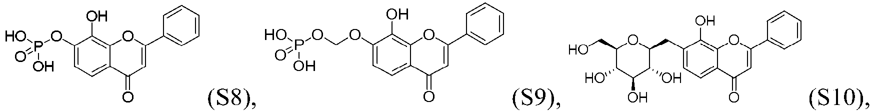

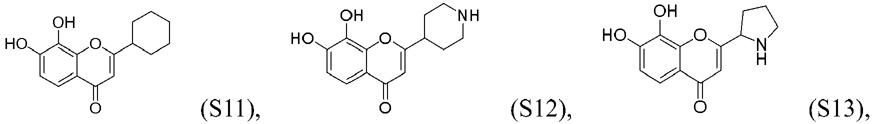

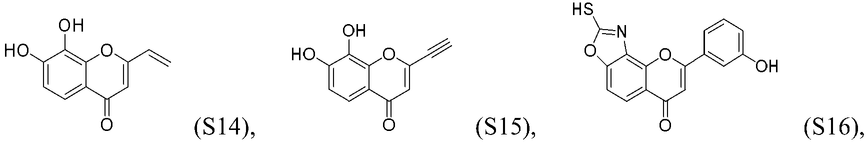

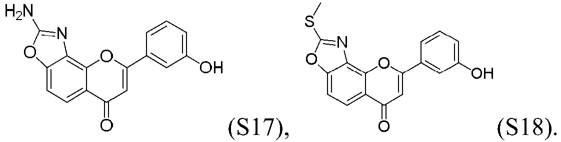

20. The compound according to claim 1, wherein the compound is a compound as shown in formula (I), or a stereoisomer, geometric isomer, tautomer, nitrogen oxide, hydrate, solvate, metabolite, pharmaceutically acceptable salt, or prodrug thereof:

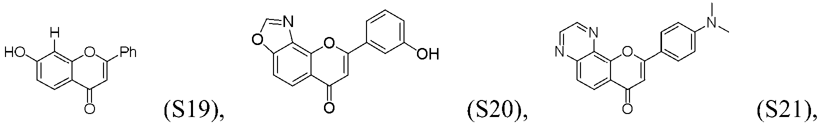

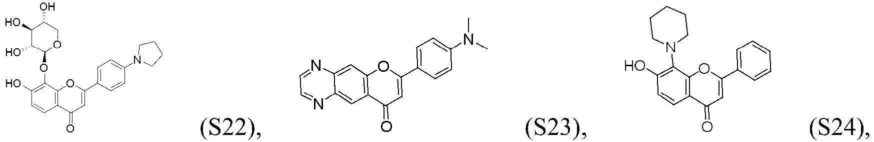

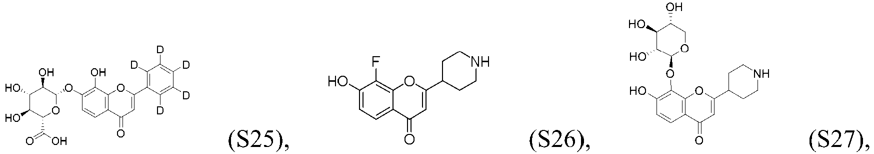

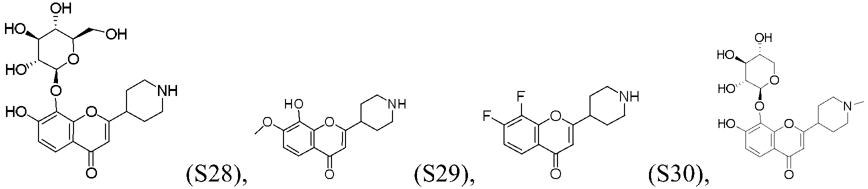

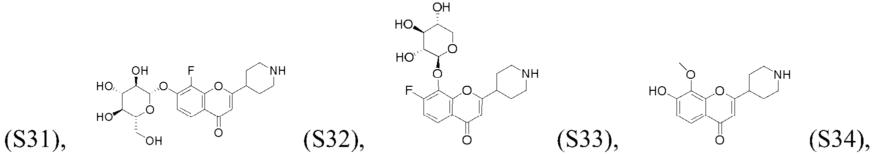

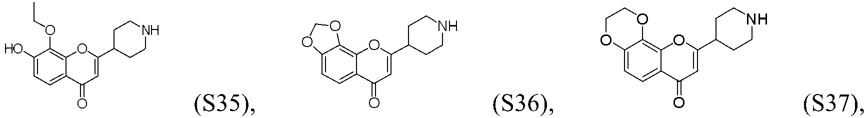

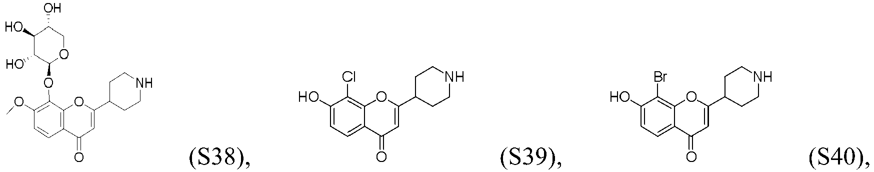

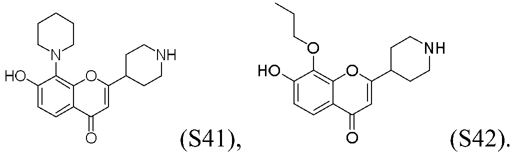

21. The compound according to claim 1, wherein it is not a compound as shown below:

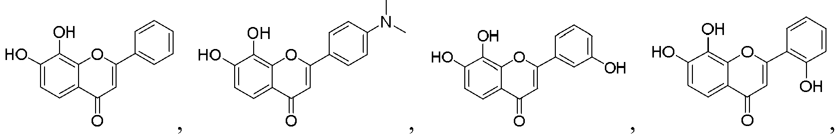

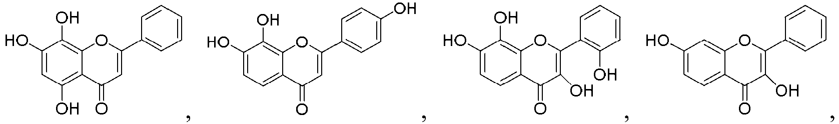

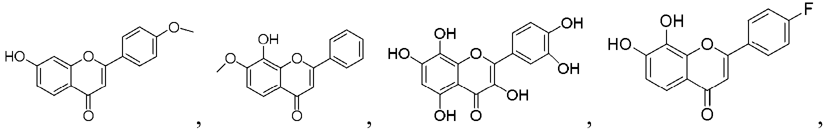

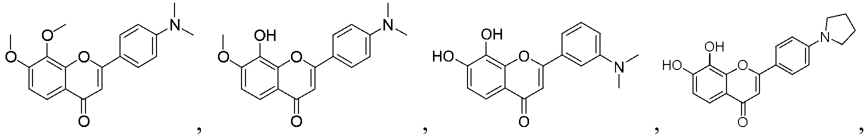

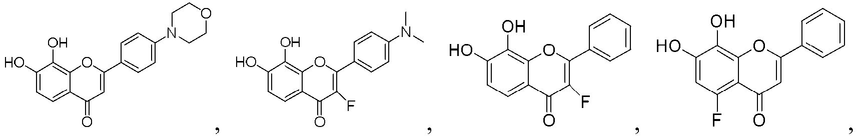

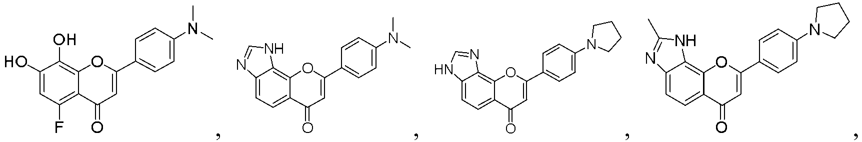

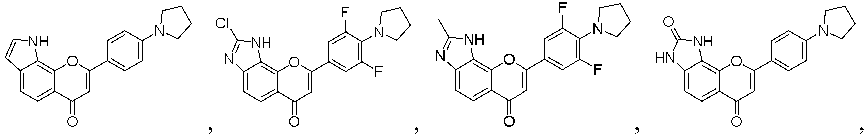

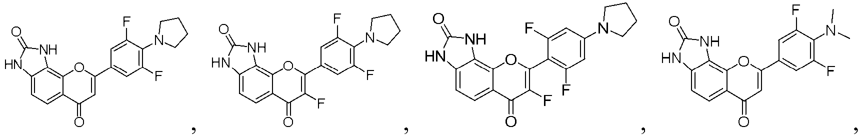

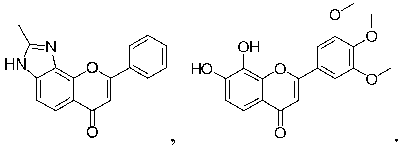

22. A pharmaceutical composition, comprising a compound according to any one of claims 1 to 21.

23. A pharmaceutical composition according to claim 22, further comprising a pharmaceutically acceptable excipient, wherein the excipient includes: a filler, a carrier, an adjuvant, a vehicle, or combinations thereof.

24. A combination drug comprising:
the pharmaceutical composition according to claim 22 or 23; and
one or more therapeutic agents for treating diseases;
wherein the diseases comprise neurological diseases and psychiatric diseases.

25. The combination drug according to claim 24, wherein the neurological diseases comprise: central nervous system injury, peripheral nerve injury, neurodegenerative diseases, and hereditary neurological dysfunctions.

26. The combination drug according to claim 24, wherein the neurological disease is brain injury.

27. The combination drug according to claim 24, wherein the neurological disease is traumatic brain injury.

28. The combination drug according to claim 24, wherein the therapeutic agents for treating diseases comprise at least one of the following: neuroprotective agents, neurotrophic agents, neurorestorative agents, B-vitamins, antidepressants, anxiolytics, lithium salts as mood stabilizers, antipsychotics, atypical antipsychotics, antiepileptics, antiparkinsonian agents, sedative-hypnotics, antihistamines, monoamine oxidase inhibitors, melatonin receptor agonists, non-opioid and opioid analgesics.

29. The combination drug according to claim 24, wherein the therapeutic agents for treating diseases comprise at least one of the following: edaravone, butylphthalide, glutathione, vitamin C, vitamin E, dexmedetomidine, ketamine, propofol, midazolam, remimazolam, citalopram, B vitamins, methylcobalamin, nerve growth factor, gangliosides, levetiracetam, oxiracetam, piracetam, aniracetam, citicoline, donepezil, rivastigmine, galantamine, selegiline, memantine, brexpiprazole, amantadine, levodopa, carbidopa, entacapone, benserazide, bromocriptine, pramipexole, ropinirole, piribedil, cabergoline, apomorphine, lisuride, rotigotine, safinamide, rasagiline, istradefylline, pimavanserin, carbamazepine, oxcarbazepine, lamotrigine, phenytoin sodium, valproic acid, phenobarbital, ethosuximide, topiramate, lacosamide, paroxetine, venlafaxine, duloxetine, escitalopram, sertraline, alprazolam, mirtazapine, morphine, codeine, oxycodone, hydrocodone, fentanyl, pethidine, methadone, dextropropoxyphene, endorphin, dynorphin, chlorpromazine, and haloperidol.

30. Use of the compound according to any one of claims 1 to 21, the pharmaceutical composition according to claim 22 or 23, or the combination drug according to any one of claims 24 to 29 in the manufacture of a medicament, wherein the medicament is used for treating neurological diseases and psychiatric diseases.

31. A method for treating neurological and psychiatric diseases, comprising administering the compound according to any one of claims 1 to 21, the pharmaceutical composition according to claim 22 or 23, or the combination drug according to any one of claims 24 to 29 to a subject in need thereof.
